# EUROPÄISCHE PATENTSCHRIFT

(11) **EP 2 398 778 B1**
(45) Veröffentlichungstag und Bekanntmachung des Hinweises auf die Patenterteilung: **26.03.2014**
(21) Anmeldenummer: 10706500.5
(22) Anmeldetag: 05.02.2010
(51) Int. Cl.: C07D 239/48, C07D 401/12, A01N 43/50

(54) **AMINOPYRIMIDINAMIDE ALS SCHÄDLINGSBEKÄMPFUNGSMITTEL**
AMINOPYRIDINEAMIDES AS PESTICIDES
AMINOPYRIDINEAMIDES EN TANT QUE PESTICIDES

(30) Priorität: 17.02.2009 EP 09152972
(43) Veröffentlichungstag der Anmeldung: 28.12.2011
(73) Patentinhaber: Bayer CropScience AG, 40789 Monheim (DE)
(72) Erfinder: MAECHLING, Simon, 50674 Köln (DE); VOERSTE, Arnd, 50674 Köln (DE); FRANKEN, Eva-Maria, 51381 Leverkusen (DE); BECKER, Angela, 40235 Düsseldorf (DE); GÖRGENS, Ulrich, 40882 Ratingen (DE); ES-SAYED, Mazen, F-69270 Couzon au Mont d'Or (FR); HEIL, Markus, 42799 Leichlingen (DE); HOLMWOOD, Graham, 51371 Leverkusen (DE); JANSEN, Johannes-Rudolf, 40789 Monheim (DE); SCHALLNER, Otto, 40789 Monheim (DE); EBBINGHAUS-KINTSCHER, Ulrich, 44287 Dortmund (DE); LÜMMEN, Peter, 65510 Idstein (DE); CEREZO-GALVEZ, Silvia, 40674 Langenfeld (DE); KUDO, Sachio, Ibaraki 305-0821 (JP); HASHIHAYATA, Takashi, Saitama-shi, 338-0003 (JP); SHIMOJO, Eiichi, Osaka, 586-0018 (JP); ICHIHARA, Teruyuki, Tochigi 323-0822 (JP); ATAKA, Masashi, Saitama, 330-0803 (JP); SHIBUYA, Katsuhiko, Tochigi 329-0433 (JP)
(86) Internationale Anmeldenummer: PCT/EP2010/000719
(87) Internationale Veröffentlichungsnummer: WO 2010/094406

(56) Entgegenhaltungen:
- EP-A1- 0 149 200
- EP-A1- 1 221 444
- WO-A1-98/23597
- WO-A1-02/067684
- WO-A1-2004/037823
- WO-A2-03/013484

## Beschreibung

Die vorliegende Anmeldung betrifft neue Aminopyrimidinamide, Verfahren zu ihrer Herstellung und ihre Verwendung als Schädlingsbekämpfungsmittel und insbesondere als Insektizide und/oder Parasitizide.

Moderne Pflanzenschutzmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

In EP 0 149 200 werden 2-Phenylimidazole und diese Verbindungen enthaltende Arzneimittel beschrieben. EP 1 221 444 beschreibt anellierte Imidazolverbindungen und ihre pharmazeutische Anwendung. WO 1998/023597 offenbart 4,5-Diaminopyrimidine und eine Methode zu ihrer Herstellung. o-Phenylendiamine und ihre pharmazeutischen Anwendungen werden in der WO 2003/013484 beschrieben. In der WO 2004/037823 sind Purinverbindungen und ihre Verwendung zur Behandlung von Krankheiten offenbart. Aus der WO 2002/067684 sind bestimmte Pyridylpyrimidine als Schädlingsbekämpfungsmittel bekannt. Sie erfüllen jedoch nicht alle Anforderungen befriedigend.

Aufgabe der vorliegenden Erfindung war es, Verbindungen bereitzustellen, durch die das Spektrum der Schädlingsbekämpfungsmittel unter verschiedenen Aspekten verbreitert wird.

Gelöst wird die Aufgabe, sowie weitere nicht explizit genannte Aufgaben, die aus den hierin diskutierten Zusammenhängen ableitbar oder erschließbar sind, durch neue Verbindungen der Formel (I) 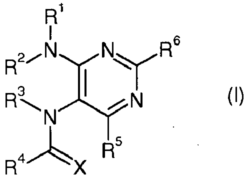 in welcher
- R¹: für einen Rest aus der Reihe C₁-C₆-Alkyl, C₂-C₆-Alkenyl, C₂-C₆-Alkinyl, C₁-C₆-Haloalkyl, Cyano-C₁-C₆-alkyl, C₃-C₈-Cycloalkyl (besonders C₃-C₆-Cycloalkyl), C₃-C₈-Halocycloalkyl, C₃-C₈-Cycloalkenyl, C₃-C₈-Halocycloalkenyl, C₁-C₆-Alkoxy-C₁-C₆-alkyl, C₁-C₆-Haloalkoxy-C₁-C₆-alkyl, C₁-C₆-Alkoxy-C₁-C₆-haloalkyl, C₁-C₆-Alkylcarbonyl-C₁-C₆-alkyl, C₁-C₆-Alkoxycarbonyl-C₁-C₆-alkyl, C₁-C6-Alkylsulfanyl-C₁-C₆-alkyl, C₁-C₆-Alkylsulfinyl-C₁-C₆-alkyl, C₁-C₆-Alkylsulfonyl-C₁-C₆-alkyl, C₁-C₆-Haloalkylsulfanyl-C₁-C₆-alkyl, C₁-C₆-Haloalkylsulfinyl-C₁-C₆-alkyl, C₁-C₆-Haloalkylsulfonyl-C₁-C₆-alkyl, C₃-C₈-Cycloalkyl-C₁-C₆-alkyl, halogeniertes C₃-C₈-Cycloalkyl-C₁-C₆-alkyl, Phenyl-C₁-C₆-alkyl, welches am Phenylring gegebenenfalls einfach oder mehrfach, gleich oder verschieden substituiert ist durch Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Alkylsulfinyl, C₁-C₆-Alkylsulfonyl, C₁-C₆-Haloalkylsulfanyl, C₁-C₆-Haloalkylsulfinyl, C₁-C₆-Haloalkylsulfonyl, Nitro oder Cyano; Heteroaryl, welches gegebenenfalls einfach oder mehrfach, gleich oder verschieden substituiert ist durch -OH, -SH, -NH₂, Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Alkylsulfinyl, C₁-C₆-Alkylsulfonyl, C₁-C₆-Haloalkylsulfanyl, C₁-C₆-Haloalkylsulinyl, C₁-C₆-Haloalkylsulfonyl, Mono-C₁-C₆-alkylamino, Di-C₁-C₆-alkylamino, Nitro oder Cyano; Heteroaryl-C₁-C₆-alkyl, welches am Heteraromaten gegebenenfalls einfach oder mehrfach, gleich oder verschieden substituiert ist durch -OH, -SH, -NH₂, Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Alkylsulfinyl, C₁-C₆-Alkylsulfonyl, C₁-C₆-Haloalkylsulfanyl, C₁-C₆-Haloalkylsulfinyl, C₁-C₆-Haloalkylsulfonyl, Mono-C₁-C₆-alkylamino, Di-C₁-C₆-alkylamino, Nitro oder Cyano; Heterocyclyl, welches gegebenenfalls einfach oder mehrfach, gleich oder verschieden substituiert ist durch -OH / =O, -SH / =S,-NH₂, Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Alkylsulfinyl, C₁-C₆-Alkylsulfonyl, C₁-C₆-Haloalkylsulfanyl, C₁-C₆-Haloalkylsulfinyl, C₁-C₆-Haloalkylsulfonyl, Mono-C₁-C₆-alkylamino, Di-C₁-C₆-alkylamino, Nitro oder Cyano und Heterocyclyl-C₁-C₆-alkyl steht, welches am Heterocyclus gegebenenfalls einfach oder mehrfach, gleich oder verschieden substituiert ist durch -OH / =O, -SH / =S, -NH₂, Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Alkylsulfinyl, C₁-C₆-Alkylsulfonyl, C₁-C₆-Haloalkylsulfanyl, C₁-C₆-Haloalkylsulfinyl, C₁-C₆-Haloalkylsulfonyl, Mono-C₁-C₆-alkylamino, Di-C₁-C₆-alkylamino, Nitro oder Cyano,
- R²: für einen Rest aus der Reihe Wasserstoff, C₁-C₆-Alkyl, C₂-C₆-Alkenyl, C₂-C₆-Alkinyl, C₁-C₆-Haloalkyl, Cyano-C₁-C₆-alkyl, C₃-C₈-Cycloalkyl (besonders C₃-C₆-Cycloalkyl), C₃-C₈-Halocycloalkyl, C₃-C₈-Cycloalkenyl, C₃-C₈-Halocycloalkenyl, C₁-C₆-Alkoxy-C₁-C₆-alkyl, C₁-C₆-Haloalkoxy-C₁-C₆-alkyl, C₁-C6-Alkylcarbonyl-C₁-C₆-alkyl, C₁-C₆-Alkoxycarbonyl-C₁-C₆-alkyl, C₁-C₆-Alkylsulfanyl-C₁-C₆-alkyl, C₁-C₆-Alkylsulfinyl-C₁-C₆-alkyl, C₁-C₆-Alkylsulfonyl-C₁-C₆-alkyl, C₁-C₆-Haloalkylsulfanyl-C₁-C₆-alkyl, C₁-C₆-Haloalkylsulfinyl-C₁-C₆-alkyl, C₁-C₆-Haloalkylsulfonyl-C₁-C₆-alkyl, C₃-C₈-Cycloalkyl-C₁-C₆-alkyl, Phenyl-C₁-C₆-alkyl, welches am Phenylring gegebenenfalls einfach oder mehrfach, gleich oder verschieden substituiert ist durch Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Alkylsulfinyl, C₁-C₆-Alkylsulfonyl, C₁-C₆-Haloalkylsulfanyl, C₁-C₆-Haloalkylsulfinyl, C₁-C₆-Haloalkylsulfonyl, Nitro oder Cyano; Heteroaryl, welches gegebenenfalls einfach oder mehrfach, gleich oder verschieden substituiert ist durch -OH, -SH, -NH₂, Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Alkylsulfinyl, C₁-C₆-Alkylsulfonyl, C₁-C₆-Haloalkylsulfanyl, C₁-C₆-Haloalkylsulfinyl, C₁-C₆-Haloalkylsulfonyl, Mono-C₁-C₆-alkylamino, Di-C₁-C₆-alkylamino, Nitro oder Cyano; Heteroaryl-C₁-C₆-alkyl, welches am Heteraromaten gegebenenfalls einfach oder mehrfach, gleich oder verschieden substituiert ist durch -OH, -SH, -NH₂, Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Alkylsulfinyl, C₁-C₆-Alkylsulfonyl, C₁-C₆-Haloalkylsulfanyl, C₁-C₆-Haloalkylsulfinyl, C₁-C₆-Haloalkylsulfonyl, Mono-C₁-C₆-alkylamino, Di-C₁-C₆-alkylamino, Nitro oder Cyano; Heterocyclyl, welches gegebenenfalls einfach oder mehrfach, gleich oder verschieden substituiert ist durch -OH / =O, -SH / =S,-NH₂, Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Alkylsulfinyl, C₁-C₆-Alkylsulfonyl, C₁-C₆-Haloalkylsulfanyl, C₁-C₆-Haloalkylsulfinyl, C₁-C₆-Haloalkylsulfonyl, Mono-C₁-C₆-alkylamino, Di-C₁-C₆-alkylamino, Nitro oder Cyano und Heterocyclyl-C₁-C₆-alkyl steht, welches am Heterocyclus gegebenenfalls einfach oder mehrfach, gleich oder verschieden substituiert ist durch -OH / =O, -SH / =S, -NH₂, Halogen, C₁-C₆-Alkyl, C₁-C₆-Haloalkyl, C₁-C₆- Alkoxy, C₁-C₆-Haloalkoxy, C₁-C₆-Alkylsulfanyl, C₁-C₆-Alkylsulfinyl, C₁-C₆-Alkylsulfonyl, C₁-C₆-Haloalkylsulfanyl, C₁-C₆-Haloalkylsulfinyl, C₁-C₆-Haloalkylsulfonyl, Mono-C₁-C₆-alkylamino, Di-C₁-C₆-alkylamino, Nitro oder Cyano,
- R³: für einen Rest aus der Reihe Wasserstoff, C₁-C₆-Alkyl, C₂-C₆-Alkenyl, C₂-C₆-Alkinyl, Halo-C₁-C₆-alkyl, Cyano-C₁-C₆-alkyl, C₃-C₈-Cycloalkyl, Halo-C₃-C₈-Cycloalkyl, C₃-C₈-Cycloalkenyl, Halo-C₃-C₈-Cycloalkenyl, C₁-C₆-Alkoxy-C₁-C₆-alkyl, C₁-C₆-Alkylcarbonyl-C₁-C₆-alkyl, C₁-C₆-Alkoxycarbonyl-C₁-C₆-alkyl, C₁-C₆-Alkylsulfanyl-C₁-C₆-alkyl, C₁-C₆-Alkylsulfinyl-C₁-C₆-alkyl, C₁-C₆-Alkylsulfonyl-C₁-C₆-alkyl, C₃-C₈-Cycloalkyl-C₁-C₆-alkyl, Phenyl, welches gegebenenfalls einfach oder mehrfach, gleich oder verschieden substituiert ist durch -OH, -SH, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, Mono-C₁-C₆-alkylamino, Di-C₁-C₆-alkylamino, Nitro oder Cyano; Phenyl-C₁-C₆-alkyl, welches am Phenylring gegebenenfalls einfach oder mehrfach, gleich oder verschieden substituiert ist durch Halogen, C₁-C₆-Alkyl, C₁-C₆-Haloalkyl, C₁-C₆-Alkoxy, Nitro oder Cyano; Heteroaryl, welches gegebenenfalls einfach oder mehrfach gleich oder verschieden substituiert ist durch -OH, -SH, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, Mono-C₁-C₆-alkylamino, Di-C₁-C₆-alkylamino, Nitro oder Cyano; Heteroaryl-C₁-C₆-alkyl, welches am Heteraromaten gegebenenfalls einfach oder mehrfach, gleich oder verschieden substituiert ist durch -OH, -SH, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, Mono-C₁-C₆-alkylamino, Di-C₁-C₆-alkylamino, Nitro oder Cyano; Heterocyclyl, welches gegebenenfalls einfach oder mehrfach, gleich oder verschieden substituiert ist durch -OH / =O, -SH / =S, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, Mono-C₁-C₆-alkylamino, Di-C₁-C₆-alkylamino, Nitro oder Cyano und Heterocyclyl-C₁-C₆-alkyl steht, welches am Heterocyclus gegebenenfalls einfach oder mehrfach, gleich oder verschieden substituiert ist durch -OH / =O, -SH / =S, -NH₂, Halogen, C₁-C₆-Alkyl, C₁-C₆- Haloalkyl, C₁-C₆-Alkoxy, C₁-C₆-Alkylsulfanyl, C₁-C₆-Alkylsulinyl, C₁-C₆-Alkylsulfonyl, Mono-C₁-C₆-alkylamino, Di-C₁-C₆-alkylamino, Nitro oder Cyano,
- R⁴: für einen Rest aus der Reihe Phenyl, 2-Pyridyl, 3-Pyridyl und Pyrazolyl steht, welche jeweils gegebenenfalls einfach oder mehrfach, gleich oder verschieden substituiert sind durch Reste aus der Reihe Halogen, C₁-C₆-Alkyl, C₁-C₆-Haloalkyl, C₃-C₆-Cycloalkyl, C₃-C₆-Halocycloalkyl, C₃-C₆-Cycloalkyl-C₁-C₆-alkyl, C₁-C₆-Alkoxy, C₁-C₆-Haloalkoxy, C₁-C₆-Alkoxy-C₁-C₆-alkyl, C₁-C₆-Alkoxy-C₁-C₆-alkoxy, COOH, Nitro, Amino, Cyano, C₁-C₆-Alkylsulfanyl, C₁-C₆- Alkylsulfinyl, C₁-C₆-Alkylsulfonyl, C₁-C₆-Haloalkylsulfanyl, C₁-C₆-Haloalkylsulfinyl, C₁-C₆-Haloalkylsulfonyl, Mono-C₁-C₆-alkylamino, Di-C₁-C₆-alkylamino, C₁-C₆-Alkylaminocarbonyl, C₃-C₆-Cycloalkylaminocarbonyl, Phenyl (welches selbst wiederum substituiert sein kann durch Halogen), und Hetaryl-C₁-C₆-alkyl (insbesondere Pyrazolyl-C₁-C₆-alkyl (welches selbst wieder substituiert sein kann durch Halogen, C₁-C₆-Alkyl und C₁-C₆-Halogenalkyl),
- R⁵: für einen Rest aus der Reihe Wasserstoff, Halogen, Cyano, C₁-C₆-Alkyl, C₁-C₆-Haloalkyl, C₃-C₈-Cycloalkyl, C₃-C₈-Halocycloalkyl sowie Phenyl, 2-Pyridyl oder 3-Pyridyl steht, welche jeweils gegebenenfalls einfach oder mehrfach, gleich oder verschieden substituiert sind durch Reste aus der Reihe Halogen, Amino, Cyano, C₁-C₆-Alkyl, C₁-C₆-Haloalkyl, C₃-C₆-Cycloalkyl, C₃-C₆-Halocycloalkyl, C₁-C₆-Alkoxy, C₁-C₆-Haloalkoxy, C₂-C₆-Alkenyl, C₁-C₆-Alkoxy-C₁-C₆-alkyl, C₁-C₆-Alkoxy-C₁-C₆-alkoxy, Nitro, Cyano, C₁-C₆-Alkylcarbonyl, C₁-C₆-Alkylcarbonylamino, C₃-C₆-Cycloalkylcarbonylamino, C₁-C₆-Alkylaminocarbonyl, C₃-C₆-Cycloalkylaminocarbonyl C₁-C₆-Alkoxycarbonyl, Aminocarbonyl, C₁-C₆-Alkoxyimino-C₁-C₆-alkyl, C₁-C₆-Alkylsulfanyl, C₁-C₆-Alkylsulfinyl, C₁-C₆-Alkylsulfonyl, C₁-C₆-Alkylsulfonyloxy, C₁-C₆-Alkylsulfonylamino, C₁-C₆-Haloalkylsulfanyl, C₁-C₆-Haloalkylsulfinyl, C₁-C₆-Haloalkylsulfonyl, Tri-C₁-C₆-alkylsilyl, S(O)(=N-CN), S(O)(=N)R (worin R für C₁-C₆-Alkyl oder C₁-C₆-Haloalkyl steht), Phenyl, Phenyl-C₁-C₆-alkyl, Phenyl-C₁-C₆-alkyloxy, Phenyloxy, Pyrazolyl (wobei die Phenylgruppen bzw. der Pyrazolylring wiederum substituiert sein können durch Halogen, C₁-C₆-Alkyl, C₃-C₈-Cycloalkyl, C₁-C₆-Alkoxy, C₁-C₆-Haloalkoxy, Nitro, Cyano, C₁-C₆-Alkylsulfanyl, C₁-C₆-Alkylsulfinyl, C₁-C₆-Alkylsulfonyl, C₁-C₆-Alkylsulfonyloxy, C₁-C₆-Alkylsulfonylamino, C₁-C₆-Haloalkylsulfanyl, C₁-C₆-Haloalkylsulfinyl, C₁-C₆-Haloalkylsulfonyl, S(O)(=N-CN), S(O)(=N)R (worin R für C₁-C₆-Alkyl oder C₁-C₆-Haloalkyl steht)), Hetaryloxy-C₁-C₆-alkyl, Hetaryloxy und Hetaryl-C₁-C₆-alkyl, (wobei der Hetarylrest jeweils ausgewählt ist aus der Reihe Pyrrolyl, Imidazolyl, Pyrazolyl, Triazolyl, Pyridyl, Pyrimidinyl und Tetrazolyl und selbst wiederum substituiert sein kann durch Halogen, Cyano, C₁-C₆-Alkyl, C₁-C₆-Haloalkyl, C₃-C₈-Cyaoalkyl, C₃-C₈-Halocycloalkyl) und wobei zwei benachbarte Substituenten auch gemeinsam für -OCH₂O-, -OCF₂O oder -OCH₂CH₂O- stehen können,
- R⁶: für einen Rest aus der Reihe Wasserstoff, Halogen, C₁-C₆-Alkyl, C₁-C₆-Haloalkyl, Cyano-C₁-C₆-alkyl, C₁-C₆-Alkoxy-C₁-C₆-alkyl, C₁-C₆-Alkylcarbonyl, C₁-C₆-Haloalkylcarbonyl, C₃-C₈-Cycloalkyl, C₃-C₈-Halocycloalkyl, C₃-C₈-Cycloalkyl-C₁-C₆-alkyl, C₁-C₆-Alkylsulfanyl, C₁-C₆-Alkylsulfonyl, C₂-C₆-Alkenyl, C₂-C₆-Alkinyl, C₁-C₆-Alkoxycarbonyl, C₂-C₆-Alkenyloxycarbonyl, C₂-C₆-Alkinyloxycarbonyl, C₁-C₆-Alkoxycarbonyl-C₁-C₆-alkyl; Phenyl, Phenyl-C₁-C₆-alkyl, welche jeweils am Phenylring gegebenenfalls einfach oder mehrfach, gleich oder verschieden substituiert sind durch Halogen, C₁-C₆-Alkyl, C₁-C₆-Haloalkyl, C₁-C₆-Alkoxy, Nitro oder Cyano; Heterocyclyl-C₁-C₆-alkyl, welches am Heterocyclus gegebenenfalls einfach oder mehrfach, gleich oder verschieden substituiert ist durch Halogen, C₁-C₆-Alkyl, C₁-C₆-Haloalkyl, C₁-C₆-Alkoxy, Nitro oder Cyano; Heteroaryl-C₁-C₆-alkyl, welches am Heteroaromaten gegebenenfalls einfach oder mehrfach, gleich oder verschieden substituiert ist durch Halogen, C₁-C₆-Alkyl, C₁-C₆-Haloalkyl, C₁-C₆-Alkoxy, Nitro oder Cyano; oder Phenylcarbonyl steht, welches am Phenylring gegebenenfalls einfach oder mehrfach gleich oder verschieden substituiert ist durch Halogen, C₁-C₆-Alkyl, C₁-C₆-Haloalkyl, C₁-C₆-Alkoxy, Nitro oder Cyano und
- X: für Sauerstoff oder Schwefel steht.

Weiter wurde gefunden, dass sich Verbindungen der Formel (I) herstellen lassen (Verfahren 1), indem man Verbindungen der Formel (II) 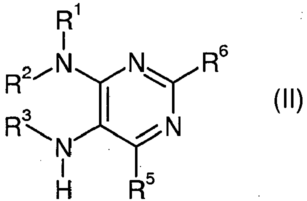 in welcher
- R¹, R², R³, R⁵ und R⁶,: die oben angegebenen Bedeutungen haben
mit einer Verbindung der Formel (III)
- R⁴: CXHal (III)
in welcher
- X: die oben angegebene Bedeutung hat und
- Hal: für Halogen, insbesondere Chlor steht
in Gegenwart eines Verdünnungsmittels und in Gegenwart einer Base umsetzt.

Verbindungen der Formel (I) lassen sich auch herstellen (Verfahren 2), indem man Verbindungen der Formel (IV) 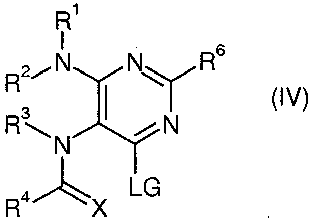 in welcher
- R¹, R², R³, R⁴ und R⁶: die oben angegebenen Bedeutung haben und
- LG: für Chlor, Brom, Iod oder Alkylsulfonyl steht,
mit Boronsäuren oder Boronsäureestern der Formel (V)

R⁵-B(OR^{a})₂

worin
- R⁵: die oben angegebenen Bedeutungen hat und
- R^{a}: für Wasserstoff oder Alkyl (insbesondere C₁-C₄-Alkyl) steht,
in Gegenwart eines Verdünnungsmittels, eines Palladium-Katalysators und einer Base umsetzt (Suzuki-Reaktion).

Schließlich wurde gefunden, das die neuen Verbindungen der Formel (I) stark ausgeprägte biologische Eigenschaften besitzen und vor allem zur Bekämpfung von tierischen Schädlingen, insbesondere von Insekten, Spinnentieren und Nematoden, die in der Landwirtschaft, in den Forsten, im Vorrats- und Materialschutz sowie auf dem Hygienesektor vorkommen, geeignet sind.

Die Verbindungen der Formel (I) können gegebenenfalls in Abhängigkeit von der Art der Substituenten als geometrische und/oder als optisch aktive Isomere oder entsprechende Isomerengemische oder in unterschiedlichen tautomeren Formen in unterschiedlicher Zusammensetzung vorliegen. Die Erfindung betrifft sowohl die reinen Isomere als auch die Isomerengemische.

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die erfindungsgemäßen Verbindungen sind durch die Formel (I) allgemein definiert.

Bevorzugte Substituenten bzw. Bereiche der in den oben und nachstehend erwähnten Formeln aufgeführten Reste werden im Folgenden erläutert.
- R¹: steht bevorzugt für einen Rest aus der Reihe C₁-C₆-Alkyl, C₂-C₆-Alkenyl, C₂-C₆-Alkinyl, C₁-C₆-Haloalkyl, C₃-C₈-Cycloalkyl (besonders C₃-C₆-Cycloalkyl), C₃-C₈-Halocycloalkyl, C₃-C₈-Cycloalkenyl, C₁-C₆-Alkoxy-C₁-C₆-alkyl, C₁-C₆-Alkylsulfanyl-C₁-C₆-alkyl, C₁-C₆-Alkylcarbonyl-C₁-C₆-alkyl, C₁-C₆-Alkoxycarbonyl-C₁-C₆-alkyl, C₃-C₆-Cycloalkyl-C₁-C₆-alkyl, halogeniertes C₃-C₆-Cycloalkyl-C₁-C₆-alkyl, Phenyl-C₁-C₆-alkyl, welches am Phenylring gegebenenfalls einfach oder mehrfach, gleich oder verschieden substituiert ist durch Halogen, C₁-C₆-Alkyl, C₁-C₆-Haloalkyl, C₁-C₆-Alkoxy, Nitro oder Cyano; Heteroaryl, welches gegebenenfalls einfach oder mehrfach, gleich oder verschieden substituiert ist durch -OH, -SH, -NH₂, Halogen, C₁-C₆-Alkyl, C₁-C₆-Haloalkyl, C₁-C₆-Alkoxy, C₁-C₆-Alkylsulfanyl, C₁-C₆- Alkylsulfinyl, C₁-C₆-Alkylsulfonyl, Mono-C₁-C₆-alkylamino, Di-C₁-C₆-alkylamino, Nitro oder Cyano; Heteroaryl-C₁-C₆-alkyl, welches am Heteraromaten gegebenenfalls einfach oder mehrfach, gleich oder verschieden substituiert ist durch -OH,-SH, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, Mono-C₁-C₆-alkylamino, Di-C₁-C₆-alkylamino, Nitro oder Cyano; Heterocyclyl-C₁-C₆-alkyl, welches am Heterocyclus gegebenenfalls einfach oder mehrfach, gleich oder verschieden substituiert ist durch -OH / =O, -SH / =S,-NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, Mono-C₁-C₆-alkylamino, Di-C₁-C₆-alkylamino, Nitro oder Cyano.
- R²: steht bevorzugt für einen Rest aus der Reihe Wasserstoff, Ci-C₆-Alkyl, C₂-C₆-Alkenyl, C₂-C₆-Alkinyl, C₁-C₆-Haloalkyl, C₃-C₈-Cycloalkyl (besonders C₃-C₆-Cycloalkyl), C₃-C₈-Halocycloalkyl, C₃-C₈-Cycloalkenyl, C₁-C₆-Alkoxy-C₁-C₆-alkyl, C₁-C₆-Alkylcarbonyl-C₁-C₆-alkyl, C₁-C₆-Alkoxycarbonyl-C₁-C₆-alkyl, Phenyl-C₁-C₆-alkyl, welches am Phenylring gegebenenfalls einfach oder mehrfach, gleich oder verschieden substituiert ist durch Halogen, C₁-C₆-Alkyl, C₁-C₆-Haloalkyl, C₁-C₆-Alkoxy, Nitro oder Cyano; Heteroaryl, welches gegebenenfalls einfach oder mehrfach, gleich oder verschieden substituiert ist durch -OH,-SH, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, Mono-C₁-C₆-alkylamino, Di-C₁-C₆-alkylamino, Nitro oder Cyano; Heteroaryl-C₁-C₆-alkyl, welches am Heteraromaten gegebenenfalls einfach oder mehrfach, gleich oder verschieden substituiert ist durch -OH, -SH, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, Mono-C₁-C₆-alkylamino, Di-C₁-C₆-alkylamino, Nitro oder Cyano; Heterocyclyl-C₁-C₆-alkyl, welches am Heterocyclus gegebenenfalls einfach oder mehrfach, gleich oder verschieden substituiert ist durch -OH / =O, -SH / =S, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, Mono-C₁-C₆-alkylamino, Di-C₁-C₆-alkylamino, Nitro oder Cyano.
- R³: steht bevorzugt für einen Rest aus der Reihe Wasserstoff, C₁-C₆-Alkyl, C₂-C₆- Alkenyl, C₂-C₆-Alkinyl, C₁-C₆-Haloalkyl, C₃-C₈-Cycloalkyl (besonders C₃-C₆-Cycloalkyl), C₃-C₈-Halocycloalkyl, C₃-C₈-Cycloalkenyl, C₁-C₆-Alkoxy-C₁-C₆-alkyl, C₁-C₆-Alkylcarbonyl-C₁-C₆-alkyl, C₁-C₆-Alkoxycarbonyl-C₁-C₆-alkyl, Phenyl-C₁-C₆-alkyl, welches am Phenylring gegebenenfalls einfach oder mehrfach, gleich oder verschieden substituiert ist durch Halogen, C₁-C₆-Alkyl, C₁-C₆-Haloalkyl, C₁-C₆-Alkoxy, Nitro oder Cyano; Heteroaryl, welches gegebenenfalls einfach oder mehrfach, gleich oder verschieden substituiert ist durch -OH,-SH, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, Mono-C₁-C₆-alkylamino, Di-C₁-C₆-alkylamino, Nitro oder Cyano; Heteroaryl-C₁-C₆-alkyl, welches am Heteraromaten gegebenenfalls einfach oder mehrfach, gleich oder verschieden substituiert ist durch -OH, -SH, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, Mono-C₁-C₆-alkylamino, Di-C₁-C₆-alkylamino, Nitro oder Cyano; Heterocyclyl-C₁-C₆-alkyl, welches am Heterocyclus gegebenenfalls einfach oder mehrfach, gleich oder verschieden substituiert ist durch -OH / =O, -SH / =S, -NH₂, Halogen, C_{I}-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, Mono-C₁-C₆-alkylamino, Di-C₁-C₆-alkylamino, Nitro oder Cyano.
- R⁴: steht bevorzugt für 3-Pyridyl oder Pyrazolyl, welche jeweils gegebenenfalls einfach oder mehrfach, gleich oder verschieden substituiert sind durch Reste aus der Reihe Halogen, C₁-C₆-Alkyl, C₁-C₆-Haloalkyl, C₃-C₆-Cycloalkyl, C₃-C₆-Halocycloalkyl, C₃-C₆-Cycloalkyl-C₁-C₆-alkyl, C₁-C₆-Alkoxy, C₁-C₆-Alkoxy-C₁-C₆-alkyl, C₁-C₆-Alkoxy-C₁-C₆-alkoxy, COOH, Nitro, Amino, Mono-C₁-C₆-alkylamino, Di-C₁-C₆-alkylamino, C₁-C₆-Alkylaminocarbonyl und C₃-C₆-Cycloalkylaminocarbonyl.
- R⁴: steht weiterhin auch bevorzugt für Pyrazolyl, welches gegebenenfalls substituiert ist durch Halogen oder C₁-C₆-Alkyl, Phenyl (welches selbst wiederum substituiert sein kann durch Halogen) oder durch Pyrazolylmethyl (welches selbst wieder substituiert sein kann durch C₁-C₆-Halogenalkyl).
- R⁵: steht bevorzugt für Phenyl, 2-Pyridyl oder 3-Pyridyl, (insbesondere für Phenyl)welche jeweils gegebenenfalls einfach oder mehrfach, gleich oder verschieden substituiert sind durch Reste aus der Reihe Halogen, C₁-C₆-Alkyl, C₁-C₆-Haloalkyl, C₃-C₆-Cycloalkyl, C₃-C₆-Halocycloalkyl, C₁-C₆-Alkoxy, C₁-C₆-Haloalkoxy, C₁-C₆-Alkoxy-C₁-C₆-alkyl, C₁-C₆-Alkoxy-C₁-C₆-alkoxy, Nitro, Amino, Cyano, Mono-C₁-C₆-alkylamino, Di-C₁-C₆-alkylamino, C₁-C₆-Alkylsulfanyl, C₁-C₆-Alkylsulfinyl, C₁-C₆-Alkylsulfonyl, C₁-C₆-Haloalkylsulfanyl, C₁-C₆-Haloalkylsulfinyl, C₁-C₆-Haloalkylsulfonyl, Hetaryloxy-C₁-C₆-alkyl, Hetaryloxy und Hetaryl-C₁-C₆-alkyl wobei der Hetarylrest ausgewählt ist aus der Reihe Pyrrolyl, Imidazolyl, Pyrazolyl, Triazolyl, Pyridyl, Pyrimidinyl und Tetrazolyl und selbst wiederum substituiert sein kann durch Substituenten aus der Reihe Halogen, Cyano, C₁-C₆-Alkyl, C₁-C₆-Haloalkyl, C₃-C₈-Cycloalkyl und C₃-C₈-Halocycloalkyl.
- R⁵: steht auch bevorzugt für Wasserstoff, Halogen, C₁-C₄-Alkyl und C₃-C₆-Cycloalkyl.
- R⁶: steht bevorzugt für einen Rest aus der Reihe Wasserstoff, Halogen, C₁-C₆-Alkyl, C₁-C₆-Haloalkyl, C₁-C₆-Alkoxy-C₁-C₆-alkyl, C₁-C₆-Alkylcarbonyl, C₁-C₆-Haloalkylcarbonyl, C₃-C₆-Cycloalkyl, C₃-C₆-Cycloalkyl-C₁-C₆-alkyl, C₁-C₆-Alkylsulfanyl, C₂-C₆-Alkenyl, C₂-C₆-Alkinyl, C₁-C₆-Alkoxycarbonyl, C₂-C₆-Alkenyloxycarbonyl, C₂-C₆-Alkinyloxycarbonyl, C₁-C₆-Alkoxycarbonyl-C₁-C₆-alkyl, Phenyl, Phenyl-C₁-C₆-alkyl, welche jeweils am Phenylring gegebenenfalls einfach oder mehrfach, gleich oder verschieden substituiert sind durch Halogen, C₁-C₆-Alkyl, C₁-C₆-Haloalkyl, C₁-C₆-Alkoxy, Nitro oder Cyano; Heterocyclyl-C₁-C₆-alkyl, welches am Heterocyclus gegebenenfalls einfach oder mehrfach, gleich oder verschieden substituiert sein kann durch Halogen, C₁-C₆-Alkyl, C₁-C₆-Haloalkyl, C₁-C₆-Alkoxy, Nitro oder Cyano; und Phenylcarbonyl, welches am Phenylring gegebenenfalls einfach oder mehrfach, gleich oder verschieden substituiert ist durch Halogen, C₁-C₆-Alkyl, C₁-C₆-Haloalkyl, C₁-C₆-Alkoxy, Nitro oder Cyano.
- X: steht bevorzugt für Sauerstoff oder Schwefel.
- R¹: steht besonders bevorzugt für einen Rest aus der Reihe C₁-C₆-Alkyl, C₁-C₆-Haloalkyl, C₂-C₆-Alkenyl, C₂-C₆-Alkinyl, C₃-C₆-Cycloalkyl, C₃-C₆-Halocycloalkyl, C₁-C₆-Alkoxy-C₁-C₆-alkyl, C₁-C₆-Alkylsulfanyl-C₁-C₆-alkyl, C₁-C₆-Alkoxycarbonyl-C₁-C₆-alkyl und C₃-C₆-Cycloalkyl-C₁-C₆-alkyl und halogeniertes C₃-C₆-Cycloalkyl-C₁-C₆-alkyl, insbesondere für Cyclopropyl.
- R²: steht besonders bevorzugt für Wasserstoff,
- R³: steht besonders bevorzugt für Wasserstoff.
- R⁴: steht besonders bevorzugt für 3-Pyridyl, welches einfach oder mehrfach substituiert ist durch Reste aus der Reihe Halogen, C₁-C₆-Alkyl, C₁-C₆-Haloalkyl, C₃-C₆-Cycloalkyl, C₃-C₆-Halocycloalkyl, C₃-C₆-Cycloalkyl-C₁-C₆-alkyl, C₁-C₆-Alkoxy, C₁-C₆-Alkylaminocarbonyl und C₃-C₆-Cycloalkylaminocarbonyl.
- R⁴: steht weiterhin besonders bevorzugt für Pyrazolyl (bevorzugt 3-Pyrazolyl), welches substituiert sein kann durch Halogen (bevorzugt F, Cl, Br) und C₁-C₆-Alkyl (bevorzugt Methyl, Ethyl und Propyl), Phenyl (welches selbst wiederum substituiert sein kann durch Halogen) oder durch Pyrazolylmethyl (welches selbst wieder substituiert sein kann durch C₁-C₆-Halogenalkyl, insbesondere fluoriertes C₁-C₆-Akyl).
- R⁵: steht besonders bevorzugt für einen Rest aus der Reihe Phenyl, 2-Pyridyl und 3-Pyridyl (insbesondere für Phenyl), welcher jeweils einfach oder mehrfach, substituiert ist durch mindestens einen Rest aus der Reihe Halogen, C₁-C₆-Alkyl, C₁-C₆-Haloalkyl, C₁-C₆-Alkoxy, C₁-C₆-Haloalkoxy, Nitro, Cyano, C₁-C₆-Alkylsulfanyl, C₁-C₆-Alkylsulfinyl, C₁-C₆-Alkylsulfonyl, C₁-C₆-Haloalkylsulfanyl, C₁-C₆-Haloalkylsulfinyl, C₁-C₆-Haloalkylsulfonyl, Hetaryloxy-C₁-C₆-alkyl, Hetaryloxy und Hetaryl-C₁-C₆-alkyl, wobei der Hetarylrest ausgewählt ist aus der Reihe Pyrrolyl, Imidazolyl, Pyrazolyl, Triazolyl, Pyridyl, Pyrimidinyl und Tetrazolyl und selbst wiederum substituiert sein kann durch Substituenten aus der Reihe Halogen, Cyano, C₁-C₆-Alkyl, C₁-C₆-Haloalkyl, C₃-C₈-Cycloalkyl und C₃-C₈-Halocycloalkyl.
- R⁵: steht auch besonders bevorzugt für einen Rest aus der Reihe Wasserstoff, Halogen, C₁-C₄-Alkyl und C₃-C₆-Cycloalkyl.
- R⁶: steht besonders bevorzugt für einen Rest aus der Reihe Wasserstoff, Halogen, C₁-C₆-Alkyl, C₁-C₆-Haloalkyl, C₃-C₆-Cycloalkyl, C₁-C₆-Akylsulfanyl und Phenyl, welches durch Halogen substituiert sein kann.
- X: steht besonders bevorzugt für Sauerstoff.
- R¹: steht hervorgehoben für einen Rest aus der Reihe CH₃, CH₂CH₃, CH(CH₃)₂, Cyclopropyl, Halocyclopropyl, C(CH₃)₃, CH(CH₃)CH₂SCH₃, CH₂CH₂CH₃, CH(CH₃)CH₂CH₃, CH₂CF₃, CHCH₃CF₃, CH(CH₃)Cyclopropyl und besonders hervorgehoben für Cyclopropyl.
- R²: steht hervorgehoben für Wasserstoff.
- R³: steht hervorgehoben für Wasserstoff.
- R⁴: steht hervorgehoben für 3-Pyridyl, welches in der 2-Position substituiert ist durch einen Substituenten aus der Reihe F, Cl, Br, Methyl, Ethyl, CF₃, CH₂CF₃, CHFCH₃, CHFCF₃, Cyclopropyl und Cyclopropylmethyl.
- R⁴: steht weiterhin hervorgehoben für Pyrazolyl (bevorzugt 3-Pyrazolyl), welches substituiert sein kann durch F, Cl, Methyl, Ethyl oder durch Pyrazolylmethyl, welches selbst wiederum substituiert sein kann durch C₁-C₆-Halogenalkyl, insbesondere durch CF₃.
- R⁵: steht hervorgehoben für Phenyl, welches einfach oder mehrfach substituiert ist durch einen Rest aus der Reihe CH₃, F, Cl, CF₃, CF(CF₃)₂ OCH₃. OCF₃, NO₂, CN, SCF₃, S(O)CF₃ und S(O)₂CF₃ und CH₂-Q.
- Q: steht für einen Rest aus der Reihe 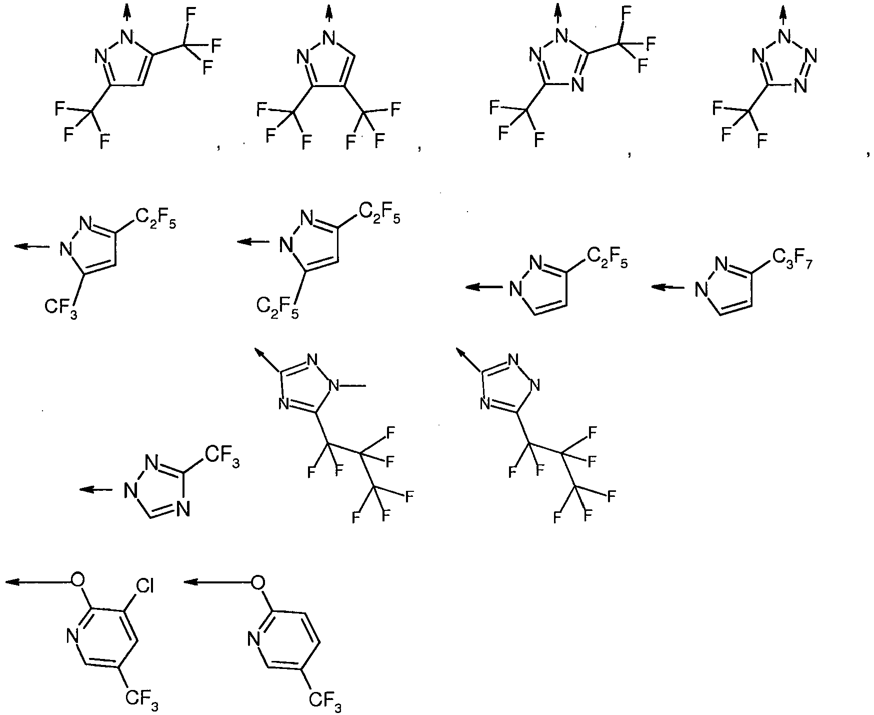
wobei der Pfeil die Stelle der Verknüpfung mit der CH₂-Gruppe angibt.
- R⁶: steht hervorgehoben für Wasserstoff.

In den zuerst genannten Definitionen ist, sofern nichts anderes angegeben ist,

Halogen (auch in Resten wie beispielsweise Haloalkyl) ausgewählt aus der Reihe Fluor, Chlor, Brom und Iod, bevorzugt wiederum aus der Reihe Fluor, Chlor und Brom,

Heteroaryl bzw. Hetaryl (auch als Teil einer größeren Einheit, wie beispielsweise Heteroarylalkyl) ausgewählt aus der Reihe Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Tetrazoyle, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl,

Heterocyclyl (auch als Teil einer größeren Einheit, wie beispielsweise Heterocyclylalkyl) ausgewählt aus der Reihe 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5 Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,3-Dihydrothien-2-yl, 2,3-Dihydrothien-3-yl, 2,4-Dihydrothien-2-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl.

In den bevorzugten Definitionen ist, sofern nichts anderes angegeben ist,
Halogen ausgewählt aus der Reihe Fluor, Chlor, Brom und Iod, bevorzugt wiederum aus der Reihe Fluor, Chlor und Brom,
Heteroaryl bzw. Hetaryl (auch als Teil einer größeren Einheit, wie beispielsweise Hetarylalkyl) ausgewählt aus der Reihe Pyrimidyl, Oxadiazolyl, Oxazolyl, Pyrazinyl, Imidazolyl, Thiazolyl und Furanyl, Pyrrolyl, Pyrazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Tetrazolyl,
Heterocyclyl (auch als Teil einer größeren Einheit, wie beispielsweise Heterocyclylalkyl) ausgewählt aus der Reihe 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5 Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,3-Dihydrothien-2-yl, 2,3-Dihydrothien-3-yl, 2,4-Dihydrothien-2-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl.

Durch Halogen substituierte Reste, z.B. Haloalkyl, sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.

Die oben aufgeführten allgemeinen oder in Vorzugsbereiche aufgeführten Restedefinitionen bzw. Erläuterungen gelten für die Endprodukte und für die Ausgangsprodukte und Zwischenprodukte entsprechend. Diese Restedefinitionen können untereinander also auch zwischen den jeweiligen Vorzugsbereichen, beliebig kombiniert werden.

Erfindungsgemäß bevorzugt werden Verbindungen der Formel (I), in welchen eine Kombination der vorstehend als bevorzugt aufgeführten Bedeutungen vorliegt.

Erfindungsgemäß besonders bevorzugt werden Verbindungen der Formel (I), in welchen eine Kombination der vorstehend als besonders bevorzugt aufgeführten Bedeutungen vorliegt.

Erfindungsgemäß hervorgehoben sind Verbindungen der Formel (I), in welchen eine Kombination der vorstehend als hervorgehoben aufgeführten Bedeutungen vorliegt.

In einer bevorzugten Gruppe von Verbindungen der Formel (I) steht R¹ für Cyclopropyl.

In einer weiteren bevorzugten Gruppe von Verbindungen der Formel (I) steht R⁷ für Ethyl.

In einer weiteren bevorzugten Gruppe von Verbindungen der Formel (I) steht R⁷ für CH₂CF₃.

In einer weiteren bevorzugten Gruppe von Verbindungen der Formel (I) steht R⁵ für gegebenenfalls substituiertes Phenyl.

In einer weiteren bevorzugten Gruppe von Verbindungen der Formel (I) steht R⁵ für gegebenenfalls substituiertes 3-Pyridyl.

Setzt man bei dem erfindungsgemäßen Verfahren 1 zur Herstellung der neuen Verbindungen der Formel (I) als Verbindung der Formel (II) beispielsweise 6-(4-Chlor-phenyl)-N-Cyclopropyl-pyrimidin-4,5-diamin und als Verbindung der Formel (III) 2-Ethyl-nicotinoylchlorid ein, so lässt sich das Verfahren 1 durch folgendes Reaktionsschema I wiedergeben:

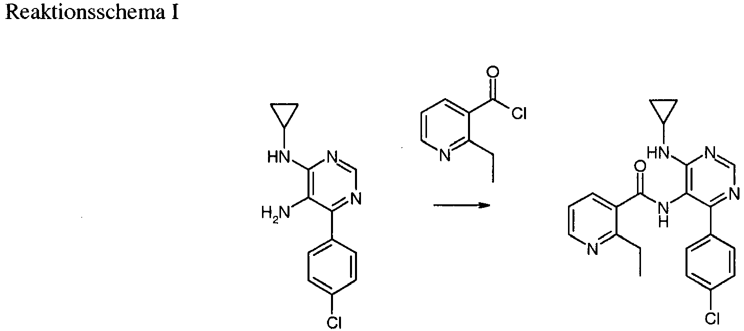

Diese Umsetzung wird im Allgemeinen so durchgeführt, dass man Verbindungen der Formel (II) mit Verbindungen der Formel (III) in Gegenwart eines basischen Hilfsmittels in einem Verdünnungsmittel bei einer Temperatur von -40°C bis 120°C umsetzt.

Als basische Hilfsmittel kommen Amine, beispielsweise Trialkylamine oder Pyridine in Frage. Genannt sei beispielsweise Pyridin.

Im Allgemeinen ist es vorteilhaft, die Verbindungen der Formel (II) mit Verbindungen der Formel (III) in einem Verdünnungsmittel umzusetzen. Hierbei können gegebenenfalls auch Gemische von Verdünnungsmitteln eingesetzt werden. Verdünnungsmittel werden vorteilhaft in einer solchen Menge eingesetzt, dass das Reaktionsgemisch während des ganzen Verfahrens gut rührbar bleibt. Als Verdünnungsmittel zur Durchführung des erfindungsgemäßen Verfahrens 1 kommen unter den Reaktionsbedingungen inerte organischen Lösungsmittel in Frage.

Als Beispiele sind zu nennen: Halogenkohlenwasserstoffe, insbesondere Chlorkohlenwasserstoffe, wie Tetrachlorethylen, Tetrachlorethan, Dichlorpropan, Methylenchlorid (DCM), Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol; Ether wie Ethylpropylether, Methyl-tert-butylether, n-Butylether, Anisol, Phenetol, Cyclohexylmethylether, Dimethylether, Diethylether, Dipropylether, Düsopropylether, Di-n-butylether, Diisobutylether, Düsoamylether, Ethylenglycoldimethylether, Tetrahydrofuran, Dioxan, Dichlordiethylether und Polyether des Ethylenoxids und/oder Propylenoxids.

Nach vollendeter Umsetzung wird der gesamte Reaktionsansatz eingeengt. Die nach Aufarbeitung anfallenden Produkte lassen sich in üblicher Weise durch Umkristallisieren, Vakuumdestillation oder Säulenchromatographie reinigen (vgl. auch die Herstellungsbeispiele).

Die Verbindungen der Formel (II) können z. T. kommerziell oder nach literaturbekannten Methoden erhalten werden (vgl. Journal of Combinatorial Chemistry (2003), 5(5), 653-659, und auch die Herstellungsbeispiele).

Die weiterhin für die Durchführung des erfindungsgemäßen Verfahrens 1 als Ausgangsstoffe zu verwendenden Verbindungen werden durch die Formel (III) beschrieben.

In der Formel (III) hat R⁴ die Bedeutungen, die bereits im Zusammenhang mit der Beschreibung der erfindungsgemäßen Stoffe der Formel (I) genannt wurden.

Die Verbindungen der Formel (III) sind zum Teil bekannt und können kommerziell oder nach literaturbekannten Methoden erhalten werden (vgl. Chemical Communications (2008), 4207-4209, und Methods of Organic Synthesis (2002) Volume E22a, Ed. Murray Goodman, JP401316361A sowie Mendeleev Communications (2006) (3), 161 - 163).

Setzt man bei dem erfindungsgemäßen Verfahren 2 zur Herstellung der Verbindungen der Formel (I) als Verbindung der Formel (IV) beispielsweise N-(4-Chlor-6-Cyclopropylamino-pyrimidin-5-yl)-2-ethyl-nicotinamid und als Verbindung der Formel (V) 2,4-Dichlorphenylboronsaure lässt sich das Verfahren durch folgendes Reaktionsschema II wiedergeben:

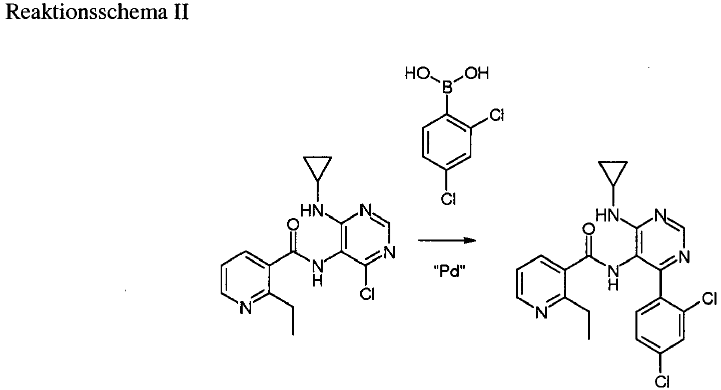

Die Verbindung der Formel (IV) wird in einem geeigneten Verdünnungsmittel wie z. B. Akoholen (z.B. Methanol oder Ethanol), Ethern (z.B. Diethylether oder Dioxan) oder Wasser oder einer Mischung dieser Verdünnungsmittel (beispielsweise Dioxan/Wasser) gegebenenfalls in Gegenwart einer Base und in Gegenwart von geeigneten Palladium-Katalysatoren und Basen im Temperaturbereich von -20 °C bis 120°C in geeigneten Lösungsmitteln zur Reaktion gebracht (Suzuki-Reaktion).

Geeignete Basen sind anorganische oder organische Basen, insbesondere Carbonate, wie beispielsweise Natriumcarbonat.

Es können eine Vielzahl von Palladiumkatalysatoren verwendet werden. Vorzugsweise verwendet man einen Katalysator aus der Reihe PdCl₂(dppf) [dppf = 1,1'-Bis(diphenylphosphino) ferrocene], Pd(PPh₃)₄, PdCl₂(PPh₃)₂, PdCl₂(CH₃CN)₂, Pd₂(dba)₃ [dba = Dibenzylidenaceton] oder Pd(OAc)₂, besonders bevorzugt Pd(PPh₃)₄.

Die Verbindungen der Formel (IV) sind zum Teil bekannt und lassen sich nach bekannten Methoden herstellen, beschrieben z.B. in Tetrahedron Letters, 49(13), 2143-2145; 2008.

Die Boronsäuren bzw. Boronsäureester der Formel (V) sind zum Teil kommerziell verfügbar oder sie sind nach bekannten Methoden herstellbar. Dies ist beispielsweise beschrieben in WO 1999/64428.

Nach vollendeter Umsetzung wird der gesamte Reaktionsansatz eingeengt. Die nach Aufarbeitung anfallenden Produkte lassen sich in üblicher Weise durch Umkristallisieren, Vakuumdestillation oder Säulenchromatographie reinigen (vgl. auch die Herstellungsbeispiele).

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..
Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.
Aus der Klasse der Bivalva z.B. Dreissena spp..
Aus der Ordnung der Chilopoda z.B. Geophilus spp., Scutigera spp..
Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..
Aus der Ordnung der Collembola z.B. Onychiurus armatus.
Aus der Ordnung der Dermaptera z.B. Forficula auricularia.
Aus der Ordnung der Diplopoda z.B. Blaniulus guttulatus.
Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.
Aus der Klasse der Gastropoda z.B. Arion spp., Biomphalaria spp., Bulinus spp., Deroceras spp., Galba spp., Lymnaea spp., Oncomelania spp., Succinea spp..
Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis tlammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp.. Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-FettalkoholEther, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, AlkylAryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat; sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Insbesondere eignen sich die erfindungsgemäßen Wirkstoffe zur Behandlung von Saatgut. So entsteht ein großer Teil des durch Schädlinge verursachten Schadens an Kulturpflanzen bereits durch den Befall des Saatguts während der Lagerung und nach dem Einbringen des Saatguts in den Boden sowie während und unmittelbar nach der Keimung der Pflanzen. Diese Phase ist besonders kritisch, da die Wurzeln und Sprosse der wachsenden Pflanze besonders empfindlich sind und bereits ein geringer Schaden zum Absterben der ganzen Pflanze führen kann. Es besteht daher ein insbesondere großes Interesse daran, das Saatgut und die keimende Pflanze durch den Einsatz geeigneter Mittel zu schützen.

Die Bekämpfung von Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen. Es ist weiterhin erstrebenswert, die Menge des eingesetzten Wirkstoffs dahingehend zu optimieren, dass das Saatgut und die keimende Pflanze vor dem Befall durch Schädlinge bestmöglich geschützt wird, ohne jedoch die Pflanze selbst durch den eingesetzten Wirkstoff zu schädigen. Insbesondere sollten Verfahren zur Behandlung von Saatgut auch die intrinsischen insektiziden Eigenschaften transgener Pflanzen einbeziehen, um einen optimalen Schutz des Saatguts und auch der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der daraus entstehenden Pflanze vor Schädlingen. Weiterhin bezieht sich die Erfindung auf Saatgut, welches zum Schutz vor Schädlingen mit einem erfindungsgemäßen Mittel behandelt wurde.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor Schädlingen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Wirkstoffe insbesondere auch bei transgenem Saatgut eingesetzt werden können, wobei die aus diesem Saatgut hervorgehenden Pflanzen zur Expression eines gegen Schädlinge gerichteten Proteins befähigt sind. Durch die Behandlung solchen Saatguts mit den erfindungsgemäßen Mitteln können bestimmte Schädlinge bereits durch die Expression des z.B. insektiziden Proteins kontrolliert werden, und zusätzlich durch die erfindungsgemäßen Mittel vor Schäden bewahrt werden.

Die erfindungsgemäßen Mittel eignen sich zum Schutz von Saatgut jeglicher Pflanzensorte wie bereits vorstehend genannt, die in der Landwirtschaft, im Gewächshaus, in Forsten oder im Gartenbau eingesetzt wird. Insbesondere handelt es sich dabei um Saatgut von Mais, Erdnuss, Canola, Raps, Mohn, Soja, Baumwolle, Rübe (z.B. Zuckerrübe und Futterrübe), Reis, Hirse, Weizen, Gerste, Hafer, Roggen, Sonnenblume, Tabak, Kartoffeln oder Gemüse (z.B. Tomaten, Kohlgewächs).

Die erfindungsgemäßen Mittel eignen sich ebenfalls zur Behandlung des Saatguts von Obstpflanzen und Gemüse wie vorstehend bereits genannt. Besondere Bedeutung kommt der Behandlung des Saatguts von Mais, Soja, Baumwolle, Weizen und Canola oder Raps zu.

Wie vorstehend bereits erwähnt, kommt auch der Behandlung von transgenem Saatgut mit einem erfindungsgemäßen Mittel eine besondere Bedeutung zu. Dabei handelt es sich um das Saatgut von Pflanzen, die in der Regel zumindest ein heterologes Gen enthalten, das die Expression eines Polypeptids mit insbesondere insektiziden Eigenschaften steuert. Die heterologen Gene in transgenem Saatgut können dabei aus Mikroorganismen wie Bacillus, Rhizobium, Pseudomonas, Serratia, Trichoderma, Clavibacter, Glomus oder Gliocladium stammen. Die vorliegende Erfindung eignet sich besonders für die Behandlung von transgenem Saatgut, das zumindest ein heterologes Gen enthält, das aus Bacillus sp. stammt und dessen Genprodukt Wirksamkeit gegen Maiszünsler und/oder Maiswurzel-Bohrer zeigt. Besonders bevorzugt handelt es sich dabei um ein heterologes Gen, das aus Bacillus thuringiensis stammt.

Im Rahmen der vorliegenden Erfindung wird der erfindungsgemäße Wirkstoff alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele für (transgene) Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready@ (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der Formel (I) behandelt werden. Die bei den Wirkstoffen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bäd verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, O-piliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Pemphigus spp., Phylloera vastatrix, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Herstellungsbeispiele:

### Beispiel 1 (Vorstufe)

### 6-Chlor-N-methyl-pyrimidin-4,5-diamin

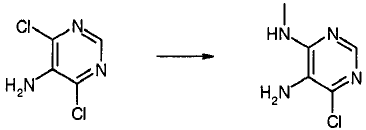

5-Amino-4,6-dichlorpyrimidin (15.0 g, 91.4 mmol) und Methylamin (2M Lösung in THF, 5 eq) wurden vorgelegt und mit Triethylamin (27.76 g, 274 mmol) versetzt. Der Ansatz wurde bis zur vollständigen Umsetzung unter Rückfluß gerührt (ca. 3 Stunden). Anschließend wurde das Reaktionsgemisch eingeengt und der Rückstand mit Wasser versetzt. Mit gesättigter NaHCO₃-Lösung wurde ein pH-Wert von 7-8 eingestellt. Man extrahierte 3x mit Methylenchlorid, trocknete die vereinigten organischen Phasen über Natriumsulfat, filtrierte und entfernte das Lösungsmittel im Vakuum.
Ausbeute: 12.0 g (82%)
LC-MS: M+(ES+)159(100%)
NMR DMSO 2.89 (3H, m), 4.77 (2H, s), 6.71 (1H, s), 7.75 (1H, s).

### Beispiel 2 (vorstufe)

### N-(4-Chlor-6-Cyclopropylamino-pyrimidin-5-yl)-2-ethyl-nicotinamid

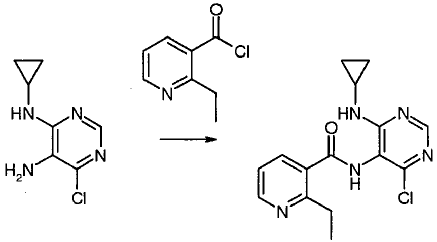

6-Chlor-N-Cyclopropyl-pyrimidin-4,5-diamin (vgl. Journal of Combinatorial Chemistry (2003), 5(5), 653-659) (3.0 g, 16.2 mmol) wurde in einem Gemisch von Methylenchlorid (12.20 ml) und Pyridin (3.94 ml) vorgelegt. Die Suspension wurde auf 0°C abgekühlt und 2-Ethyl-nicotinoylchlorid (1.2 eq) als Feststoff zugeben. Man ließ das Reaktionsgemisch auf Raumtemperatur kommen und rührte bis zur vollständigen Umsetzung. Anschließend gab man 10 ml Methyl-enchlorid zu, wobei die Suspension in eine Lösung übergeht. Die organische Phase wurde mit ca. 10 ml halbgesättigter NaHCO₃-Lösung versetzt, dabei wurde mit einem Magnetrührer in einem Kolben kräftig gerührt, es fielen schwerlösliche Kristalle (Produkt) aus, die abgesaugt, 3x mit etwas Wasser und 3x mit n-Heptan gewaschen und getrocknet wurden.
Fraktion 1: 3.41g, 66%
LC/MS:M+(ES+)=318 (100%)
NMR 0.56 (2H, m), 0.76 (2H, m), 1.25 (3H, t), 2.87 (1H, m), 2.96 (2H, q), 7.22 (1H, s), 7.32 (1H, m), 8.10 (1H, m), 8.30 (1H, m), 8.59 (1H, m), 9.55 (1H, m).

### Beispiel 3 (Endstufe)

### N-[4-Cyclopropylamino-6-(2,4-dichlor-phenyl)-pyrimidin-5-yl]-2-ethyl-nicotinamid

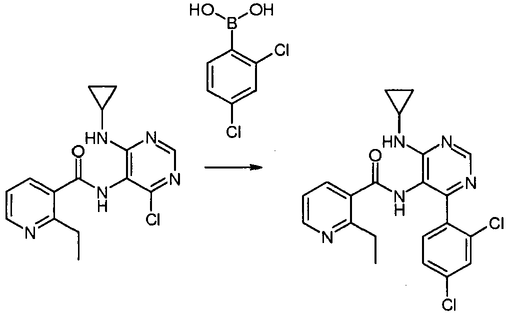

In einem ausgeheizten und bei 100°C vorgeheizten Reaktionsröhrchen wurden N-(4-Chlor-6-Cyclopropylamino-pyrimidin-5-yl)-2-ethyl-nicotinamid (0.41 mmol), Natriumcarbonat (3 eq) und 2,4-Dichlorphenylboronsaure (2 eq) mit dem entgasten Verdünnungsmittelgemisch Dioxan (2 ml) / Wasser (0.7 ml) versetzt. Es wurden 5 mol% Tetrakis(triphenylphosphin)palladium(0) zugegeben und das Reaktionsgemisch 3 Stunden lang bei 90°C gerührt. Anschließend wurde mit Wasser verdünnt, 3x mit Methylenchlorid extrahiert, die vereinten organischen Phasen mit wässriger NaHCO₃-Lösung gewaschen, über Na₂SO₄ getrocknet, filtriert und im Vakuum eingeengt.
LC/MS: M+(ES+)=430 (50%) NMR DMSO 0.57 (2H, m), 0.79 (2H, m), 1.03 (3H, t), 2.91 (1H, m), 6.86 (1H, m), 7.263 (1H, m), 7.44 (2H, m), 7.61 (1H, m), 7.72 (1H, m), 8.50 (2H, m), 9.33 (1H, s).

Der Rückstand wurde am Companion mit einer Kartusche Chromabond Flash RS 70 SiOH unter Verwendung von Methylenchlorid/MeOH getrennt.
LC/MS: M+(ES+)=428 (100%)

### Beispiel 4 (Vorstufe)

### 6-(4-Chlor-phenyl)-N-Cyclopropyl-pyrimidin-4,5-diamin

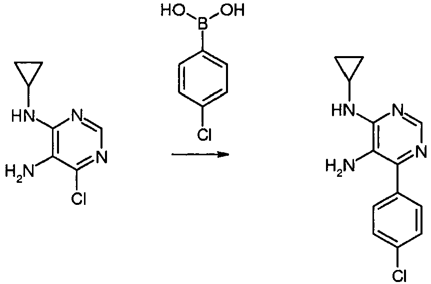

In einem ausgeheizten und bei 100°C vorgeheizten Reaktionsröhrchen eines Reaktorkarussels wurden 6-Chlor-*N*-Cyclopropyl-pyrimidin-4,5-diamin (10 g, 54.1 mmol), Natriumcarbonat (17.22 g, 162 mmol) vorgelegt und mit dem entgasten Lösungsmittelgemisch Dioxan (20 ml) / Wasser (7 ml) versetzt. Es wurden 5 mol% Tetrakis(triphenylphosphin)palladium(0) und 4-Chlorphenylboronsaure (108,2 mmol) zugegeben und das Reaktionsgemisch über Nacht bei 90°C gerührt. Die Lösung wurde abgekühlt, filtriert, eigeengt und der Rückstand aus Acetonitril umkristallisiert.
Ausbeute: 8193 mg; 58%
LC-MS: M+(ES)=261 (100%)
NMR DMSO 0.52 (2H, m), 0.53 (2H, m), 2.89 (1H, m), 4.52 (1H, s), 6.76 (1H, s), 7.47 (2H, m), 7.65 (2H, m), 8.04 1H, s).

### Beispiel 5 (Endstufe)

### N-[4-(4-Chlor-phenyl)-6-Cyclopropylamino-pyrimidin-5-yl]-2-ethyl-nicotinamid

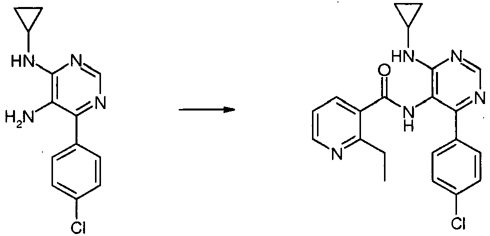

6-(4-Chlor-phenyl)-N-Cyclopropyl-pyrimidin-4,5-diamin (1.00g, 3.83 mmol) wurden in einem Gemisch aus DCM (3.20 ml) und Pyridin (0.93 ml) vorgelegt und auf 0°C gekühlt. Man gab 2-Ethyl-nicotinoylchlorid (780 mg, 4.60 mmol) als Feststoff zu, ließ das Reaktionsgemisch auf Raumtemperatur erwärmen und rührte noch 1 Stunde. Nach Zugabe von halbgesättigter NaHCO₃-Lösung wurde gerührt, die organische Phase abgetrennt und im Vakuum eingeengt.

Der Rückstand wurde am Companion mit einer Kartusche Chromabond Flash RS 120 SiOH unter Verwendung von DCM/MeOH getrennt.
Fraktion 1: 394 mg; 25%
LC/MS: M+(ES+)=394 (98%) NMR DMSO 0.55 (2H, m), 0.78 (2H, m), 1.063 (3H, t), 2.89 (1H, m), 6.94 (1H, s), 7.28 (1H, m), 7.45 (2H, d), 7.67 (2H, d), 7.88 (1H, d), 8.52 (2H, m), 9.37 (1H, s).

### Beispiel 6

### 6-Chlor-N⁴-[4-(trifluoromethyl)phenyl]pyrimidin-4,5-diamin

### (Vgl. auch Tetrahedron Letters., 48, 1489 (2007))

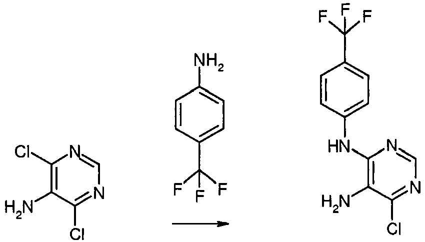

Eine Mischung von 4,6-Dichloroaminopyrimidin (1.5g), Isopropanol (20ml), 4-Trifluormethylanilin (1.47g) und einem Tropfen konz. wässriger Salzsäure wurde für 10 min bei 150°C in einem Mikrowellengefäß erhitzt. Nach dem Abkühlen wurde der entstandene Niederschlag abfiltriert.
Ausbeute 580 mg.

In der folgenden Tabelle sind weitere erfindungsgemäße Verbindungen aufgeführt.

Es wurden folgende Abkürzungen verwendet:
Ph = Phenyl = Ar

R in der Spalte R¹ (vgl. z.B. Bsp.Nr. 1-222, 1-229, 1-230, 1-250, 3-7 usw.) kennzeichnet die Verknüpfungsstelle zum Rest des Moleküls.

**Tabelle 1**

| | | | | | | | | | | |
|---|---|---|---|---|---|---|---|---|---|---|
| Verbindungen der Formel | | 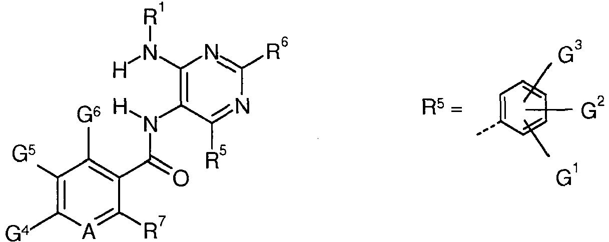 | | | | | | | | |

| **Bsp. Nr.** | **R¹** | **R⁷** | **G¹** | **G²** | **G³** | **R⁶** | **A** | **G⁴** | **G⁵** | **G⁶** |
|---|---|---|---|---|---|---|---|---|---|---|
| 1-1 | Cyclopropyl | CH₃ | 4-F | H | H | H | N | H | H | H |
| 1-2 | Cyclopropyl | CH₃ | 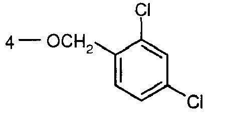 | H | H | H | N | H | H | H |
| 1-3 | Cyclopropyl | CH₃ | 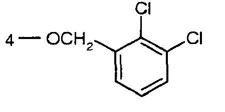 | H | H | H | N | H | H | H |
| 1-4 | Cyclopropyl | CH₂CH₃ | 3-F | 4-F | H | H | N | H | H | H |
| 1-5 | Cyclopropyl | CH₂CH₃ | 2-F | 4-F | H | H | N | H | H | H |
| 1-6 | Cyclopropyl | CH₂CH₃ | 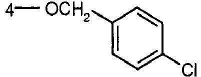 | H | H | H | N | H | H | H |
| 1-7 | Cyclopropyl | CH₂CH₃ | 4-OCH₃ | H | H | H | N | H | H | H |
| 1-8 | Cyclopropyl | CH₂CH₃ | 4-CH₃ | H | H | H | N | H | H | H |
| 1-9 | Cyclopropyl | CH₂CH₃ | 2-F | 3-F | H | H | N | H | H | H |
| 1-10 | Cyclopropyl | CH₂CH₃ | 4-SCH₃ | H | H | H | N | H | H | H |
| 1-11 | Cyclopropyl | CH₂CH₃ | 2-F | H | H | H | N | H | H | H |
| 1-12 | Cyclopropyl | CH₂CH₃ | 3,4-OCH₂CH₂-O- | | H | H | N | H | H | H |
| 1-13 | Cyclopropyl | CH₂CH₃ | 3-Cl | H | H | H | N | H | H | H |
| 1-14 | Cyclopropyl | CH₂CH₃ | 3-NH₂ | H | H | H | N | H | H | H |
| 1-15 | Cyclopropyl | CH₂CH₃ | 3-F | 4-F | 5-F | H | N | H | H | H |
| 1-16 | Cyclopropyl | CH₂CH₃ | 2-F | 3-F | 4-F | H | N | H | H | H |
| 1-17 | Cyclopropyl | CH₂CH₃ | 4-COCH₃ | H | H | H | N | H | H | H |
| 1-18 | Cyclopropyl | CH₂CH₃ | 4-C(CH₃)₃ | H | H | H | N | H | H | H |
| 1-19 | Cyclopropyl | CH₂CH₃ | 4-N(CH₃)₂ | H | H | H | N | H | H | H |
| 1-20 | Cyclopropyl | CH₂CH₃ | 3-Cl | 4-F | H | H | N | H | H | H |
| 1-21 | CH(CH₃)₂ | CH₂CH₃ | 4-F | H | H | H | N | H | H | H |
| 1-22 | CH(CH₃)₂ | CH₂CH₃ | 4-OCF₃ | H | H | H | N | H | H | H |
| 1-23 | CH(CH₃)CH₃SCH₃ | CH₂CH₃ | 4-OCF₃ | H | H | H | N | H | H | H |
| 1-24 | CH(CH₃)CH₂SCH₃ | CH₂CH₃ | 4-F | H | H | H | N | H | H | H |
| 1-25 | Cyclopropyl | CH₂CH₃ | 2-F | 3-Cl | H | H | N | H | H | H |
| 1-26 | Cyclopropyl | CH₂CH₃ | 3,4-OCF₂O | | H | H | N | H | H | H |
| 1-27 | Cyclopropyl | CH₂CH₃ | 3-CH₃ | 4-F | H | H | N | H | H | H |
| 1-28 | Cyclopropyl | CH₂CH₃ | 3-C(CH₃)NOCH₃ | H | H | H | N | H | H | H |
| 1-29 | Cyclopropyl | CH₂CH₃ | 3-CHNOCH₃ | H | H | H | N | H | H | H |
| 1-30 | Cyclopropyl | CH₂CH₃ | 2-F | 5-CF₃ | H | H | N | H | H | H |
| 1-31 | Cyclopropyl | CH₂CH₃ | CONH₂ | H | H | H | N | H | H | H |
| 1-32 | Cyclopropyl | CH₂CH₃ | 3,4-OCH₂CH₂O- | | H | H | N | H | H | H |
| 1-33 | Cyclopropyl | CH₂CH₃ | 3-NH₂ | OCH₃ | 5-NH₂ | H | N | H | H | H |
| 1-34 | Cyclopropyl | CH₂CH₃ | 4-CO₂CH₃ | H | H | H | N | H | H | H |
| 1-35 | Cyclopropyl | CH₂CH₃ | 3-SCH₃ | H | H | H | N | H | H | H |
| 1-36 | Cyclopropyl | CH₂CH₃ | 4-CH₂OCH₃ | H | H | H | N | H | H | H |
| 1-37 | Cyclopropyl | CH₂CH₃ | 2-F | 4-F | 5-Cl | H | N | H | H | H |
| 1-38 | Cyclopropyl | CH₂CH₃ | 3-CH₂OCH₃ | H | H | H | N | H | H | H |
| 1-39 | Cyclopropyl | CH₂CH₃ | 4-NHCOCH₃ | H | H | H | N | H | H | H |
| 1-40 | Cyclopropyl | CH₂CH₃ | 3-F | 4-NH₂ | H | H | N | H | H | H |
| 1-41 | Cyclopropyl | CH₂CH₃ | 3-F | 4-OCH₃ | H | H | N | H | H | H |
| 1-42 | Cyclopropyl | CH₂CH₃ | 3-F | 4-CH₃ | H | H | N | H | H | H |
| 1-43 | Cyclopropyl | CH₂CH₃ | 2-F | 4-CF3 | H | H | N | H | H | H |
| 1-44 | Cyclopropyl | CH₂CH₃ | 3-F | 4-Cl | H | H | N | H | H | H |
| 1-45 | Cyclopropyl | CH₂CH₃ | 2-F | 5-COCH₃ | H | H | N | H | H | H |
| 1-46 | Cyclopropyl | CH₂CH₃ | 3-F | 5-OCH₃ | H | H | N | H | H | H |
| 1-47 | Cyclopropyl | CH₂CH₃ | 2-F | 5-CH₃ | H | H | N | H | H | H |
| 1-48 | Cyclopropyl | CH₂CH₃ | 2-F | 4-OCH₃ | H | H | N | H | H | H |
| 1-49 | Cyclopropyl | CH₂CH₃ | 3-F | 5-Cl | H | H | N | H | H | H |
| 1-50 | Cyclopropyl | CH₂CH₃ | 3-S(O)₂CH₃ | H | H | H | N | H | H | H |
| 1-51 | Cyclopropyl | CH₂CH₃ | 3-CN | 4-F | H | H | N | H | H | H |
| 1-52 | Cyclopropyl | CH₂CH₃ | 2-F | 3-OCH₃ | H | H | N | H | H | H |
| 1-53 | Cyclopropyl | CH₂CH₃ | 3-Si(CH₃)₃ | H | H | H | N | H | H | H |
| 1-54 | Cyclopropyl | CH₂CH₃ | 2-OCH₃ | 4-F | H | H | N | H | H | H |
| 1-55 | Cyclopropyl | CH₂CH₃ | 3-NO₂ | H | H | H | N | H | H | H |
| 1-56 | Cyclopropyl | CH₂CH₃ | 2-Cl | 5-Cl | H | H | N | H | H | H |
| 1-57 | Cyclopropyl | CH₂CH₃ | 3-CH₃ | 5-CH₃ | H | H | N | H | H | H |
| 1-58 | Cyclopropyl | CH₂CH₃ | 3-Ar | H | H | H | N | H | H | H |
| 1-59 | Cyclopropyl | CH₂CH₃ | 2-Cl | 5-Cl | H | H | N | H | H | H |
| 1-60 | Cyclopropyl | CH₂CH₃ | 4-CH₂CH₂ | H | H | H | N | H | H | H |
| 1-61 | Cyclopropyl | CH₂CH₃ | 2-Cl | 3-Cl | H | H | N | H | H | H |
| 1-62 | Cyclopropyl | CH₂CH₃ | 4-Ar | H | H | H | N | H | H | H |
| 1-63 | Cyclopropyl | CH₂CH₃ | 3-CH₃ | 4-CH₃ | H | H | N | H | H | H |
| 1-64 | Cyclopropyl | CH₂CH₃ | 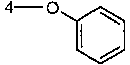 | H | H | H | N | H | H | H |
| 1-65 | Cyclopropyl | CH₂CH₃ | 2-F | 4-OCH₃ | 5-F | H | N | H | H | H |
| 1-66 | Cyclopropyl | CH₂CH₃ | 3-NH(SO₂)CH₃ | H | H | H | N | H | H | H |
| 1-67 | Cyclopropyl | CH₂CH₃ | 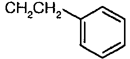 | H | H | H | N | H | H | H |
| 1-68 | Cyclopropyl | CH₂CH₃ | 4-OCH(CH₃) | H | H | H | N | H | H | H |
| 1-69 | Cyclopropyl | CH₂CH₃ | 3-Cl | 4-Cl | 5-Cl | H | N | H | H | H |
| 1-70 | Cyclopropyl | CH₂CH₃ | 4-NO₂ | H | H | H | N | H | H | H |
| 1-71 | Cyclopropyl | CH₂CH₃ | 3-F | 5-NH₂ | H | H | N | H | H | H |
| | | | | | | | | | | |
| 1-73 | Cyclopropyl | CH₂CH₃ | 4-OCF₃ | H | H | H | N | H | H | H |
| 1-74 | CH(CH₃)₂ | CH₃ | 4-OCF₃ | H | H | H | N | H | H | H |
| 1-75 | Cyclopropyl | Cl | 4-OCF₃ | H | H | H | N | H | H | H |
| 1-76 | CH(CH₃)₂ | Cl | 4-OCF₃ | H | H | H | N | H | H | H |
| | | | | | | | | | | |
| | | | | | | | | | | |
| | | | | | | | | | | |
| | | | | | | | | | | |
| | | | | | | | | | | |
| 1-82 | Cyclopropyl | Cl | 4-CF₃ | H | H | H | N | H | H | H |
| 1-83 | Cyclopropyl | CH₃ | 4-CF₃ | H | H | H | N | H | H | H |
| 1-84 | Cyclopropyl | CF₃ | 4-CF₃ | H | H | H | N | H | H | H |
| 1-85 | Cyclopropyl | CH(CH₃)₂ | 4-CF₃ | H | H | H | N | H | H | H |
| | | | | | | | | | | |
| 1-87 | Cyclopropyl | CH₃ | 4-CF₃ | H | H | CH₃ | N | H | H | H |
| | | | | | | | | | | |
| 1-89 | Cyclopropyl | CH₃ | 4-CF₃ | H | H | Cyclopro-pyl | N | H | H | H |
| 1-90 | CH(CH₃)₂ | CH₃ | 4-OCF₃ | H | H | H | N | H | H | H |
| 1-91 | 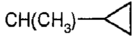 | CH₃ | 4-OCF₃ | H | H | H | N | H | H | H |
| 1-92 | CH(CH₃)CH₂CH₃ | CH₃ | 4-OCF₃ | H | H | H | N | H | H | H |
| 1-93 | Cyclopropyl | CH₃ | 4-OCF₃ | H | H | H | N | H | H | H |
| 1-94 | CH(CH₃)₂ | CH₃ | 4-OCF₃ | H | H | H | N | H | H | H |
| 1-95 | 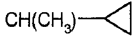 | CH₃ | 4-OCF₃ | H | H | H | N | H | H | H |
| 1-96 | Cyclopropyl | CH₃ | 4-OCF₃ | H | H | H | N | H | H | H |
| 1-97 | Cyclopropyl | CH₃ | 4-OCF₃ | H | H | C(CH₃) | N | H | H | H |
| 1-98 | Cyclopropyl | CH₃ | 4-OCF₃ | H | H | 4-Cl-Ph | N | H | H | H |
| 1-99 | 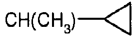 | Cl | 4-OCF₃ | H | H | H | N | H | H | H |
| 1-100 | 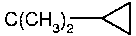 | CH₃ | 4-OCF₃ | H | H | H | N | H | H | H |
| 1-101 | CH(CH₃)CH₂CH₃ | CH₃ | 4-OCF₃ | H | H | H | N | H | H | H |
| 1-102 | CH(CH₃)CH₂CH₃ | Cl | 4-OCF₃ | H | H | H | N | H | H | H |
| 1-103 | 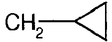 | CH₃ | 4-OCF₃ | H | H | H | N | H | H | H |
| 1-104 | 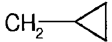 | Cl | 4-OCF₃ | H | H | H | N | H | H | H |
| 1-105 | C(CH₃)₃ | Cl | 4-OCF₃ | H | H | H | N | H | H | H |
| 1-106 | CH(CH₂CH₃)₂ | CH₃ | 4-OCF₃ | H | H | H | N | H | H | H |
| 1-107 | CH(CH₂CH₃)₃ | Cl | 4-OCF₃ | H | H | H | N | H | H | H |
| 1-108 | CH(CH₃)CH(CH₃)₂ | CH₃ | 4-OCF₃ | H | H | H | N | H | H | H |
| 1-109 | CH(CH₃)CH(CH₃)₃ | Cl | 4-OCF₃ | H | H | H | N | H | H | H |
| 1-110 | 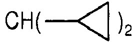 | CH₃ | 4-OCF₃ | H | H | H | N | H | H | H |
| 1-111 | 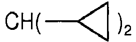 | Cl | 4-OCF₃ | H | H | H | N | H | H | H |
| | | | | | | | | | | |
| | | | | | | | | | | |
| 1-114 | Cyclopropyl | CH₂CH₃ | 4-SCF₃ | H | H | H | N | H | H | H |
| 1-115 | Cyclopropyl | CH₂CH₃ | 3-NH₂ | 4-OCH(CH₃)₂ | H | H | N | H | H | H |
| 1-116 | Cyclopropyl | CH₃ | 4-SCF₃ | H | H | H | N | H | H | H |
| 1-117 | Cyclopropyl | CH₃ | S(O)CF₃ | H | H | H | N | H | H | H |
| 1-118 | Cyclopropyl | CH₃ | S(O)CHF₂ | H | H | H | N | H | H | H |
| 1-119 | Cyclopropyl | CH₃ | 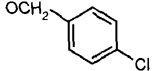 | H | H | H | N | H | H | H |
| 1-120 | Cyclopropyl | CH₃ | OCF₃ | CH₃ | H | H | N | H | H | H |
| 1-121 | Cyclopropyl | CH₃ | 4-OSO₂CH(CH₃)₂ | H | H | H | N | H | H | H |
| 1-122 | Cyclopropyl | CH₃ | 3-F | 4-CF₃ | 5-F | H | N | H | H | H |
| 1-123 | Cyclopropyl | CH₃ | 4-OCF₂CHFCF₃ | 4-CF₃ | H | H | N | H | H | H |
| 1-124 | Cyclopropyl | CH₂CH₃ | 4-F | H | H | H | N | H | H | H |
| 1-125 | CH₃ | CH₂CH₃ | 4-F | H | H | H | N | H | H | H |
| 1-126 | Cyclopropyl | CH₂CH₃ | 2-Cl | 4-Cl | H | H | N | H | H | H |
| 1-127 | Cyclopropyl | CH₂CH₃ | H | H | H | H | N | H | H | H |
| 1-128 | Cyclopropyl | H | H | H | H | H | N | H | H | H |
| 1-129 | Cyclopropyl | Cl | 4-F | H | H | H | N | H | H | H |
| 1-130 | Cyclopropyl | Cl | 4-Cl | H | H | H | N | H | H | H |
| 1-131 | Cyclopropyl | Br | 4-Cl | H | H | H | N | H | H | H |
| 1-132 | Cyclopropyl | Br | 4-F | H | H | H | N | H | H | H |
| 1-133 | Cyclopropyl | CH₂CH₃ | 4-CN | H | H | H | N | H | H | H |
| 1-134 | C(CH₃)₃ | CH₂CH₃ | 4-F | H | H | H | N | H | H | H |
| 1-135 | C(CH₃)₃ | CH₂CH₃ | 4-Cl | H | H | H | N | H | H | H |
| 1-136 | C(CH₃)₃ | CH₂CH₃ | 4-CN | H | H | H | N | H | H | H |
| 1-137 | C(CH₃)₃ | CH₂CH₃ | 4-CF₃ | H | H | H | N | H | H | H |
| 1-138 | C(CH₃)₃ | CH₂CH₃ | OCF₃ | H | H | H | N | H | H | H |
| 1-139 | Cyclopropyl | CH₂CH₃ | 2-Cl | 4-F | H | H | N | H | H | H |
| 1-140 | Cyclopropyl | CH₂CH₃ | 2-Cl | H | H | H | N | H | H | H |
| 1-141 | C(CH₃)₃ | CH₂CH₃ | 3-NH₂ | 4-F | H | H | N | H | H | H |
| 1-142 | C(CH₃)₃ | CH₂CH₃ | 2-Cl | 4-Cl | H | H | N | H | H | H |
| 1-143 | 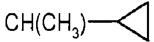 | CH₂CH₃ | 4-F | H | H | H | N | H | H | H |
| 1-144 | 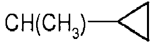 | CH₂CH₃ | 4-Cl | H | H | H | N | H | H | H |
| 1-145 | 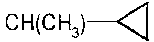 | CH₂CH₃ | 4-CN | H | H | H | N | H | H | H |
| 1-146 | 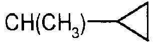 | CH₂CH₃ | 4-OCF₃ | H | H | H | N | H | H | H |
| 1-147 | 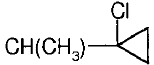 | CH₂CH₃ | 4-F | H | H | H | N | H | H | H |
| 1-148 | 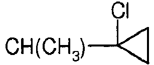 | CH₂CH₃ | 2-Cl | 4-Cl | H | H | N | H | H | H |
| 1-149 | 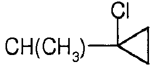 | CH₂CH₃ | 4-Cl | H | H | H | N | H | H | H |
| 1-150 | 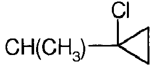 | CH₂CH₃ | 4-CN | H | H | H | N | H | H | H |
| 1-151 | 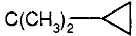 | CH₂CH₃ | 4-Cl | H | H | H | N | H | H | H |
| 1-152 | 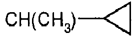 | CH₂CH₃ | H | H | H | H | N | H | H | H |
| 1-153 | Cyclopropyl | CH₂CH₃ | 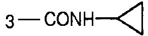 | H | H | H | N | H | H | H |
| 1-154 | Cyclopropyl | OCH₃ | 4-Cl | H | H | H | N | H | H | H |
| 1-155 | Cyclopropyl | OCH₃ | 4-F | H | H | H | N | H | H | H |
| 1-156 | Cyclopropyl | OCH₃ | 4-CN | H | H | H | N | H | H | H |
| 1-157 | Cyclopropyl | OCH₃ | 4-OCF₃ | H | H | H | N | H | H | H |
| 1-158 | Cyclopropyl | OCH₂CH₃ | 4-Cl | H | H | H | N | H | H | H |
| 1-159 | Cyclopropyl | OCH₂CH₃ | 4-F | H | H | H | N | H | H | H |
| 1-160 | Cyclopropyl | OCH₂CH₃ | 4-CN | H | H | H | N | H | H | H |
| 1-161 | Cyclopropyl | OCH₂CH₃ | 4-OCF₃ | H | H | H | N | H | H | H |
| 1-162 | Cyclopropyl | O(CH₃)₂ | 4-Cl | H | H | H | N | H | H | H |
| 1-163 | Cyclopropyl | OCH(CH₃)₂ | 4-F | H | H | H | N | H | H | H |
| 1-164 | Cyclopropyl | OCH(CH₃)₂ | 4-CN | H | H | H | N | H | H | H |
| 1-165 | Cyclopropyl | OCH(CH₃)₂ | 4-OCF₃ | H | H | H | N | H | H | H |
| 1-166 | Cyclopropyl | OCH₂CF₃ | 4-Cl | H | H | H | N | H | H | H |
| 1-167 | Cyclopropyl | OCH₂CF₃ | 4-F | H | H | H | N | H | H | H |
| 1-168 | Cyclopropyl | OCH₂CF₃ | 4-CN | H | H | H | N | H | H | H |
| 1-169 | Cyclopropyl | OCH₂CF₃ | 4-OCF₃ | H | H | H | N | H | H | H |
| 1-170 | Cyclopropyl | CF₃ | 4-F | H | H | H | N | H | H | H |
| 1-171 | Cyclopropyl | CN | 4-F | H | H | H | N | H | H | H |
| 1-172 | Cyclopropyl | 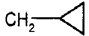 | 4-H | H | H | H | N | H | H | H |
| 1-173 | Cyclopropyl | 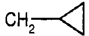 | 4-Cl | H | H | H | N | H | H | H |
| 1-174 | Cyclopropyl | CHFCH₃ | 4-Cl | H | H | H | N | H | H | H |
| 1-175 | Cyclopropyl | CH₂CH₃ | 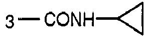 | H | H | H | N | H | H | H |
| 1-176 | Cyclopropyl | CH₂CF₃ | 4-Cl | H | H | H | N | H | H | H |
| 1-177 | Cyclopropyl | CH₂CH₃ | 4-Cl | H | H | H | N | H | H | H |
| 1-178 | Cyclopropyl | CF₃ | 4-Cl | H | H | H | N | H | H | H |
| 1-179 | Cyclopropyl | CF₂H | 4-Cl | H | H | H | N | H | H | H |
| 1-180 | Cyclopropyl | CH₂CH₃ | 2-CH₃ | 4-F | H | H | N | H | H | H |
| 1-181 | Cyclopropyl | CH₂CH₃ | 2-CH₃ | 4-CN | H | H | N | H | H | H |
| 1-182 | Cyclopropyl | CH₂CH₃ | 2-CH₃ | H | H | H | N | H | H | H |
| 1-183 | Cyclopropyl | CH₂CH₃ | 2-CH₃ | 3-NH₂ | 4-F | H | N | H | H | H |
| 1-184 | Cyclopropyl | CH₂CH₃ | 2-CH₃ | 5-F | H | H | N | H | H | H |
| 1-185 | Cyclopropyl | CH₂CH₃ | 4-OCF₃ | H | H | H | N | H | H | H |
| 1-186 | Cyclopropyl | CH₂CH₃ | 4-CF₃ | H | H | H | N | H | H | H |
| 1-187 | Cyclopropyl | CH₂CH₃ | 2-F | 4-Cl | H | H | N | H | H | H |
| 1-188 | Cyclopropyl | CH₂CH₃ | 2-CH₃ | 4-Cl | H | H | N | H | H | H |
| 1-189 | Cyclopropyl | CH₂CH₃ | 2-CH₃ | 4-CF₃ | H | H | N | H | H | H |
| 1-190 | Cyclopropyl | CH₂CH₃ | 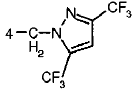 | H | H | H | N | H | H | H |
| 1-191 | Cyclopropyl | CH₂CH₃ | 2-CH₃ | 4-OCF₃ | H | H | N | H | H | H |
| 1-192 | Cyclopropyl | CH₂CH₃ | 4-Cl | H | H | H | N | H | Cl | H |
| 1-193 | Cyclopropyl | CH₃ | 4-OCF₃ | H | H | H | N | CF₃ | H | H |
| 1-194 | Cyclopropyl | CHFCH₃ | 4-Cl | H | H | H | N | H | Cl | H |
| 1-195 | Cyclopropyl | CH₃ | 4-Cl | H | H | H | N | CH₃ | H | CH₃ |
| 1-196 | Cyclopropyl | CH₂CH₃ | 4-F | H | H | H | N | CF₃ | H | H |
| 1-197 | Cyclopropyl | Cyclopropyl | 4-Cl | H | H | H | N | H | Cl | H |
| 1-198 | Cyclopropyl | Cyclopropyl | 4-F | H | H | H | N | H | Cl | H |
| 1-199 | Cyclopropyl | CH₃ | 4-Cl | H | H | H | N | CF₃ | H | H |
| 1-200 | Cyclopropyl | CH₂CH₃ | 4-Cl | H | H | H | N | H | H | CF₃ |
| 1-201 | Cyclopropyl | CH₂CH₃ | 4-F | H | H | H | N | H | H | CF₃ |
| 1-202 | Cyclopropyl | CH₂CH₃ | 4-CN | H | H | H | N | H | H | CF₃ |
| 1-203 | Cyclopropyl | CH₂CH₃ | 4-CF₃ | H | H | H | N | H | H | CF₃ |
| 1-204 | Cyclopropyl | CH₂CH₃ | 4-OCF₃ | H | H | H | N | H | H | CF₃ |
| 1-205 | Cyclopropyl | CH₂CH₃ | 2-F | 4-F | H | H | N | H | H | CF₃ |
| 1-206 | Cyclopropyl | CH₂CH₃ | 2-Cl | 4-Cl | H | H | N | H | H | CF₃ |
| 1-207 | Cyclopropyl | CH₃ | 4-Cl | H | H | H | N | H | H | CF₃ |
| 1-208 | Cyclopropyl | CH₃ | 4-F | H | H | H | N | H | H | CF₃ |
| 1-209 | Cyclopropyl | CH₃ | 4-CN | H | H | H | N | H | H | CF₃ |
| 1-210 | Cyclopropyl | CH₃ | 4-CF₃ | H | H | H | N | H | H | CF₃ |
| 1-211 | Cyclopropyl | CH₃ | 4-OCF₃ | H | H | H | N | H | H | CF₃ |
| 1-212 | Cyclopropyl | CH₃ | 2-F | 4-F | H | H | N | H | H | CF₃ |
| 1-213 | Cyclopropyl | CH₃ | 2-Cl | 4-Cl | H | H | N | H | H | CF₃ |
| 1-214 | Cyclopropyl | Cyclopropyl | 4-Cl | H | H | H | N | H | H | CF₃ |
| 1-215 | Cyclopropyl | Cyclopropyl | 4-F | H | H | H | N | H | H | CF₃ |
| 1-216 | Cyclopropyl | Cyclopropyl | 4-CN | H | H | H | N | H | H | CF₃ |
| 1-217 | Cyclopropyl | Cyclopropyl | 4-CF₃ | H | H | H | N | H | H | CF₃ |
| 1-218 | Cyclopropyl | Cyclopropyl | 4-OCF₃ | H | H | H | N | H | H | CF₃ |
| 1-219 | Cyclopropyl | Cyclopropyl | 2-F | 4-F | H | H | N | H | H | CF₃ |
| 1-220 | Cyclopropyl | Cyclopropyl | 2-Cl | 4-Cl | H | H | N | H | H | CF₃ |
| 1-221 | CH(CH₃)₂ | CH₂CH₃ | 4-Cl | H | H | H | N | H | H | H |
| 1-222 | 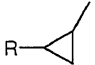 | CH₂CH₃ | 2-F | 4-F | H | H | N | H | H | H |
| 1-223 | CH(CH₃)CH₂SCH₃ | CH₂CH₃ | 4-Cl | H | H | H | N | H | H | H |
| 1-224 | Cyclobutyl | CH₂CH₃ | 4-F | H | H | H | N | H | H | H |
| 1-225 | Cyclobutyl | CH₂CH₃ | 2-F | 4-F | H | H | N | H | H | H |
| 1-226 | Cyclobutyl | CH₂CH₃ | 4-Cl | H | H | H | N | H | H | H |
| 1-227 | Cyclopentyl | CH₂CH₃ | 4-F | H | H | H | N | H | H | H |
| 1-228 | Cyclopentyl | CH₂CH₃ | 2-F | 4-F | H | H | N | H | H | H |
| 1-229 | 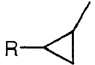 | CH₂CH₃ | 4-F | H | H | H | N | H | H | H |
| 1-230 | 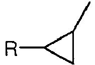 | CH₂CH₃ | 2-F | 4-Cl | H | H | N | H | H | H |
| 1-231 | Cyclobutyl | CH₂CH₃ | 2-F | 4-Cl | H | H | N | H | H | H |
| 1-232 | Cyclopropyl | CH₂CH₃ | 4-iC₃F₇ | H | H | H | N | H | H | H |
| 1-233 | Cyclopropyl | Cyclopropyl | 4-iC₃F₇ | H | H | H | N | H | H | H |
| 1-234 | Cyclopropyl | Cyclopropyl | H | H | H | H | N | H | H | H |
| 1-235 | Cyclopropyl | Cyclopropyl | 4-F | H | H | H | N | H | H | H |
| 1-236 | Cyclopropyl | Cyclopropyl | 4-Cl | H | H | H | N | H | H | H |
| 1-237 | Cyclopropyl | Propyl | H | H | H | H | N | H | H | H |
| 1-238 | Cyclopropyl | Propyl | 4-F | H | H | H | N | H | H | H |
| 1-239 | Cyclopropyl | Propyl | 4-Cl | H | H | H | N | H | H | H |
| 1-240 | Cyclopropyl | Cyclopropyl | 2-F | 4-F | H | H | N | H | H | H |
| 1-241 | Cyclopropyl | C₂F₅ | 4-Cl | H | H | H | N | H | H | H |
| 1-242 | Cyclopropyl | Cyclopropyl | 4-CN | H | H | H | N | H | H | H |
| 1-243 | Cyclopropyl | Cyclopropyl | 4-OCF₃ | H | H | H | N | H | H | H |
| 1-244 | Cyclopropyl | Propyl | 2-Cl | 4-Cl | H | H | N | H | H | H |
| 1-245 | Cyclopropyl | Propyl | 2-F | 4-F | H | H | N | H | H | H |
| 1-246 | Cyclopropyl | Propyl | 4-CN | H | H | H | N | H | H | H |
| 1-247 | Cyclopropyl | CH₂CH₃ | 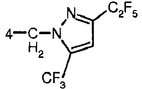 | H | H | H | N | H | H | H |
| 1-248 | Cyclopropyl | CH₂CH₃ | 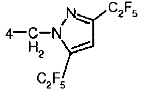 | H | H | H | N | H | H | H |
| 1-249 | Cyclopropyl | C₃F₇ | 4-Cl | H | H | H | N | H | H | H |
| 1-250 | 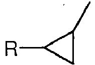 | CH₂CH₃ | 4-Cl | H | H | H | N | H | H | H |
| 1-251 | Cyclopropyl | C₃F₇ | 4-F | H | H | H | N | H | H | H |
| 1-252 | Cyclopropyl | Cyclopropyl | 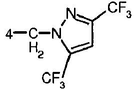 | H | H | H | N | H | H | H |
| 1-253 | Cyclopropyl | Cyclopropyl | 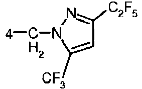 | H | H | H | N | H | H | H |
| 1-254 | Cyclopropyl | CH₂CF₃ | 2-F | 4-F | H | H | N | H | H | H |
| | | | | | | | | | | |
| | | | | | | | | | | |
| | | | | | | | | | | |
| 1-258 | Cyclopropyl | CH₂CH₃ | 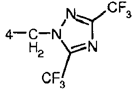 | H | H | H | N | H | H | H |
| 1-259 | Cyclopropyl | Cyclopropyl | 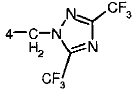 | H | H | H | N | H | H | H |
| 1-260 | Cyclopropyl | C₂F₅ | 2-F | 4-F | H | H | N | H | H | H |
| 1-261 | Cyclopropyl | C₂F₅ | 4-F | H | H | H | N | H | H | H |
| 1-262 | Cyclopropyl | C(=O)NHCH(CH2)2 | 4-F | H | H | H | N | H | H | H |
| | | | | | | | | | | |
| 1-264 | Cyclopropyl | CH₂CH₂CH₃ | 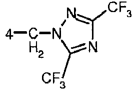 | H | H | H | N | H | H | H |
| | | | | | | | | | | |
| 1-266 | Cyclopropyl | CH₂CH₃ | 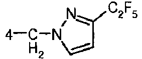 | H | H | H | N | H | H | H |
| 1-267 | Cyclopropyl | CH₂CH₃ | 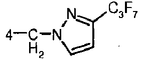 | H | H | H | N | H | H | H |
| 1-268 | Cyclopropyl | CH₂CH₃ | 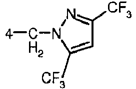 | H | H | H | N | H | H | H |
| 1-269 | Cyclopropyl | CH₂CH₃ | 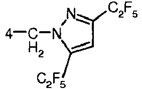 | H | H | H | N | H | H | H |
| 1-270 | Cyclopropyl | CH₂CH₃ | 2-F | 4-F | H | SCH₃ | N | H | H | H |
| 1-271 | Cyclopropyl | OCF₃ | 2-F | 4-F | H | H | N | H | H | H |
| 1-272 | Cyclopropyl | CH₂CH₃ | 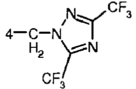 | H | H | H | N | H | H | H |
| 1-273 | Cyclopropyl | CH₂CH₃ | 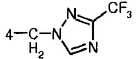 | H | H | H | N | H | H | H |
| 1-274 | CH(CF₃)CH₃ | CH₂CH₃ | 2-F | 4-F | H | H | N | H | H | H |
| 1-275 | Cyclopropyl | CH₂CH₃ | H | H | H | CH₃ | N | H | H | H |
| 1-276 | Cyclopropyl | CH₂CH₃ | 4-F | H | H | CH₃ | N | H | H | H |
| 1-277 | Cyclopropyl | CH₂CH₃ | 2-Cl | 4-Cl | H | CH₃ | N | H | H | H |
| 1-278 | Cyclopropyl | CH₂CH₃ | 2-F | 4-F | H | CH₃ | N | H | H | H |
| 1-279 | Cyclopropyl | CH₂CH₃ | 4-OCF₃ | H | H | CH₃ | N | H | H | H |
| | | | | | | | | | | |
| 1-281 | Cyclopropyl | CH₂CH₃ | 4-F | H | H | CH₃ | N | H | H | H |
| 1-282 | Cyclopropyl | CH₂CH₃ | 4-Cl | H | H | CH₃ | N | H | H | H |
| 1-283 | Cyclopropyl | CH₂CH₃ | 2-Cl | 4-Cl | H | CH₃ | N | H | H | H |
| 1-284 | Cyclopropyl | CH₂CH₃ | 2-Cl | 4-Cl | H | CH₃ | N | H | H | H |
| 1-285 | Cyclopropyl | CH₂CH₃ | 2-Cl | 4-Cl | H | CH₃ | N | H | H | H |
| | | | | | | | | | | |
| 1-287 | Cyclopropyl | Cyclopropyl | 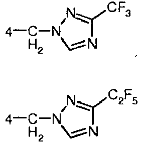 | H | H | H | N | H | H | H |
| 1-288 | Cyclopropyl | CH₂CH₃ | | H | H | H | N | H | H | H |
| 1-289 | Cyclopropyl | CH₂CH₃ | 4-CN | H | H | CH₃ | N | H | H | H |
| 1-290 | Cyclopropyl | CH₂CH₃ | 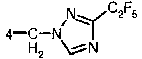 | H | H | SCH₃ | N | H | H | H |
| | | | | | | | | | | |
| 1-292 | Cyclopropyl | Cyclopropyl | 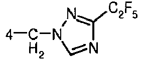 | H | H | H | N | H | H | H |
| | | | | | | | | | | |
| | | | | | | | | | | |
| | | | | | | | | | | |
| | | | | | | | | | | |
| | | | | | | | | | | |
| | | | | | | | | | | |
| 1-299 | CH(CF₃)CH₃ | CH₂CH₃ | 4-F | H | H | H | N | H | H | H |
| | | | | | | | | | | |
| 1-301 | Cyclopropyl | CH₂CH₃ | 4-Cl | H | H | CH₃ | N | H | H | H |
| | | | | | | | | | | |
| | | | | | | | | | | |
| | | | | | | | | | | |
| 1-305 | Cyclopropyl | CH₂CH₃ | 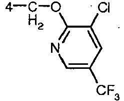 | H | H | H | N | H | H | H |
| 1-306 | Cyclopropyl | CH₂CH₃ | 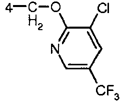 | H | H | H | N | H | H | H |
| 1-307 | Cyclopropyl | CH₂CF₃ | 4-F | H | H | H | N | H | H | H |
| 1-308 | Cyclopropyl | CHFCF₃ | 4-F | H | H | H | N | H | H | H |
| 1-309 | Cyclopropyl | CH₂CH₃ | 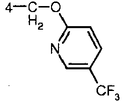 | H | H | H | N | H | H | H |
| 1-310 | Cyclopropyl | CH₂CH₃ | 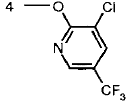 | H | H | H | N | H | H | H |
| 1-311 | Cyclopropyl | CH₂CH₃ | 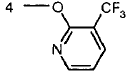 | H | H | H | N | H | H | H |
| 1-312 | Cyclopropyl | CH₂CH₃ | 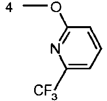 | H | H | H | N | H | H | H |
| 1-313 | Cyclopropyl | CH₂CH₃ | 4-F | H | H | CF₃ | N | H | H | H |
| 1-314 | Cyclopropyl | CH₂CH₃ | 2-F | 4-F | H | CF₃ | N | H | H | H |
| 1-315 | Cyclopropyl | CH₂CH₃ | 4-F | H | H | H | N | H | H | Cl |
| 1-316 | Cyclopropyl | CH₂CH₃ | 4-Cl | H | H | H | N | H | H | Cl |
| 1-317 | Cyclopropyl | CH₂CH₃ | 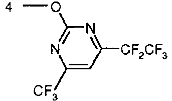 | H | H | H | N | H | H | H |
| 1-318 | Cyclopropyl | CH₂CH₃ | 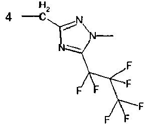 | H | H | H | N | H | H | H |
| 1-319 | Cyclopropyl | CH₂CH₃ | 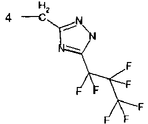 | H | H | H | N | H | H | H |
| | | | | | | | | | | |
| | | | | | | | | | | |
| 1-322 | Cyclopropyl | CH₂CH₃ | 4-F | H | H | H | N | H | F | H |
| 1-323 | Cyclopropyl | CH₂CH₃ | 4-F | H | H | H | N | H | Cl | H |
| 1-324 | Cyclopropyl | CH₂CF₃ | 4-F | H | H | CF₃ | N | H | H | H |
| 1-325 | Cyclopropyl | CH₂CF₃ | 4-F | H | H | Cl | N | H | H | H |
| 1-326 | Cyclopropyl | CH₂CH₃ | 4-F | H | H | Cl | N | H | H | H |
| | | | | | | | | | | |
| | | | | | | | | | | |
| | | | | | | | | | | |
| | | | | | | | | | | |

**Tabelle 2: Physikochemische Charakterisierung**

| **Bsp. Nr.** | **MH+** | **LogP(HCOOH)** | **NMR (400MHz, DMSO-d6, wenn nichts anderes angegeben ist)** |
|---|---|---|---|
| 1-1 | 364.2 | 0.71 | (MeOH d4); 0.68 (2H, m), 0.90 (2H, m), 2.38 (3H, s), 2.94 (1H, m), 7.22 (2H, t), 7.35 (1H, m), 7.61 (2H, m), 7.95 (1H, m), 8.49 (1H, m), 8.59 (1H, s). |
| 1-2 | 538.1 | 2.31 | |
| 1-3 | 538.1 | 2.27 | |
| 1-4 | 396.2 | 1.34 | |
| 1-5 | 396.0 | | 0.56 (2H, m), 0.78 (2H, m), 1.06 (3H, t), 2.90 (1H, m), 6.88 (1H, s), 7.11 (1H, m), 7.22 (2H, m), 7.50 (1H, m), 7.74 (1H, m), 8.52 (2H, m), 9.36 (1H, s). |
| 1-8 | 374.2 | | |
| 1-9 | 396.1 | | |
| 1-10 | 406.1 | | |
| 1-11 | 378.1 | | |
| 1-12 | 404.1 | | |
| 1-13 | 394.1 | | |
| 1-14 | 361.1 | | (MeCN d3); 0.56 (2H, m), 0.80 (2H, m), 1.13 (3H, t), 2.67 (2H, q), 2.89 (1H, m), 6.12 (1H, m), 7.22 (1H, m), 7.41 (1H, m), 7.69 (1H, m), 7.97 (1H, m), 8.08 (1H, s), 8.58 (3H, m), 8.81 (1H, m). |
| | | | |
| 1-73 | 430.0 | 1.45 | 0.60 (m, 2H), 0.78 (m, 2H), 2.24 (s, 3H), 2.91 (m, 1H), 7.29 (m, 1H), 7.39 (d, 2H), 7.76 (d, 2H), 7.95 (d, 1H), 8.49 (m, 1H), 8.56 (m, 1H), 9.56 (s, 1H). |
| 1-74 | 432.1 | 1.55 | 1.26 (d, 6H), 2.36 (s, 3H), 4.41 (m, 1H), 5.75 (m, 1H), 7.31 (m, 1H), 7.40 (m, 2H), 7.78 (m, 2H), 7.99 (m, 1H), 8.01 (m, 1H), 8.49 (m, 1H). |
| 1-75 | 450.1 | 1.99 | 0.78 (m, 2H), 0,89 (m, 2H), 3.15 (m, 1H), 7.34 (m, 3H), 7.76 (m, 2H), 7.95 (m, 1H), 8.40 (m, 1H), 8.60 (m, 1H), 9.42 (m, 1H). |
| 1-76 | 452.1 | 2.05 | 1.27 (d, 6H), 4.40 (m, 1H), 7.13 (m, 1H), 7.48 (m, 3H), 7.77 (m, 2H), 7.90 (m, 1H), 8.48 (m, 1H), 8.64 (m, 1H), 10.02 (m, 1H). |
| | | | |
| | | | |
| | | | |
| 1-82 | 434.0 | 1.94 | 0.54 (m, 2H), 0.80 (m, 2H), 2.91 (m, 1H), 6.81 (m, 1H), 7.50 (m, 1H), 7.78 (d, 2H), 7.99 (d, 1H), 8.06 (m, 1H), 8.46 (m, 1H), 8.56 (m, 1H), 8.57 (m, 1H), 9.72 (s, 1H). |
| 1-83 | 414.1 | 1.43 | 0.58 (m, 2H), 0.77 (m, 2H), 2.21 (s, 3H), 2.90 (m, 1H), 7.14 (m, 1H), 7.28 (m, 1H), 7.76 (d, 2H), 7.84 (d, 2H), 8.11 (m, 1H), 8.47 (m, 1H), 8.54 (m, 1H), 9.45 (s, 1H). |
| 1-84 | 468.1 | 2.19 | |
| 1-85 | 442.1 | 2.02 | 0.57 (m, 2H), 0.78 (m, 2H), 1.01 (d, 6H), 2.89 (m, 1H), 7.05 (m, 1H), 7.23 (m, 1H), 7.76 (d, 2H), 7.83 (d, 2H), 7.85 (d, 2H), 8.54 (s, 1H), 9.70 (s, 1H). |
| | | | |
| 1-87 | 428.2 | 1.27 | |
| | | | |
| 1-89 | 455.1 | 1.72 | 0.62 (m, 2H), 0.78 (m, 2H), 1.07 (m, 2H), 1.15 (m, 2H), 1.25 (m, 1H), 2.21 (s, 3H), 2.95 (m, 1H), 7.33 (m, 1H), 7.80 (m, 5H), 7.86 (m, 1H), 8.49 (m, 1H), 9.70 (s, 1H). |
| 1-99 | 478.0 | 2.44 | 0.31 (m, 1H), 0.36 (m, 1H), 0.45 (, 1H), 0.50 (m, 1H), 1.00 (m, 1H), 1.20 (d, 3H), 3.76 (m, 1H), 5.81 (m, 1H), 7.39 (m, 3H), 7.70 (m, 3H), 8.24 (s, 1H), 8.45 (s, 2H). |
| 1-100 | 458.1 | 1.80 | 0.31 (m, 1H), 0.36 (m, 1H), 0.45 (m, 1H), 0.50 (m, 1H), 1.10 (m, 1H), 1.28 (d, 3H), 3.80 (m, 1H), 6.59 (m, 1H), 7.30 (m, 2H), 7.41 (m, 2H), 7.79 (m, 2H), 8.44 (s, 1H), 8.50 (s, 1H), 9.49 (s, 1H). |
| 1-111 | 504.1 | 2.65 | 0.36 (m, 4H), 0.50 (m, 4H), 1.11 (m, 2H), 3.56 (m, 1H), 6.21 (m, 1H), 7.44 (m, 2H), 7.53 (m, 2H), 7.79 (m, 2H), 8.42 (s, 1H), 8.50 (s, 1H), 9.91 (s, 1H). |
| | | | |
| | | | |
| 1-114 | 460 | 1.4 | 0.56 (2H, m), 0.78 (2H, m), 1.04 (3H, t), 2.91 (1H, m), 7.00 (1H, m), 7.26 (1H, m), 7.74 (4H, m), 7.85 (1H, 8.54 (2H, m), 9.42 (1H, s). |
| 1-115 | 419 | 1.40 | 0.55 (2H, m), 0.77 (2H, m), 1.04 (3H, t), 2.55 (2H, q), 2.89 (1H, m), 6.74 (1H, d), 6.96 (1H, m), 7.28 (1H, m), 7.92 (2H, m), 8.44 (1H, m), 8.55 (2H, m), 9.41 (1H, s). |
| 1-116 | 446 | 1.81 | 0.58 (2H, m), 0.79 (2H, m), 2.23 (3H, s), 2.90 (1H, m), 7.10 (1H, s), 7.25 (1H, m), 7.75 (4H, m), 7.85 (1H, m), 8.48 (1H, m), 8.54 (1H, s), 9.41 (1H, s). |
| 1-117 | 477 | 1.65 | |
| 1-119 | 485 | 1.80 | 0.55 (2H, m), 0.76 (2H, m), 2.32 (3H, s), 2.88 (1H, m), 5.13 (2H, s), 6.87 (1H, m), 7.01 (2H, d), 7.25 (1H, m), 7.42 (4H, m), 7.64 (2H, d), 7.88 (1H, m), 8.49 (1H, m), 9.34 (1H, s). |
| 1-120 | 444 | 1.52 | |
| 1-121 | 467 | 1.15 | |
| 1-122 | 450 | 1.95 | 0.58 (2H, m), 0.78 (2H, m), 2.91 (1H, m), 7.33 (2H, m), 7.61 (2H, m), 7.95 (1H, m), 8.52 (1H, m), 8.57 (1H, s), 9.41 (1H, s). |
| 1-123 | 512 | 1.79 | 0.58 (2H, m), 0.77 (2H, m), 2.25 (3H, s), 6.33 (1H, m), 7.04 (1H, m), 7.29 (3H, m), 7.74 (2H, m), 7.90 (1H, m), 8.48 (1H, m), 8.83 (1H, s), 9.39 (1H, s). |
| 1-124 | 378 | 1.03 | |
| 1-126 | 329 | 1.60 | 0.57 (2H, m), 0.79 (2H, m), 1.03 (3H, t), 2.91 (1H, m), 6.86 (1H, m), 7.263 (1H, m), 7.44 (2H, m), 7.61 (1H, m), 7.72 (1H, m), 8.50 (2H, m), 9.33 (1H, s). |
| 1-127 | 360 | 1.01 | 0.55 (2H, m), 0.76 (2H, m), 1.06 (3H, t), 2.55 (2H, q), 2.89 (1H, m), 6.86 (1H, m), 7.25 (1H, m), 7.37 (3H, m), 7.62 (2H, m), 7.84 (1H, m), 8.52 (2H, m), 9.38 (1H, s). |
| 1-128 | 332 | 0.63 | 0.56 (2H, m), 0.73 (2H, m), 2.86 (1H, m), 7.17 (1H, s), 7.30 (3H, m), 7.46 (1H, m), 7.62 (2H, m), 8.10 (1H, m), 8.52 (1H, s), 8.67 (1H, m), 8.96 (1H, m), 9.65 (1H, s). |
| 1-129 | 384 | 1.27 | 0.54 (2H, m), 0.79 (2H, m), 2.90 (1H, m), 6.64 (1H, m), 7.23 (2H, m), 7.51 (1H, m), 7.70 (2H, m), 7.94 (1H, m), 8.47 (1H, m), 8.53 (1H, s), 9.69 (1H, s). |
| 1-130 | 401 | 1.55 | 0.61 (2H, m), 0.85 (2H, m), 3.00 (1H, m), 7.47 (1H, m), 7.53 (2H, m), 7.69 (2H, m), 8.02 (1H, m), 8.48 (1H, m), 8.65 (1H, s). |
| 1-133 | 385.2 | 1.10 | 0.56 (2H, m), 0.78 (2H, m), 1.04 (3H, t), 2.89 (1H, m), 7.07 (1H, m), 7.27 (1H, m), 7.84 (6H, m), 8.55 (2H, m), 9.40 (1H, s). |
| 1-134 | 394.2 | 1.53 | 1.34 (3H, t), 1.48 (9H, s), 2.67 (2H, q), 5.78 (1H, s), 7.29 (3H, m), 7.60 (1H, m), 7.68 (2H, m), 8.48 (1H, s), 8.56 (1H, m), 9.61 (1H, s). |
| 1-135 | 410.1 | 1.86 | 1.31 (3H, t), 1.48 (9H, s), 2.65 (1H, s), 5.81 (1H, s), 7.30 (1H, m), 7.50 (2H, m), 7.64 (3H, m), 8.48 (1H, s), 8.55 (1H, s), 9.61 (1H, s). |
| 1-136 | 401.2 | 1.81 | 1.11 (3H, t), 1.48 (9H, s), 2.61 (2H, q), 5.93 (1H, s), 7.30 (1H, m), 7.61 (1H, m), 7.81 (2H, d), 7.88 (2H, d), 8.51 (1H, s), 8.55 (1H, m), 9.64 (1H, s). |
| 1-137 | 444.2 | 2.34 | 1.08 (3H, t), 1.49 (9H, s), 2.57 (2H, q), 5.88 (1H, s), 7.30 (1H, m), 7.61 (1H, m), 7.82 (4H, m), 8.51 (1H, s), 8.54 (1H, s), 9.65 (1H, s). |
| 1-138 | 460.2 | 2.32 | 1.11 (2H, q), 1.48 (9H, s), 2.63 (2H, q), 5.82 (1H, s), 7.29 (1H, m), 7.39 (2H, m), 7.58 (1H, m), 7.75 (2H, m), 8.49 (1H, s), 8.55 (1H, m), 9.63 (1H, s). |
| 1-139 | 412.1 | 0.79 | 0.56 (2H, m), 0.78 (2H, m), 1.04 (3H, t), 2.90 (1H, m), 6.82 (1H, m), 7.23 (2H, m), 7.44 (2H, m), 7.67 (1H, m), 8.50 (2H, m), 9.30 (1H, m). |
| 1-140 | 394.1 | 1.07 | 1.19 (2H, m) 0.78 (2H, m), 1.03 (3H, t), 2.91 (1H, m), 6.80 (1H, m), 7.37 (1H, m), 7.37 (3H, m), 7.49 (1H, m), 7.62 (1H, m), 8.48 (1H, m), 8.53 (1H, m), 9.28 (1H, m). |
| 1-141 | 395.2 | 1.65 | 1.12 (3H, t), 1.48 (9H, s), 2.66 (2H, q), 5.97 (1H, s), 7.24 (1H, m), 7.30 (1H, m), 7.62 (1H, m), 8.20 (1H, m), 8.50 (2H, m), 8.55 (1H, m), 9.64 (1H, s). |
| 1-142 | 444.1 | 1.89 | 1.08 (3H, t), 1.48 (9H, m), 5.84 (1H, s), 7.26 (1H, m), 7.42 (3H, m), 7.64 (1H, m), 8.48 (1H, s), 8.52 (1H, m), 9.56 (1H, s). |
| 1-143 | 406.2 | 1.47 | 0.41 (4H, m), 1.03 (1H, m), 1.12 (3H, t), 1.25 (3H, d), 2.67 (2H, q), 3.79 (1H, m), 6.40 (1H, d), 7.23 (2H, t), 7.29 (2H, m), 7.72 (3H, m), 8.42 (1H, s), 8.55 (1H, m), 9.48 (1H, s). |
| 1-144 | 422.1 | 1.62 | 0.45 (4H, m), 1.05 (1H, m), 1.13 (3H, t), 1.24 (3H, d), 2.65 (2H, q), 3.77 (1H, m), 6.43 (1H, d), 7.30 (1H, m), 7.48 (2H, d), 7.67 (2H, d), 7.76 (1H, d), 8.42 (1H, s), 8.56 (1H, d), 9.49 (1H, s). |
| 1-145 | 413.2 | 1.67 | 0.40 (4H, m), 1.10 (4H, m), 1.24 (3H, d), 2.61 (2H, q), 3.79 (1H, m), 6.59 (1H, d), 7.31 (1H, m), 7.76 (1H, m), 7.84 (4H, m), 8.45 (1H, s), 8.56 (1H, m), 9.51 (1H, s). |
| 1-146 | 472.2 | 2.18 | 0.47 (4H, m) 1.09 (3H, t), 1.26 (3H, d), 2.62 (2H, q), 3.79 (1H, m), 6.45 (1H, d), 7.29 (1H, m), 7.40 (2H, d), 7.76 (3H, m), 8.43 (1H, s), 8.55 (1H, m), 9.51 (1H, s). |
| 1-147 | 441.1 | 1.95 | 1.13 (4H, m), 1.35 (3H, d), 2.71 (2H, q), 4.25 (1H, m), 6.26 (1H, d), 7.26 (3H, m), 7.63 (1H, m), 7.72 (2H, m), 8.48 (1H, s), 8.55 (1H, m), 9.68 (1H, s). |
| 1-148 | 490.1 | 2.67 | 1.09 (4H, m), 1.37 (2H, d), 2.59 (1H, m), 4.22 (1H, m), 6.25 (1H, d), 7.27 (1H, m), 7.44 (1H, m), 7.50 (2H, m), 7.66 (1H, m), 8.50 (1H, s), 8.54 (1H, m), 9.67 (1H, s). |
| 1-149 | 458.1 | 2.26 | 1.16 (4H, m); 1.35 (3H, d), 2.70 (2H, q), 4.25 (1H, m) 6.30 (1H, m), 7.32 (1H, m), 7.51 (2H, d), 7.68 (3H, m), 8.49 (1H, s), 8.56 (1H, m), 9.69 (1H, s). |
| 1-150 | 449.1 | 2.08 | 1.19 (4H, m), 1.35 (3H, d), 2.66 (1H, m), 4.3 (1H, m), 6.43 (1H, d), 7.32 (1H, m), 7.69 (4H, m), 8.52 (1H, s), 8.57 (1H, m), 9.71 (1H, s). |
| 1-151 | 436.2 | 2.22 | 0.41 (6H, m), 1.13 (3H, t), 1.37 (4H, m), 1.50 (1H, m), 2.69 (2H, q), 5.73 (1H, s), 7.30 (1H, m), 7.50 (2H, s), 7.57 (1H, d), 7.63 (2H, d), 8.47 (1H, s), 8.55 (1H, m), 9.73 (1H, s). |
| 1-152 | 386.2 | 1.32 | |
| 1-154 | 396.1 | 1.83 | 0.54 (2H, m), 0.75 (2H, m), 2.87 (1H, m), 3.99 (3H, s), 6.86 (1H, s), 7.10 (1H, m), 7.42 (2H, d), 7.70 (2H, d), 8.01 (1H, m), 8.30 (1H, m), 8.52 (1H, s), 9.37 (1H, s). |
| 1-155 | 360.2 | 1.49 | 0.54 (2H, m); 0.75 (2H, m), 2.88 (1H, m), 3.99 (3H, s), 6.81 (1H, s), 7.14 (1H, m), 7.16 (2H, t), 7.74 (2H, m), 8.00 (1H, m), 8.29 (1H, m), 8.51 (1H, s), 9.37 (1H, s). |
| 1-156 | 387.3 | 1.59 | 0.55 (2H, m), 0.75 (2H, m), 2.87 (1H, m), 3.99 (3H, s), 6.96 (1H, m), 7.09 (1H, m), 7.81 (4H, m), 7.99 (1H, m), 8.29 (1H, m), 8.55 (1H, m), 9.38 (1H, s). |
| 1-157 | 446.1 | 2.21 | 0.54 (2H, m), 0.75 (2H, m), 2.88 (1H, m), 3.97 (3H, s), 6.86 (1H, s), 7.08 (1H, m), 7.33 (2H, m), 7.79 (2H, m), 7.99 (1H, m); 8.28 (1H, m), 8.53 (1H, s), 9.38 (1H, s). |
| 1-158 | 410.0 | 2.54 | 0.53 (2H, m); 0.75 (2H, m), 1.31 (3H, t), 2.87 (1H, m), 4.47 (2H, q), 6.83 (1H, s), 7.09 (1H, m), 7.40 (2H, d), 7.69 (2H, d), 8.03 (1H, m), 8.28 (1H, m), 8.52 (1H, s), 9.28 (1H, s). |
| 1-159 | 394.2 | 1.84 | 0.53 (2H, m), 0.74 (2H, m), 1.30 (3H, t), 2.86 (1H, m), 4.49 (2H, q), 6.79 (1H, s), 7.07 (1H, m), 7.16 (2H, t), 7.72 (2H, m), 8.01 (1H, m), 8.28 (1H, m), 8.51 (1H, s), 9.29 (1H, s). |
| 1-160 | 401.2 | 1.80 | 0.54 (2H, m), 0.77 (2H, m), 1.31 (3H, t), 2.89 (1H, m), 4.49 (2H, q), 6.95 (1H, s), 7.07 (1H, m), 7.81 (4H, m), 8.01 (1H, m), 8.23 (1H, m), 8.55 (1H, s), 9.30 (1H, s). |
| 1-161 | 460.1 | 2.64 | 0.53 (2H, m), 0.75 (2H, m), 1.27 (3H, t), 2.88 (1H, m), 4.45 (2H, q), 6.83 (1H, s), 7.07 (1H, m), 7.33 (2H, m), 7.77 (2H, m), 8.03 (1H, m), 8.27 (1H, m), 8.53 (1H, s), 9.30 (1H, s). |
| 1-162 | 424.1 | 2.31 | 0.53 (2H, m), 0.75 (2H, m), 1.28 (6H, d), 2.86 (1H, m), 5.40 (1H, m), 6.82 (1H, m), 7.08 (1H, m), 7.42 (2H, d), 7.63 (2H, d), 8.06 (1H, m), 8.28 (1H, m), 8.52 (1H, s), 9.15 (1H, s). |
| 1-163 | 408.2 | 2.19 | 0.54 (2H, m), 0.76 (2H, m), 1.28 (6H, d), 2.86 (1H, m), 5.40 (1H, m), 6.78 (1H, s), 7.06 (1H, m), 7.17 (2H, t), 7.67 (2H, m), 8.06 (1H, m), 8.28 (1H, m), 8.529.16 (1H, s). |
| 1-164 | 415.1 | 2.27 | 0.55 (2H, m), 0.75 (2H, m), 1.27 (6H, d), 2.87 (1H, m), 5.40 (1H, m), 6.93 (1H, m), 7.06 (1H, m), 7.80 (4H, m), 8.02 (1H, m), 8.27 (1H, m), 8.55 (1H, s), 9.16 (1H, s). |
| 1-165 | 474.1 | 2.32 | 0.54 (2H, m), 0.75 (2H, m), 1.24 (6H, d), 2.86 (1H, m), 5.39 (1H, m), 6.82 (1H, m), 7.07 (1H, m), 7.34 (2H, m), 7.73 (2H, m), 8.05 (1H, m), 8.28 (1H, m), 8.53 (1H, s), 9.18 (1H, s). |
| 1-166 | 464.1 | 2.48 | 0.52 (2H, m), 0.76 (2H, m), 2.86 (1H, m), 5.10 (2H, q), 6.78 (1H, s), 7.22 (1H, m), 7.38 (2H, d), 7.70 (2H, d), 8.04 (1H, m), 8.32 (1H, m), 8.53 (1H, s), 9.26 (1H, s). |
| 1-167 | 448.1 | 2.12 | 0.52 (2H, m), 0.75 (2H, m), 2.86 (1H, m), 5.10 (2H, q), 6.72 (1H, m), 7.14 (2H, q), 7.21 (1H, m), 7.71 (2H, m), 8.02 (1H, m), 8.31 (1H, m), 8.52 (1H, s), 9.26 (1H, s). |
| 1-168 | 455.2 | 2.11 | 0.53 (2H, m), 0.76 (2H, m), 2.89 (1H, m), 5.11 (2H, q), 6.89 (1H, m), 7.22 (2H, m), 7.83 (4H, m), 8.01 (1H, m), 8.31 (1H, m), 8.56 (1H, s), 9.28 (1H, s). |
| 1-169 | 514.0 | 2.84 | 0.52 (2H, m), 0.75 (2H, m), 2.8 (1H, m), 5.11 (2H, q), 6.78 (1H, m), 7.21 (1H, m), 7.31 (2H, m), 7.77 (2H, m), 8.03 (1H, m), 8.32 (1H, m), 8.54 (1H, s), 9.28 (1H, s). |
| 1-170 | 418.3 | 1.59 | 0.50 (2H, m), 0.79 (3H, m), 2.91 (1H, m), 6.50 (1H, m), 7.26 (2H, t), 7.33 (2H, m), 7.93 (1H, m), 8.54 (1H, s), 8.80 (1H, m), 9.72 (1H, s). |
| 1-177 | 394.1 | 1.31 | 0.55 (2H, m), 0.78 (2H, m), 1.063 (3H, t), 2.89 (1H, m), 6.94 (1H, s), 7.28 (1H, m), 7.45 (2H, d), 7.67 (2H, d), 7.88 (1H, d), 8.52 (2H, m), 9.37 (1H, s). |
| 1-178 | 434.2 | 1.76 | 0.49 (2H, m), 0.80 (2H, m), 2.93 (1H, m), 6.56 (1H, m), 7.51 (2H, m), 7.70 (2H, m), 7.82 (1H, m), 7.96 (1H, m), 8.54 (1H, s), 8.81 (1H, m), 9.7 (1H, s). |
| 1-179 | 416.1 | 1.58 | |
| 1-180 | 390.1 | 1.10 | 0.57 (2H, m), 0.78 (2H, m), 1.02 (3H, t), 2.20 (3H, s), 2.42 (2H, q), 2.89 (1H, m), 6.82 (1H, s), 6.96 (1H, m), 7.07 (1H, m), 7.20 (2H, m), 7.68 (1H, m), 8.50 (2H, m), 9.21 (1H, s). |
| 1-181 | 397.2 | 1.22 | 0.58 (2H, m), 0.79 (2H, m), 1.00 (3H, t), 2.25 (3H, s), 2.38 (2H, q), 2.90 (1H, m), 6.97 (1H, m), 7.21 (1H, m), 7.38 (1H, m), 7.61 (1H, m), 7.71 (3H, m), 8.49 (1H, m), 8.52 (1H, s), 9.28 (1H, s). |
| 1-182 | 374.2 | 0.94 | |
| 1-183 | 393.1 | 0.94 | |
| 1-184 | | | (MeOH d4); (0.66 (2H, m), 0.89 (2H, m), 1.10 (3H, t), 2.21 (3H, s), 2.50 (2H, q), 2.88 (1H, m), 7.01 (1H, m), 7.07 (1H, m), 7.27 (1H, m), 7.32 (1H, m), 7.72 (1H, d), 8.49 (1H, m), 8.53 (1H, s). |
| 1-185 | | | (MeOH d4); 0.63 (2H, m), 0.89 (2H, m), 1.10 (3H, t), 2.55 (2H, q), 7.30 (1H, m), 7.36 (2H, d), 7.68 (2H,d), 7.91 (1H, m), 8.52 (2H, m). |
| 1-186 | | | (MeOH d4); 0.65 (2H, m); 0.89 (2H, m), 1.06 (3H, t), 2.49 (2H, q), 2.86 (1H, m), 7.31 (1H, m), 7.76 (4H, s), 7.94 (1H, m), 8.52 (1H, m), 8.56 (1H, s). |
| 1-187 | 412.1 | 1.46 | 0.56 (2H, m), 0.78 (2H, m), 1.05 (3H, t), 2.89 (1H, m), 6.90 (1H, s), 7.25 (1H, m), 7.33 (1H, m), 7.44 (2H, m), 7.76 (1H, m), 8.5 (2H, m), 9.38 (1H, s). |
| 1-188 | 408.2 | 1.40 | 0.58 (2H, m), 0.78 (2H, m), 1.02 (3H, t), 2.20 (3H, s), 2.90 (1H, m), 6.86 (1H, m), 7.20 (3H, m), 7.32 (1H, m), 7.73 (1H, m), 8.50 (2H, m), 9.23 (1H, s). |
| 1-189 | 442.0 | 1.77 | 0.59 (2H, m), 0.78 (2H, m), 0.95 (3H, t), 2.28 (3H, s), 2.91 (1H, m), 6.93 (1H, m), 7.19 (1H, m), 7.40 (1H, m), 7.51(1H, m), 7.60 (1H, m), 7.73 (1H, m), 8.47 (1H, m), 8.53 (1H, s), 9.28 (1H, s). |
| 1-196 | 446.2 | 2.43 | 0.56 (2H, m), 0.79 (2H, m), 1.07 (2H, q), 2.89 (1H, m), 7.04 (1H, m), 7.20 (2H, t), 7.68 (2H, m), 7.80 (1H, d), 8.18 (1H, d), 8.53 (1H, s), 9.56 (1H, s). |
| 1-197 | 440.1 | 2.52 | 0.58 (2H, m); 0.76 (4H, m), 0.86 (2H, m), 1.77 (1H, m); 2.86 (1H, m), 7.18 (1H, m), 7.42 (2H, d), 7.58 (2H, d), 8.14 (1H, m), 8.46 (1H, m), 8.52 (1H, s), 9.62 (1H, s). |
| 1-198 | 424.1 | 2.16 | 0.58 (2H, m), 0.73 (2H, m), 0.79 (2H, m), 0.86 (2H, m), 1.83 (1H, m), 2.87 (1H, m), 7.20 (3H, m), 7.61 (2H, m), 8.11 (1H, m), 8.45 (1H, m), 8.51 (1H, s), 9.61 (1H, s). |
| 1-199 | 448.1 | 2.35 | 0.57 (2H, m), 1.12 (2H, m), 2.47 (3H, s), (2.89 (1H, m), 7.15 (1H, s), 7.47 (2H, d), 7.65 (2H, d), 7.82 (1H, d), 8.16 (1H, d), 8.53 (1H, s), 9.57 (1H, s). |
| 1-224 | 392.1 | 1.36 | |
| 1-229 | 408.1 | 1.58 | |
| 1-230 | 426.1 | 1.72 | |
| 1-254 | 450.1 | 1.83 | |
| 1-258 | 577.3 | 2.07 | |
| 1-262 | 433 | 1.45 | (d7-DMF): 0.87 (8H, m), 3.03 (1H, m), 3.18 (1H, m), 7.39 (2H, t), 7.70 (m, 1H), 7.80 (3H, m), 8.19 (1H, d), 8.55 (1H, s), 8.68 (1H, dd), 9.11 (1H, bd), 9.72 (1H, bd) |
| | | | |
| 1-264 | 591.2 | 1.9 | |
| 1-273 | 509.2 | 1.11 | |
| 1-275 | 374 | 0.76 | 0.55 (2 H, m), 0.76 (2 H, m), 1.05 (3 H, t), 2.53 (2 H, q), 2.92 (1 H, m), 6.63 (1 H, m), 7.24 (1 H, m), 7.38 (3 H, m), 7.60 (2 H, m), 7.80 (1 H, m), 8.51 (1 H, m), 9.27 (1 H, m). |
| 1-276 | 392 | 0.88 | 0.55 (2 H, m), 0.76 (2 H, m), 1.06 (3 H, t), 2.53 (2 H, q), 2.92 (1 H, m), 3.13 (3 H, s), 6.69 (1 H, m), 7.20 (2 H, m), 7.25 (1 H, m), 7.65 (2 H, m), 7.83 (1 H, m), 8.52 (1 H, m), 9.28 (1 H, m). |
| 1-277 | 442/4 44 | 1.34 | 0.55 (2 H, m), 0.77 (2 H, m), 1.03 (3 H, t), 2.45 (2 H, q), 2.93 (1 H, m), 6.66 (1 H, m), 7.21 (1 H, m), 7.43 (2 H, m), 7.60 (1 H, m), 7.68 (1 H, m), 8.49 (1 H, m), 9.22 (1 H, m). |
| 1-278 | 410 | 1 | 0.55 (2 H, m), 0.77 (2 H, m), 1.05 (3 H, t), 2.47 (3 H, s), 2.50 (2 H, q), 2.93 (1 H, m), 6.66 (1 H, m), 7.10 (1 H, m), 7.21 (2 H, m), 7.47 (1 H, m), 7.71 (1 H, m), 8.50 (1 H, m), 9.25 (1 H, m). |
| 1-279 | 458 | 1.44 | 0.55 (2 H, m), 0.76 (2 H, m), 1.03 (3 H, t), 2.92 (1 H, m), 6.75 (1 H, m), 7.24 (1 H, m), 7.35 (2 H, m), 7.73 (2 H, m), 7.85 (1 H, m), 8.52 (1 H, m), 9.31 (1 H, m). |
| 1-287 | 521.2 | 1.45 | |
| 1-290 | 605.2 | 2.47 | |
| 1-299 | 433.1 | 1.85 | 1.17 (3H, CH3), 1.40 (3H, CH3, s),2.75 (2H, CH2), 5.23 (1H, m), 6.08 (1H, d), 7.20 (3H, m), 7.66 (3H, m), 8.14 (1H, s), 8.55 (2H, m) |
| | | | |
| | | | |
| | | | |
| 1-305 | 589.1 | 2.04 | |
| 1-306 | 535.1 | 1.84 | |
| 1-308 | 450 | 1.83 | 0.55 (2 H, m), 0.77 (2 H, m), 2.88 (1 H, m), 6.13 (1 H, dq), 7.18 (3 H, m), 7.67 (3 H, m), 8.23 (1 H, m), 8.54 (1 H, m), 8.80 (1 H, m), 8.89 (1 H, m). |
| 1-309 | 535.2 | 1.77 | |
| 1-310 | 555.1 | 1.84 | |
| 1-315 | 411 | 2.18 | (d3-MeCN): 0.53 (2H, m), 0.83 (2H, m), 0.92 (3H, t), 2.02 (2H, q), 2.95 (1H, m), 6.03 (1H, m), 7.19 (3H, m), 7.31 (2H, m), 7.55 (2H, m), 8.11 (1H, bs), 8.55 (1H, s) |
| 1-316 | 427 | 2.54 | (d3-MeCN): 0.53 (2H, m), 0.83 (2H, m), 0.93 (3H, t), 2.01 (2H, q), 2.94 (1H, m), 6.03 (1H, bs), 7.20 (1H, m), 7.30 (2H, m), 7.48 (3H, m), 7.63 (1H, m),8.09 (1H, bs), 8.56 (1H, s) |
| | | | |
| | | | |
| | | | |
| | | | |

**Tabelle 3**

| | | | | | | | | |
|---|---|---|---|---|---|---|---|---|
| Verbindungen der Formel | | | 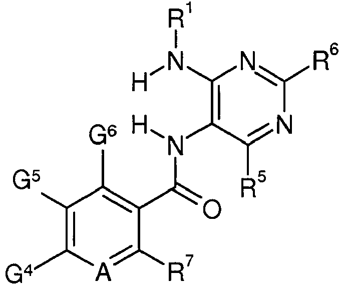 | | | | | |

| **Bsp. Nr** | **R¹** | **R⁵** | **R⁷** | **R⁶** | **A** | **G⁴** | **G⁵** | **G⁶** |
|---|---|---|---|---|---|---|---|---|
| 3-1 | Cyclopropyl | Cl | CH₂CH₃ | H | N | H | H | H |
| 3-2 | Cyclopropyl | H | CH₂CH₃ | H | N | H | H | H |
| 3-3 | Cyclopropyl | Br | CH₂CH₃ | H | N | H | H | H |
| 3-4 | Cyclopropyl | Cl | OCH₂CF₃ | H | N | H | H | H |
| 3-5 | Cyclopropyl | Cl | CH₂CF₃ | H | N | H | H | H |
| 3-6 | Cyclobutyl | Cl | CH₂CH₃ | H | N | H | H | H |
| 3-7 | 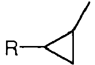 | Cl | CH₂CH₃ | H | N | H | H | H |
| 3-8 | Cyclopropyl | CH₂CH₃ | CH₂CH₃ | H | N | H | H | H |
| 3-9 | Cyclopropyl | Cl | CH₂CH₃ | CH₃ | N | H | H | H |
| 3-10 | CH(CF₃)CH₃ | Cl | CH₂CH₃ | CH₃ | N | H | H | H |
| 3-11 | Cyclopropyl | Cl | CH₂CH₃ | CF₃ | N | H | H | H |
| 3-12 | 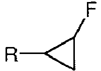 | Cl | CH₂CH₃ | H | N | H | H | H |
| 3-13 | Cyclopropyl | Cyclopropyl | CH₂CF₃ | Cl | N | H | H | H |
| 3-14 | 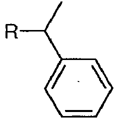 | Cl | CH₂CH₃ | CH₃ | N | H | H | H |
| 3-15 | 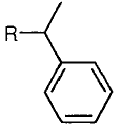 | Cl | CH₂CH₃ | H | N | H | H | H |
| 3-16 | 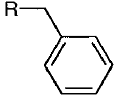 | Cl | CH₂CH₃ | H | N | H | H | H |

**Tabelle 4: Physikochemische Charakterisierung**

| **Bsp. Nr** | **MH+** | **LogP(HCOOH)** |
|---|---|---|
| 3-1 | 318.2 | 0.79 |
| 3-3 | 364.1 | 0.84 |
| 3-4 | 388.0 | 2.25 |
| 3-5 | 370.1 | 1.61 |
| 3-6 | 331.1 | 1.34 |
| 3-7 | 331.1 | 1.26 |
| 3-8 | 312.2 | 1.74 |
| 3-9 | 332.2 | 1.41 |
| 3-10 | 374.0 | 1.65 |
| 3-11 | 384.1 | 2.27 |
| 3-12 | 412.1 | 2.22 |

**Tabelle 7**

| | | | | | | | | | | |
|---|---|---|---|---|---|---|---|---|---|---|
| Verbindungen der Formel | | 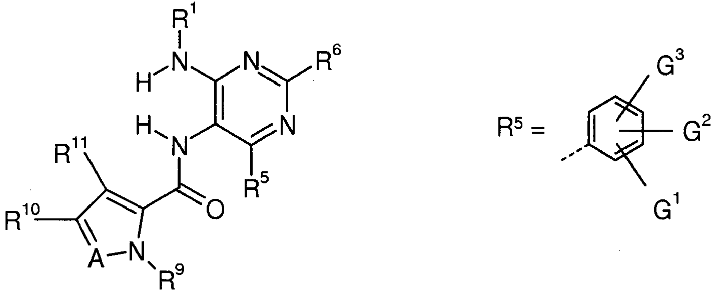 | | | | | | | | |

| **Bsp.Nr.** | **R¹** | **R⁹** | **R¹⁰** | **R¹¹** | **R⁵** | **G¹** | **G²** | **G³** | **R⁶** | **A** |
|---|---|---|---|---|---|---|---|---|---|---|
| 7-1 | Cyclopropyl | CH₂C^{H}3 | H | H | Ph | 4-F | H | H | H | N |
| 7-2 | Cyclopropyl | CH₂CH₃ | H | Cl | Ph | 4-F | H | H | H | N |
| 7-3 | Cyclopropyl | CH₂CH₃ | H | H | Ph | H | H | H | H | N |
| 7-4 | Cyclopropyl | CH₂CH₃ | H | H | Ph | 4-Cl | H | H | H | N |
| 7-5 | Cyclopropyl | CH₂CH₃ | H | H | Ph | 4-OCF₃ | H | H | H | N |
| 7-6 | Cyclopropyl | CH₂CH₃ | H | H | Ph | 4-SCF₃ | H | H | H | N |
| 7-7 | Cyclopropyl | CH₂CH₃ | H | Cl | Ph | H | H | H | H | N |
| 7-8 | Cyclopropyl | CH₂CH₃ | H | Cl | Ph | 4-Cl | H | H | H | N |
| 7-9 | Cyclopropyl | CH₂CH₃ | H | Cl | Ph | 4-OCF₃ | H | H | H | N |
| 7-10 | Cyclopropyl | CH₂CH₃ | H | Cl | Ph | 4-SCF₃ | H | H | H | N |
| 7-11 | Cyclopropyl | CH₂CH₃ | 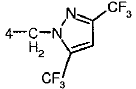 | H | Cl | H | H | H | H | N |
| 7-12 | Cyclopropyl | CH₂CH₃ | H | H | Ph | 4-CN | H | H | H | N |
| 7-13 | Cyclopropyl | CH₂CH₃ | 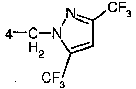 | H | Ph | H | H | H | H | N |
| 7-14 | Cyclopropyl | CH₂CH₃ | 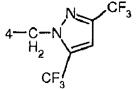 | H | Ph | 2-Cl | 4-Cl | H | H | N |
| 7-15 | Cyclopropyl | CH₂CH₃ | 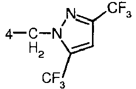 | H | Ph | 4-OCF₃ | H | H | H | N |
| 7-16 | Cyclopropyl | CH₂CH₃ | 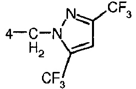 | H | Ph | 4-Cl | H | H | H | N |
| 7-17 | Cyclopropyl | CH₂CH₃ | 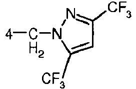 | H | Ph | 4-F | H | H | H | N |
| 7-18 | Cyclopropyl | CH₂CH₃ | H | H | Ph | 2-Cl | 4-Cl | H | H | N |
| 7-19 | Cyclopropyl | CH₂CH₃ | H | Cl | Ph | 2-Cl | 4-Cl | H | H | N |
| 7-20 | Cyclopropyl | CH₂CH₃ | H | Cl | Ph | 4-CN | H | H | H | N |
| 7-21 | Cyclopropyl | CH(CH₃)₂ | H | Cl | Ph | 4-F | H | H | H | N |
| 7-22 | Cyclopropyl | CH(CH₃)₂ | H | Cl | Ph | 4-Cl | H | H | H | N |
| 7-23 | Cyclopropyl | CH₂CF₃ | H | H | Ph | 4-F | H | H | H | N |
| 7-24 | Cyclopropyl | n-Propyl | H | Cl | Ph | 4-F | H | H | H | N |
| 7-25 | Cyclopropyl | n-Propyl | H | Cl | Ph | 4-Cl | H | H | H | N |

**Tabelle 8: Physikochemische Charakterisierung**

| **Bsp. Nr.** | **MH+** | **LogP(HCOOH)** | **NMR (DMSO d6, 400MHz)** |
|---|---|---|---|
| 7-1 | 367 | 1,31 | 0,52 (2 H, m), 0.77 (2 H, m), 1,22 (3 H, t), 2,91 (1 H, m), 4,12 (2 H, q), 6.86 (1 H, m), 7.18 (2 H, m), 7.59 (1 H, s), 7.71 (2 H, m), 8.53 (1 H, s), 9.19 (1 H, s) |
| 7-2 | 401/403 | 1,86 | 0,51 (2 H, m), 0.77 (2 H, m), 1,21 (3 H, t), 2,87 (1 H, m), 4,14 (2 H, q), 7.23 (2 H, m), 7.26 (1 H, m), 7.64 (1 H, s), 7.71 (2 H, m), 8.54 (1 H, s), 9.27 (1 H, s) |
| 7-3 | 349 | 1,12 | 0,56 (2 H, m), 0.73 (2 H, m), 1,18 (3 H, t), 2,87 (1 H, m), 4,30 (2 H, q), 6.84 (1 H, m), 7.12 (1H, m), 7.33 (3 H, m), 7.43 (1 H, m), 7.63 (2 H, m), 8.51 (1 H, s), 9.36 (1 H, s) |
| 7-4 | 383/385 | 1,6 | 0,55 (2 H, m), 0.74 (2 H, m), 1,16 (3 H, t), 2,84 (1 H, m), 4,31 (2 H, q), 6.85 (1 H, m), 7.42 (2 H, m), 7.48 (1 H, m), 7.65 (2 H, m), 8.52 (1 H, s), 9.55 (1 H, s) |
| 7-5 | 433 | 1,99 | 0,57 (2 H, m), 0.74 (2 H, m), 1,14 (3 H, t), 2,88 (1 H, m), 4,29 (2 H, q), 6.87 (1 H, m), 7.22 (1 H, m), 7.30 (2 H, m), 7.44 (1 H, m), 7.75 (2 H, m), 8.52 (1 H, s), 9.39 (1 H, s) |
| 7-6 | 449 | 2,29 | 0,57 (2 H, m), 0.74 (2 H, m), 1,13 (3 H, t), 2,89 (1 H, m), 4,27 (2 H, q), 6.86 (1 H, m), 7.25 (1 H, m), 7.44 (1 H, m), 7.66 (2 H, m), 7.76 (2 H, m), 8.53 (1 H, s), 9.41 (1 H, s) |
| 7-7 | 383 | 1,63 | 0,52 (2 H, m), 0.77 (2 H, m), 1,19 (3 H, t), 2,91 (1 H, m), 4,09 (2 H, q), 6.78 (1 H, m), 7.37 (3 H, m), 7.58 (1 H, m), 7.65 (2 H, s), 8.54 (1 H, s), 9.21 (1 H, s) |
| 7-8 | 417/419 | 2,20 | 0,52 (2 H, m), 0.77 (2 H, m), 1,20 (3 H, t), 2,91 (1 H, m), 4,12 (2 H, q), 6.90 (1 H, m), 7.43 (2 H, m), 7.59 (1 H, m), 7.64 (1 H, s), 7.68 (2 H, m), 8.54 (1 H, s), 9.20 (1 H, s) |
| 7-9 | 467/469 | 2,63 | 0,52 (2 H, m), 0.77 (2 H, m), 1,20 (3 H, t), 2,92 (1 H, m), 4,11 (2 H, q), 6.88 (1 H, m), 7.35 (2 H, m), 7.58 (1 H, s), 7.75 (2 H, m), 8.55 (1 H, s), 9.24 (1 H, s) |
| 7-10 | 483/485 | 2,99 | 0,52 (2 H, m), 0.77 (2 H, m), 1,19 (3 H, t), 2,92 (1 H, m), 4,10 (2 H, q), 6.88 (1 H, m), 7.58 (1 H, s), 7.74 (4 H, m), 8.56 (1 H, s), 9.25 (1 H, s) |
| 7-11 | 523/525 | 3,29 | 0,52 (2 H, m), 0.72 (2 H, m), 1,31 (3 H, t), 2,83 (1 H, m), 4,45 (2 H, q), 5.57 (2 H, s), 6.94 (1 H, m), 7.49 (2 H, m), 8.27 (1 H, s), 9.57 (1 H, s) |
| 7-12 | 374 | 1,45 | 0,58 (2 H, m), 0.74 (2 H, m), 1,15 (3 H, t), 2,88 (1 H, m), 4,28 (2 H, q), 6.86 (1 H, m), 7.31 (1 H, m), 7.45 (1 H, m), 7.79 (4 H, m), 8.54 (1 H, s), 9.40 (1 H, s) |
| 7-13 | 565 | 2,80 | 0,53 (2 H, m), 0.72 (2 H, m), 1,17 (3 H, t), 2,86 (1 H, m), 4,27 (2 H, q), 5.52 (2 H, s), 6.72 (1 H, m), 7.18 (1 H, m), 7.30 (3 H, m), 7.51 (1 H, s), 7.60 (2 H, m), 8.50 (1 H, s), 9.41 (1 H, s) |
| 7-14 | 633/635 | 3,79 | 0,54 (2 H, m), 0.73 (2 H, m), 1,14 (3 H, t), 2,87 (1 H, m), 4,23 (2 H, q), 5.50 (2 H, s), 6.62 (1 H, m), 7.21 (1 H, m), 7.28 (2 H, m), 7.49 (2 H, m), 8.49 (1 H, s), 9.29 (1 H, s) |
| 7-15 | 649 | 3,76 | 0,54 (2 H, m), 0.73 (2 H, m), 1,14 (3 H, t), 2,86 (1 H, m), 4,26 (2 H, q), 5.53 (2 H, s), 6.74 (1 H, m), 7.26 (3 H, m), 7.50 (1 H, s), 7,71 (2 H, m), 8.50 (1 H, s), 9.43 (1 H, s) |
| 7-16 | 599/601 | 3,41 | 0,53 (2 H, m), 0.72 (2 H, m), 1,17 (3 H, t), 2,85 (1 H, m), 4,28 (2 H, q), 5.53 (2 H, s), 6.77 (1 H, m), 7.26 (1 H, m), 7.35 (2 H, m), 7.50 (1 H, m), 7,60 (2 H, m), 8.50 (1 H, s), 9.42 (1 H, s) |
| 7-17 | 583 | 3,05 | 0,52 (2 H, m), 0.73 (2 H, m), 1,17 (3 H, t), 2,85 (1 H, m), 4,28 (2 H, q), 5.54 (2 H, s), 6.74 (1 H, m), 7.11 (2 H, m), 7.23 (1 H, m), 7.51 (1 H, s), 7.66 (2 H, m), 8.49 (1 H, s), 9.41 (1 H, s) |
| 7-18 | 417/419 | 1,95 | 0,57 (2 H, m), 0.76 (2 H, m), 1,15 (3 H, t), 2,88 (1 H, m), 4,27 (2 H, q), 6.73 (1 H, m), 7.17 (1 H, m), 7.3 - 7.6 (4 H, m), 8.59 (1 H, s), 9.25 (1 H, s) |
| 7-19 | 451/453 | 2,57 | |
| 7-20 | 408/410 | 1,90 | 0,53 (2 H, m), 0.76 (2 H, m), 1,20 (3 H, t), 2,91 (1 H, m), 4,11 (2 H, q), 7.03 (1 H, m), 7.58 (1 H, m), 7.82 (4 H, m), 8.56 (1 H, s), 9.21 (1 H, s) |
| 7-21 | 415 | 2.18 | 0.49 (2H, m), 0.78 (2H, m), 1.27 (6H, d), 2.91 (1H, m), 4.55 (1H, m), 6.66 (1H, bs), 7.20 (2H, m), 7.61 (1H, s), 7.70 (2H, m), 8.54 (1H, s), 9.36 (1H, bs) |
| 7-22 | 431 | 2.58 | 0.49 (2H, m), 0.78 (2H, m), 1.27 (6H, d), 2.92 (1H, m), 4.54 (1H, m), 6.69 (1H, bs), 7.44 (2H, m), 7.61 (1H, s), 7.67 (2H, m), 8.54 (1H, s), 9.37 (1H, bs) |
| 7-23 | 421 | 1.68 | 0.54 (2H, m), 0.75 (2H, m), 2.86 (1H, m), 5.34 (2H, m), 7.09 (1H, s), 7.17 (2H, m), 7.39 (1H, s), 7.68 (3H, m), 8.54 (1H, s), 9.76 (1H, bs) |
| 7-24 | 415 | 2.13 | 0.49 (2H, m), 0.75 (5H, m), 1.57 (2H, m), 2.88 (1H, m), 4.06 (t, 2H), 7.07 (1H, m), 7.22 (2H, m), 7.64 (1H, m), 7.73 (2H, m), 8.54 (1H, s), 9.30 (1H, bs) |
| 7-25 | 431 | 2.51 | 0.49 (2H, m), 0.74 (5H, m), 1.57 (2H, m), 2.88 (1H, m), 4.05 (t, 2H), 7.10 (1H, m), 7.46 (2H, m), 7.65 (1H, m), 7.69 (2H, m), 8.55 (1H, s), 9.31 (1H, bs) |

**Tabelle 9**

| | | | | | | | | | | | |
|---|---|---|---|---|---|---|---|---|---|---|---|
| Verbindungen der Formel | | 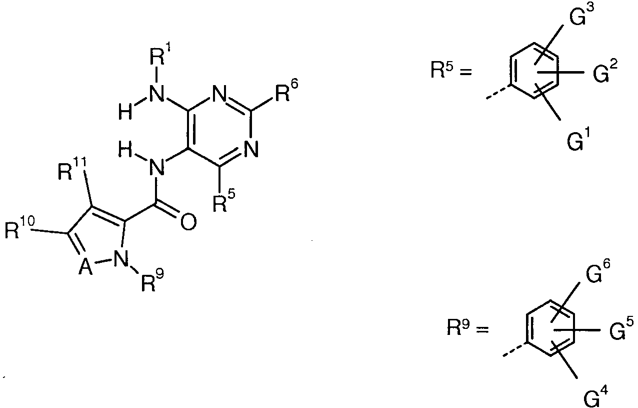 | | | | | | | | | |

| **Bsp. Nr** | **R¹** | **G⁴** | **G⁵** | **G⁶** | **R¹⁰** | **R¹¹** | **G¹** | **G²** | **G³** | **R⁶** | **A** |
|---|---|---|---|---|---|---|---|---|---|---|---|
| 9-1 | Cyclopropyl | 4-F | H | H | H | H | 4-F | H | H | H | N |
| 9-2 | Cyclopropyl | 4-Cl | H | H | H | H | 4-F | H | H | H | N |
| 9-3 | Cyclopropyl | 2-Cl | H | H | H | H | 4-F | H | H | H | N |
| 9-4 | Cyclopropyl | 2-Cl | H | H | H | H | 4-Cl | H | H | H | N |

**Tabelle 10: Physikochemische Charakterisierung**

| **Bsp. Nr** | **MH+** | **LogP(HCOOH)** | **NMR (DMSO d6, 400MHz)** |
|---|---|---|---|
| 9-1 | 433 | 1.70 | 0.57 (2H, m), 0.78 (2H, m), 2.87 (1H, m), 7.06 (1H, s), 7.20 (4H, m), 7.32 (2H, m), 7.51 (1H, m), 7.73 (3H, m), 8.50 (1H, s), 9.69 (1H, bs) |
| 9-2 | 449 | 2.02 | 0.57 (2H, m), 0.77 (2H, m), 2.87 (1H, m), 7.07 (1H, s), 7.18 (2H, m), 7.31 (2H, m), 7.49 (3H, m), 7.66 (2H, m), 7.76 (1H, s), 8.51 (1H, s), 9.70 (1H, bs) |
| 9-3 | 449 | 1.76 | 0.52 (2H, m), 0.73 (2H, m), 2.81 (1H, m), 7.18 (4H, m), 7.27 (1H, m), 7.35 (1H, m), 7.42 (1H, m), 7.49 (1H, m), 7.67 (2H, m), 7.80 (1H, m), 8.48 (1H, s), 9.68 (1H, bs) |
| 9-4 | 465 | 2.05 | 0.53 (2H, m), 0.73 (2H, m), 2.81 (1H, m), 7.18 (1H, m), 7.24 (2H, m), 7.35 (1H, m), 7.44 (3H, m), 7.52 (1H, m), 7.63 (2H,m), 7.81 (1H, m), 8.49 (1H, s), 9.68 (1H, bs) |

### Biologische Beispiele

### Lucilia cuprina-Test

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Gefäße, die Pferdefleisch enthalten, das mit der Wirkstoffzubereitung der gewünschten Konzentration behandelt wurde, werden mit *Lucilia cuprina* Larven besetzt.

Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z. B. die folgenden Verbindungen der Herstellungsbeispiele eine Wirkung von ≥ 80 % bei einer Aufwandmenge von 100 ppm:
Bsp Nr : 1-1, 1-21, 1-4, 1-5, 1-73, 1-74, 1-75, 1-76, 1-82, 1-83, 1-85, 1-100, 1-111, 1-114, 1-116, 1-123, 1-124, 1-126, 1-133, 1-177, 1-179, 1-180, 1-181, 1-185, 1-186, 1-187, 1-188, 1-190

### Musca domestica-Test

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Gefäße, die einen Schwamm enthalten, der mit der Wirkstoffzubereitung der gewünschten Konzentration behandelt wurde, werden mit *Musca domestica Adulten* besetzt.

Nach 2 Tagen wird die Abtötung in % bestimmt. Dabei bedeutet 100 %, dass alle Fliegen abgetötet wurden; 0 % bedeutet, dass keine Fliegen abgetötet wurden.

Bei diesem Test zeigen z. B. die folgenden Verbindungen der Herstellungsbeispiele eine Wirkung von ≥ 80 % bei einer Aufwandmenge von 100 ppm:
Bsp Nr : 1-1, 1-4, 1-73, 1-75, 1-114, 1-124, 1-126, 1-177, 1-179, 1-180, 1-181, 1-185, 1-186, 1-187,1-190

### Boophilus microplus -Test (Injektion)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Lösungsmittel und verdünnt das Konzentrat mit Wasser auf die gewünschte Konzentration. Die Wirkstofflösung wird in das Abdomen *(Boophilus microplus)* injiziert, die Tiere werden in Schalen überführt und in einem klimatisierten Raum aufbewahrt. Die Wirkungskontrolle erfolgt auf Ablage fertiler Eier.

Nach 7 Tagen wird die Wirkung in % bestimmt. Dabei bedeutet 100%, dass keine Zecke fertile Eier gelegt hat.

Bei diesem Test zeigen z. B. die folgenden Verbindungen der Herstellungsbeispiele eine Wirkung von ≥ 80 % bei einer Aufwandmenge von 20 µg / Tier:
Bsp Nr : 1-5, 1-100, 1-111, 1-177, 1-187

### Myzus-Test (Spritzbehandlung)

| | |
|---|---|
| Lösungsmittel: | 78 GewichtsteileAceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele eine Wirkung von ≥ 80 % bei einer Aufwandmenge von 500 ppm:
Bsp Nr : 1-1, 1-10, 1-11, 1-114, 1-115, 1-116, 1-121, 1-123, 1-124, 1-126, 1-127, 1-129, 1-13, 1-130, 1-131, 1-132, 1-133, 1-139, 1-145, 1-146, 1-147, 1-148, 1-149, 1-150, 1-152, 1-154, 1-16, 1-174, 1-175, 1-176, 1-177, 1-178, 1-179, 1-180, 1-181, 1-183, 1-184, 1-185, 1-186, 1-187,1-188, 1-189, 1-19, 1-190,1-20, 1-21, 1-26, 1-27, 1-22, 1-23, 1-24, 1-25, 1-29, 1-30, 1-31, 1-32, 1-33, 1-37, 1-38, 1-4, 1-40, 1-44, 1-48, 1-5, 1-52, 1-59, 1-60, 1-7, 1-70, 1-71, 1-8, 1-9, 1-100, 1-73, 1-85, 1-99

### Phaedon-Test (Spritzbehandlung)

| | |
|---|---|
| Lösungsmittel: | 78,0 GewichtsteileAceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele eine Wirkung von ≥ 80 % bei einer Aufwandmenge von 500 ppm:
Bsp Nr : 1-1, 1-11, 1-114, 1-115, 1-116, 1-119, 1-122, 1-123, 1-124, 1-126, 1-127, 1-129, 1-13, 1-130, 1-131, 1-132, 1-133, 1-134, 1-135, 1-136, 1-137, 1-139, 1-14, 1-140, 1-141, 1-142, 1-143, 1-144, 1-149, 1-15, 1-151, 1-154, 1-156, 1-157, 1-158, 1-159, 1-16, 1-160, 1-161, 1-162, 1-163, 1-164, 1-165, 1-167, 1-169, 1-170, 1-172, 1-173, 1-174, 1-175, 1-176, 1-177, 1-178, 1-179, 1-180, 1-181, 1-183, 1-184, 1-185, 1-186, 1-187, 1-188, 1-189, 1-19, 1-190, 1-191, 1-192, 1-193, 1-194, 1-195, 1-2, 1-20, 1-21, 1-25, 1-29, 1-3, 1-30, 1-33, 1-34, 1-37, 1-38, 1-4, 1-40, 1-43, 1-44, 1-45, 1-46, 1-47, 1-48, 1-49, 1-5, 1-52, 1-56, 1-58, 1-6, 1-60, 1-61, 1-64, 1-67, 1-68, 1-69, 1-7, 1-70, 1-71,1-83, 1-84, 1-87, 1-9, 1-111, 1-72, 1-73, 1-74, 1-75, 1-76, 1-82, 1-83, 1-85, 1-89, 1-99

### Spodoptera frugiperda-Test (Spritzbehandlung)

| | |
|---|---|
| Lösungsmittel: | 78,0 GewichtsteileAceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele eine Wirkung von ≥ 80 % bei einer Aufwandmenge von 500 ppm:
Bsp Nr : 1-1, 1-10, 1-11, 1-114, 1-115, 1-12, 1-123, 1-124, 1-126, 1-127, 1-129, 1-13, 1-130, 1-131, 1-132, 1-133, 1-134, 1-135, 1-139, 1-14, 1-143, 1-146, 1-147, 1-149, 1-15, 1-151, 1-152, 1-155, 1-159, 1-16, 1-160, 1-164, 1-167, 1-170, 1-172, 1-173, 1-174, 1-176, 1-177, 1-183, 1-185, 1-186, 1-187, 1-188, 1-189, 1-19, 1-190, 1-191, 1-192, 1-2, 1-20, 1-21, 1-22, 1-25, 1-29, 1-3, 1-30, 1-4, 1-5, 1-6, 1-67, 1-7, 1-70, 1-71, 1-8, 1-9, 1-111, 1-72, 1-73, 1-74, 1-75, 1-76, 1-83, 1-85, 1-86, 1-99

### Tetranychus-Test, OP-resistent (Spritzbehandlung)

| | |
|---|---|
| Lösungsmittel: | 78,0 GewichtsteileAceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben *(Phaseolus vulgaris)*, die von allen Stadien der Gemeinen Spinnmilbe *(Tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von ≥ 80 % bei einer Aufwandmenge von 500 ppm:
Bsp Nr : 1-116, 1-192, 1-199, 1-5, 1-83

### Boophilus microplus -Test (Injektion)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Lösungsmittel und verdünnt das Konzentrat mit Wasser auf die gewünschte Konzentration. Die Wirkstofflösung wird in das Abdomen *(Boophilus microplus)* injiziert, die Tiere werden in Schalen überführt und in einem klimatisierten Raum aufbewahrt. Die Wirkungskontrolle erfolgt auf Ablage fertiler Eier.

Nach 7 Tagen wird die Wirkung in % bestimmt. Dabei bedeutet 100%, dass keine Zecke fertile Eier gelegt hat.

Bei diesem Test zeigen z.B. die folgenden Verbindungen der Herstellungsbeispiele eine Wirkung von ≥ 80 % bei einer Aufwandmenge von 20µg /Tier

| | | | |
|---|---|---|---|
| 1-222 | 1-225 | 1-245 | 1-274 |
| 1-224 | 1-235 | 1-254 | 1-299 |

### Lucilia cuprina (48h)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.Gefäße, die Pferdefleisch enthalten, das mit der Wirkstoffzubereitung der gewünschten Konzentration behandelt wurde, werden mit *Lucilia cuprina* Larven besetzt.

Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von ≥ 80 % bei einer Aufwandmenge von 100 ppm

| | | | | | |
|---|---|---|---|---|---|
| 1-127 | 1-176 | 1-234 | 1-246 | 1-279 | 7-1 |
| 1-131 | 1-222 | 1-235 | 1-254 | 1-299 | 7-2 |
| 1-132 | 1-224 | 1-238 | | 1-305 | 7-20 |
| 1-147 | 1-225 | 1-239 | 1-258 | 1-308 | |
| 1-172 | 1-230 | 1-245 | 1-274 | 1-326 | |

### Musca domestica - Test

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration. Gefäße, die einen Schwamm enthalten, der mit der Wirkstoffzubereitung der gewünschten Konzentration behandelt wurde, werden mit *Musca domestica Adulten* besetzt.

Nach 2 Tagen wird die Abtötung in % bestimmt. Dabei bedeutet 100 %, dass alle Fliegen abgetötet wurden; 0 % bedeutet, dass keine Fliegen abgetötet wurden.

Bei diesem Test zeigen z.B. die folgenden Verbindungen der Herstellungsbeispiele eine Wirkung von ≥ 80 % bei einer Aufwandmenge von 100 ppm

| | | | | |
|---|---|---|---|---|
| 1-147 | 1-238 | 1-246 | 1-258 | 1-258 |
| 1-172 | 1-239 | 1-254 | 1-274 | 1-274 |
| 1-234 | 1-245 | 1-255 | 1-279 | 1-279 |
| 7-2 | | | | |

Bei diesem Test zeigen z.B. die folgenden Verbindungen der Herstellungsbeispiele eine Wirkung von von ≥ 80 % bei einer Aufwandmenge von 20 ppm
1-127, 1-222, 1-224, 1-225, 1-230, 1-299, 7-20

### Myzus-Test (Spritzbehandlung)

| | |
|---|---|
| Lösungsmittel: | 78 GewichtsteileAceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von ≥ 80 % bei einer Aufwandmenge von 500g / ha

| | | | | | | | | |
|---|---|---|---|---|---|---|---|---|
| 1-22 | 1-229 | 1-242 | 1-272 | 1-279 | 1-314 | 3-4 | 3-12 | 7-23 |
| 1-222 | 1-230 | 1-246 | 1-274 | 1-289 | 1-316 | 3-5 | 7-1 | 9-1 |
| 1-223 | 1-231 | 1-254 | 1-275 | 1-299 | 1-322 | 3-6 | 7-2 | |
| 1-224 | 1-235 | 1-258 | 1-276 | 1-301 | 1-325 | 3-7 | 7-7 | |
| 1-225 | 1-236 | 1-261 | 1-277 | 1-307 | 1-326 | 3-8 | 7-20 | |
| 1-226 | 1-239 | 1-271 | 1-278 | 1-308 | 3-1 | 3-11 | 7-21 | |

### Phaedon-Test (Spritzbehandlung)

| | |
|---|---|
| Lösungsmittel: , | 78,0 GewichtsteileAceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben *(Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von ≥ 80 % bei einer Aufwandmenge von 500g / ha

| | | | | | | | |
|---|---|---|---|---|---|---|---|
| 1-221 | 1-238 | | 1-283 | 1-301 | | 7-11 | 7-25 |
| 1-222 | 1-239 | | 1-284 | | | 7-13 | 7-3 |
| 1-223 | 1-241 | 1-271 | 1-285 | | | 7-14 | 7-4 |
| 1-224 | 1-242 | 1-274 | 1-289 | | | 7-15 | 7-5 |
| 1-225 | 1-246 | 1-275 | | 1-307 | | 7-16 | 7-6 |
| 1-226 | 1-250 | 1-276 | | 1-308 | | 7-17 | 7-7 |
| 1-227 | 1-254 | 1-277 | | 1-315 | 3-13 | 7-18 | 7-8 |
| 1-229 | | 1-278 | | | 3-7 | 7-2 | 7-9 |
| 1-230 | | 1-279 | | | 3-8 | 7-20 | 9-1 |
| 1-231 | 1-258 | 1-280 | | 1-322 | 3-9 | 7-21 | 9-2 |
| 1-235 | 1-260 | 1-281 | 1-299 | 1-325 | 7-1 | 7-22 | 9-3 |
| 1-236 | 1-261 | 1-282 | | 1-326 | 7-10 | 7-24 | 9-4 |

### Spodoptera frugiperda-Test (Spritzbehandlung)

| | |
|---|---|
| Lösungsmittel: | 78,0 GewichtsteileAceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von ≥ 80 % bei einer Aufwandmenge von 500g / ha

| | | | | | | | | |
|---|---|---|---|---|---|---|---|---|
| 1-221 | 1-231 | 1-254 | 1-271 | | 1-308 | | 3-7 | 7-25 |
| 1-222 | 1-235 | | 1-272 | | 1-314 | | 3-9 | 7-4 |
| 1-224 | 1-236 | | 1-274 | | 1-315 | | 7-19 | 7-7 |
| 1-225 | 1-238 | 1-258 | 1-276 | 1-299 | 1-316 | | 7-2 | 7-8 |
| 1-226 | 1-239 | 1-260 | 1-277 | 1-301 | | 3-10 | 7-20 | |
| 1-227 | 1-241 | 1-261 | 1-278 | | 1-322 | 3-12 | 7-21 | |
| 1-228 | 1-242 | 1-262 | 1-293 | | 1-325 | 3-13 | 7-22 | |
| 1-229 | 1-246 | | | | 1-326 | 3-14 | 7-23 | |
| 1-230 | 1-250 | 1-270 | | 1-307 | | 3-15 | 7-24 | |

### Tetranychus-Test; OP-resistent (Spritzbehandlung)

| | |
|---|---|
| Lösungsmittel: | 78,0 GewichtsteileAceton |
| | 1,5 Gewichtsteile Dimethylformamid |
| | |
| Emulgator : | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bohnenblattscheiben *(Phaseolus vulgaris),* die von allen Stadien der Gemeinen Spinnmilbe *(tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von von ≥ 80 % bei einer Aufwandmenge von 500g / ha

| | | |
|---|---|---|
| 1-258 | 1-275 | |
| | | 7-2 |

### Spodoptera litura-Test (Spritzbehandlung)

| | |
|---|---|
| Lösungsmittel: | 3 Gewichtsteile Dimethylformamid |
| Emulgator: | 1 Gewichtsteil Polyoxyethylene alkylphenylether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Blätter der Süßkartoffel (*pomoea batatas*) werden mit einer Wirkstoffzubereitung der gewünschten Konzentration getaucht und nach dem Abtrocknen mit Raupen des Baumwollwurms (*spodoptera litura*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von 100 % bei einer Aufwandmenge von 100 ppm
1-177, 1-190, 1-232, 1-234, 1-235, 1-236, 1-237, 1-238, 1-239, 1-240, 1-241, 1-242, 1-243, 1-244, 1-245, 1-246, 1-247, 1-248, 1-252, 1-253, 1-254, 11-258, 1-259, 1-264, 1-266, 1-267, 1-268, 1-269, 1-288, 1-305, 1-310, 1-311, 1-312, 1-313, 1-314, 1-317, 1-318, 1-319, 1-324, 1-325, 1-326,

### Aulacophora femoralis -Test (Spritzbehandlung)

| | |
|---|---|
| Lösungsmittel: | 3 Gewichtsteile Dimethylformamid |
| Emulgator: | 1 Gewichtsteil Polyoxyethylene alkylphenylether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Blätter der Gurke (*cucumis sativus*) werden mit einer Wirkstoffzubereitung der gewünschten Konzentration getaucht und nach dem Abtrocknen mit Larven der Aulacophora femoralis besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von 100 % bei einer Aufwandmenge von 500 ppm
1-177, 1-190, 1-232, 1-233, 1-234, 1-235, 1-236,1-237, 1-238, 1-239, 1-240, 1-241, 1-242, 1-243, 1-244, 1-245, 1-246, 1-247, 1-248, 1-249, 1-251, 1-252, 1-253, 1-254, 1-258, 1-259, 1-260, 1-261, 1-264, 1-266, 1-267, 1-268, 1-269, 1-270, 1-273, 1-287, 1-288, 1-290, 1-292, 1-305, 1-306, 1-309, 1-310, 1-311, 1-312, 1-314, 1-318, 1-319, 1-322, 1-324, 1-325, 1-326, 3-13, 3-16,

## Patentansprüche

1. Verbindungen der Formel (I) 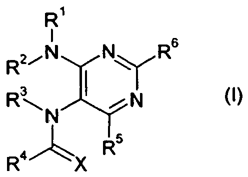 in welcher
R¹ für einen Rest aus der Reihe C₁-C₆-Alkyl, C₂-C₆-Alkenyl, C₂-C₆-Alkinyl, C₁-C₆-Haloalkyl, Cyano-C₁-C₆-alkyl, C₃-C₈-Cycloalkyl, C₃-C₈-Halocycloalkyl, C₃-C₈-Cycloalkenyl, C₃-C₈-Halocycloalkenyl, C₁-C₆-Alkoxy-C₁-C₆-alkyl, C₁-C₆-Haloalkoxy-C₁-C₆-alkyl, C₁-C₆-Alkoxy-C₁-C₆-haloalkyl, C₁-C₆-Alkylcarbonyl-C₁-C₆-alkyl, C₁-C₆-Alkoxycarbonyl-C₁-C₆-alkyl, C₁-C₆-Alkylsulfanyl-C₁-C₆-alkyl, C₁-C₆-Alkylsulfinyl-C₁-C₆-alkyl, C₁-C₆-Alkylsulfonyl-C₁-C₆-alkyl, C₁-C₆-Haloalkylsulfanyl-C₁-C₆-alkyl, C₁-C₆-Haloalkylsulfinyl-C₁-C₆-alkyl, C₁-C₆-Haloalkylsulfonyl-C₁-C₆-alkyl, C₃-C₈-Cycloalkyl-C₁-C₆-alkyl, halogeniertes C₃-C₈-Cycloalkyl-C₁-C₆-alkyl, Phenyl-C₁-C₆-alkyl, welches am Phenylring gegebenenfalls einfach oder mehrfach, gleich oder verschieden substituiert ist durch Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Alkylsulfinyl, C₁-C₆-Alkylsulfonyl, C₁-C₆-Haloalkylsulfanyl, C₁-C₆-Haloalkylsulfinyl, C₁-C₆-Haloalkylsulfonyl, Nitro oder Cyano; Heteroaryl, welches gegebenenfalls einfach oder mehrfach, gleich oder verschieden substituiert ist durch -OH, -SH, -NH₂, Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Alkylsulfinyl, C₁-C₆-Alkylsulfonyl, C₁-C₆-Haloalkylsulfanyl, C₁-C₆-Haloalkylsulfinyl, C₁-C₆-Haloalkylsulfonyl, Mono-C₁-C₆-alkylamino, Di-C₁-C₆-alkylamino, Nitro oder Cyano; Heteroaryl-C₁-C₆-alkyl, welches am Heteraromaten gegebenenfalls einfach oder mehrfach, gleich oder verschieden substituiert ist durch -OH, -SH, -NH₂, Halo-gen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Alkylsulfinyl, C₁-C₆-Alkylsulfonyl, C₁-C₆-Haloalkylsulfanyl, C₁-C₆-Haloalkylsulfinyl, C₁-C₆-Haloalkylsulfonyl, Mono-C₁-C₆-alkylamino, Di-C₁-C₆-alkylamino, Nitro oder Cyano; Heterocyclyl, welches gegebenenfalls einfach oder mehrfach, gleich oder verschieden substituiert ist durch -OH / =O, -SH / =S, - NH₂, Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Alkylsulfinyl, C₁-C₆-Alkylsulfonyl, C₁-C₆-Haloalkylsulfanyl, C₁-C₆-Haloalkylsulfinyl, C₁-C₆-Hatoalkylsulfonyl, Mono-C₁-C₆-alkylamino, Di-C₁-C₆-alkylamino, Nitro oder Cyano und Heterocyclyl-C₁-C₆-alkyl steht, welches am Heterocyclus gegebenenfalls einfach oder mehrfach, gleich oder verschieden substituiert ist durch -OH / =O, -SH / =S, -NH₂, Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Alkylsulfinyl, C₁-C₆-Alkylsulfonyl, C₁-C₆-Haloalkylsulfanyl, C₁-C₆-Haloalkylsulfinyl, C₁-C₆-Haloalkylsulfonyl, Mono-C₁-C₆-alkylamino, Di-C₁-C₆-alkylamino, Nitro oder Cyano,
R² für einen Rest aus der Reihe Wasserstoff, C₁-C₆-Alkyl, C₂-C₆-Alkenyl, C₂-C₆-Alkinyl, C₁-C₆-Haloalkyl, Cyano-C₁-C₆-alkyl, C₃-C₈-Cycloalkyl, C₃-C₈-Halocycloalkyl, C₃-C₈-Cycloalkenyl, C₃-C₈-Halocycloalkenyl, C₁-C₆-Alkoxy-C₁-C₆-alkyl, C₁-C₆-Haloalkoxy-C₁-C₆-alkyl, C₁-C₆-Alkylcarbonyl-C₁-C₆-alkyl, C₁-C₆-Alkoxycarbonyl-C₁-C₆-alkyl, C₁-C₆-Alkylsulfanyl-C₁-C₆-alkyl, C₁-C₆-Alkylsulfinyl-C₁-C₆-alkyl, C₁-C₆-Alkylsulfonyl-C₁-C₆-alkyl, C₁-C₆-Haloalkylsulfanyl-C₁-C₆-alkyl, C₁-C₆-Haloalkylsulfinyl-C₁-C₆-alkyl, C₁-C₆-Haloalkylsulfonyl-C₁-C₆-alkyl, C₃-C₈-Cycloalkyl-C₁-C₆-alkyl, Phenyl-C₁-C₆-alkyl, welches am Phenylring gegebenenfalls einfach oder mehrfach, gleich oder verschieden substituiert ist durch Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Alkylsulfinyl, C₁-C₆-Alkylsulfonyl, C₁-C₆-Haloalkylsulfanyl, C₁-C₆-Haloalkylsulfinyl, C₁-C₆-Haloalkylsulfonyl, Nitro oder Cyano; Heteroaryl, welches gegebenenfalls einfach oder mehrfach, gleich oder verschieden substituiert ist durch -OH, -SH, -NH₂, Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Alkylsulfinyl, C₁-C₆-Alkylsulfonyl, C₁-C₆-Haloalkylsulfanyl, C₁-C₆-Haloalkylsulfinyl, C₁-C₆-Haloalkylsulfonyl, Mono-C₁-C₆-alkylamino, Di-C₁-C₆-alkylamino, Nitro oder Cyano; Heteroaryl-C₁-C₆-alkyl, welches am Heteraromaten gegebenenfalls einfach oder mehrfach, gleich oder verschieden substituiert ist durch -OH, -SH, -NH₂, Halogen, C₁-C₆-Alkyl, C₁-C₆-Haloalkyl, C₁-C₆-Alkoxy, C₁-C₆-Hatoalkoxy, C₁-C₆-Alkylsulfanyl, C₁-C₆-Alkylsulfinyl, C₁-C₆-Alkylsulfonyl, C₁-C₆-Haloalkylsulfanyl, C₁-C₆-Haloalkylsulfinyl, C₁-C₆-Haloalkylsulfonyl, Mono-C₁-C₆-alkylamino, Di-C₁-C₆-alkylamino, Nitro oder Cyano; Heterocyclyl, welches gegebenenfalls einfach oder mehrfach, gleich oder verschieden substituiert ist durch -OH / =O, -SH / =S, -NH₂, Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Alkylsulfinyl, C₁-C₆-Alkylsulfonyl, C₁-C₆-Haloalkylsulfanyl, C₁-C₆-Haloalkylsulfinyl, C₁-C₆-Haloalkylsulfonyl, Mono-C₁-C₆-alkylamino, Di-C₁-C₆-alkylamino, Nitro oder Cyano und Heterocyclyl-C₁-C₆-alkyl steht, welches am Heterocyclus gegebenenfalls einfach oder mehrfach, gleich oder verschieden substituiert ist durch -OH / =O, - SH / =S, -NH₂, Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Alkylsulfinyl, C₁-C₆-Alkylsulfonyl, C₁-C₆-Haloalkylsulfanyl, C₁-C₆-Haloalkylsulfinyl, C₁-C₆-Haloalkylsulfonyl, Mono-C₁-C₆-alkylamino, Di-C₁-C₆-alkylamino, Nitro oder Cyano,
R³ für einen Rest aus der Reihe Wasserstoff, C₁-C₆-Alkyl, C₂-C₆-Alkenyl, C₂-C₆-Alkinyl, Halo-C₁-C₆-alkyl, Cyano-C₁-C₆-alkyl, C₃-C₈-Cycloalkyl, Halo-C₃-C₈-Cycloalkyl, C₃-C₈-Cycloalkenyl, Halo-C₃-C₈-Cycloalkenyl, C₁-C₆-Alkoxy-C₁-C₆-alkyl, C₁-C₆-Alkylcarbonyl-C₁-C₆-alkyl, C₁-C₆-Alkoxycarbonyl-C₁-C₆-alkyl, C₁-C₆-Alkylsulfanyl-C₁-C₆-alkyl, C₁-C₆-Alkylsulfinyl-C₁-C₆-alkyl, C₁-C₆-Alkylsulfonyl-C₁-C₆-alkyl, C₃-C₈-Cycloalkyl-C₁-C₆-alkyl, Phenyl, welches gegebenenfalls einfach oder mehrfach, gleich oder verschieden substituiert ist durch -OH, -SH, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, Mono-C₁-C₆-alkylamino, Di-C₁-C₆-alkylamino, Nitro oder Cyano; Phenyl-C₁-C₆-alklyl, welches am Phenylring gegebenenfalls einfach oder mehrfach, gleich oder verschieden substituiert ist durch Halogen, C₁-C₆-Alkyl, C₁-C₆-Haloalkyl, C₁-C₆-Alkoxy, Nitro oder Cyano; Heteroaryl, welches gegebenenfalls einfach oder mehrfach gleich oder verschieden substituiert ist durch - OH, -SH, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, Mono-C₁-C₆-alkylamino, Di-C₁-C₆-alkylamino, Nitro oder Cyano; Heteroaryl-C₁-C₆-alkyl, welches am Heteraromaten gegebenenfalls einfach oder mehrfach, gleich oder verschieden substituiert ist durch -OH, -SH, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, Mono-C₁-C₆-alkylamino, Di-C₁-C₆-alkylamino, Nitro oder Cyano; Heterocyclyl, welches gegebenenfalls einfach oder mehrfach, gleich oder verschieden substituiert ist durch - OH / =O, -SH / =S, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, Mono-C₁-C₆-alkylamino, Di-C₁-C₆-alkylamino, Nitro oder Cyano und Heterocyclyl-C₁-C₆-alkyl steht, welches am Heterocyclus gegebenenfalls einfach oder mehrfach, gleich oder verschieden substituiert ist durch -OH / =O, -SH / =S, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, Mono-C₁-C₆-alkylamino, Di-C₁-C₆-alkylamino, Nitro oder Cyano,
R⁴ für einen Rest aus der Reihe 2-Pyridyl, 3-Pyridyl und Pyrazolyl steht, welche jeweils gegebenenfalls einfach oder mehrfach, gleich oder verschieden substituiert sind durch Reste aus der Reihe Halogen, C₁-C₆-Alkyl, C₁-C₆-Haloalkyl, C₃-C₆-Cycloalkyl, C₃-C₆-Halocycloalkyl, C₃-C₆-Cycloalkyl-C₁-C₆-alkyl, C₁-C₆-Alkoxy, C₁-C₆-Haloalkoxy, C₁-C₆-Alkoxy-C₁-C₆-alkyl, C₁-C₆-Alkoxy-C₁-C₆-alkoxy, COOH, Nitro, Amino, Cyano, C₁-C₆-Alkylsulfanyl, C₁-C₆-Alkylsulfinyl, C₁-C₆-Alkylsulfonyl, C₁-C₆-Haloalkylsulfanyl, C₁-C₆-Haloalkylsulfinyl, C₁-C₆-Haloalkylsulfonyl, Mono-C₁-C₆-alkylamino, Di-C₁-C₆-alkylamino, C₁-C₆-Alkylaminocarbonyl, C₃-C₆-Cycloalkylaminocarbonyl, Phenyl (welches selbst wiederum substituiert sein kann durch Halogen), und Hetaryl-C₁-C₆-alkyl (insbesondere Pyrazolyl-C₁-C₆-alkyl (welches selbst wieder substituiert sein kann durch Halogen, C₁-C₆-Alkyl und C₁-C₆-Halogenalkyl),
R⁵ für einen Rest aus der Reihe Wasserstoff, Halogen, Cyano, C₁-C₆-Alkyl, C₁-C₆-Haloalkyl, C₃-C₈-Cycloalkyl, C₃-C₈-Haloeycloalkyl sowie Phenyl, 2-Pyridyl oder 3-Pyridyl steht, welche jeweils gegebenenfalls einfach oder mehrfach, gleich oder verschieden substituiert sind durch Reste aus der Reihe Halogen, Amino, Cyano, C₁-C₆-Alkyl, C₁-C₆-Haloalkyl, C₃-C₆-Cycloalkyl, C₃-C₆-Halocycloalkyl, C₁-C₆-Alkoxy, C₁-C₆-Haloalkoxy, C₂-C₆-Alkenyl, C₁-C₆-Alkoxy-C₁-C₆-alkyl, C₁-C₆-Alkoxy-C₁-C₆-alkoxy, Nitro, Cyano, C₁-C₆-Alkylcarbonyl, C₁-C₆-Alkylcarbonylamino, C₃-C₆-Cycloalkylcarbonylamino, C₁-C₆-Alkylaminocarbonyl, C₃-C₆-Cycloalkylaminocarbonyl C₁-C₆-Alkoxycarbonyl, Aminocarbonyl, C₁-C₆-Alkoxyimino-C₁-C₆-alkyl, C₁-C₆-Alkylsulfanyl, C₁-C₆-Alkylsulfinyl, C₁-C₆-Alkylsulfonyl, C₁-C₆-Alkylsulfonyloxy, C₁-C₆-Alkylsulfonylamino, C₁-C₆-Haloalkylsulfanyl, C₁-C₆-Haloalkylsulfinyl, C₁-C₆-Haloalkylsulfonyl, Tri-C₁-C₆-alkylsilyl, S(O)(=N-CN), S(O)(=N)R (worin R für C₁-C₆-Alkyl oder C₁-C₆-Haloalkyl steht), Phenyl, Phenyl-C₁-C₆-alkyl, Phenyl-C₁-C₆-alkyloxy, Phenyloxy, Pyrazolyl (wobei die Phenylgruppen bzw. der Pyrazolylring wiederum substituiert sein können durch Halogen, C₁-C₆-Alkyl, C₃-C₈-Cycloalkyl, C₁-C₆-Alkoxy, C₁-C₆-Haloalkoxy, Nitro, Cyano, C₁-C₆-Alkylsulfanyl, C₁-C₆-Alkylsulfinyl, C₁-C₆-Alkylsulfonyl, C₁-C₆-Alkylsulfonyloxy, C₁-C₆-Alkylsulfonylamino, C₁-C₆-Haloalkylsulfanyl, C₁-C₆-Haloalkylsulfinyl, C₁-C₆-Haloalkylsulfonyl, S(O)(=N-CN), S(O)(=N)R (worin R für C₁-C₆-Alkyl oder C₁-C₆-Haloalkyl steht)), Hetaryloxy-C₁-C₆-alkyl, Hetaryloxy und Hetaryl-C₁-C₆-alkyl, (wobei der Hetarylrest jeweils ausgewählt ist aus der Reihe Pyrrolyl, Imidazolyl, Pyrazolyl, Triazolyl, Pyridyl, Pyrimidinyl und Tetrazolyl und selbst wiederum substituiert sein kann durch Halogen, Cyano, C₁-C₆-Alkyl, C₁-C₆-Haloalkyl, C₃-C₈-Cycloalkyl, C₃-C₈-Halocycloalkyl) und wobei zwei benachbarte Substituenten auch gemeinsam für - OCH₂O-, -OCF₂O- oder -OCH₂CH₂O- stehen können,
R⁶ für einen Rest aus der Reihe Wasserstoff, Halogen, C₁-C₆-Alkyl, C₁-C₆-Haloalkyl, Cyano-C₁-C₆-alkyl, C₁-C₆-Alkoxy-C₁-C₆-alkyl, C₁-C₆-Alkylcarbonyl, C₁-C₆-Haloalkylcarbonyl, C₃-C₈-Cycloalkyl, C₃-C₈-Halocycloalkyl, C₃-C₈-Cycloalkyl-C₁-C₆-alkyl, C₁-C₆-Alkylsulfanyl, C₁-C₆-Alkylsulfonyl, C₂-C₆-Alkenyl, C₂-C₆-Alkinyl, C₁-C₆-Alkoxycarbonyl, C₂-C₆-Alkenyloxycarbonyl, C₂-C₆-Alkinyloxycarbonyl, C₁-C₆-Alkoxycarbonyl-C₁-C₆-alkyl; Phenyl, Phenyl-C₁-C₆-alkyl, welche jeweils am Phenylring gegebenenfalls einfach oder mehrfach, gleich oder verschieden substituiert sind durch Halogen, C₁-C₆-Alkyl, C₁-C₆-Haloalkyl, C₁-C₆-Alkoxy, Nitro oder Cyano; Heterocyclyl-C₁-C₆-alkyl, welches am Heterocyclus gegebenenfalls einfach oder mehrfach, gleich oder verschieden substituiert ist durch Halogen, C₁-C₆-Alkyl, C₁-C₆-Haloalkyl, C₁-C₆-Alkoxy, Nitro oder Cyano; Heteroaryl-C₁-C₆-alkyl, welches am Heteroaromaten gegebenenfalls einfach oder mehrfach, gleich oder verschieden substituiert ist durch Halogen, C₁-C₆-Alkyl, C₁-C₆-Haloalkyl, C₁-C₆-Alkoxy, Nitro oder Cyano; oder Phenylcarbonyl steht, welches am Phenylring gegebenenfalls einfach oder mehrfach gleich oder verschieden substituiert ist durch Halogen, C₁-C₆-Alkyl, C₁-C₆-Haloalkyl, C₁-C₆-Alkoxy, Nitro oder Cyano und
X für Sauerstoff oder Schwefel steht.

2. Verbindungen der Formel (I) gemäß Anpruch 1, worin
R¹ für einen Rest aus der Reihe C₁-C₆-Alkyl, C₂-C₆-Alkenyl, C₂-C₆-Alkinyl, C₁-C₆-Haloalkyl, C₃-C₈-Cycloalkyl, C₃-C₈-Halocycloalkyl, C₃-C₈-Cycloalkenyl, C₁-C₆-Alkoxy-C₁-C₆-alkyl, C₁-C₆-Alkylsulfanyl-C₁-C₆-alkyl, C₁-C₆-Alkylcarbonyl-C₁-C₆-alkyl, C₁-C₆-Alkoxycarbonyl-C₁-C₆-alkyl, C₃-C₆-Cycloalkyl-C₁-C₆-alkyl, halogeniertes C₃-C₆-Cycloalkyl-C₁-C₆-alkyl, Phenyl-C₁-C₆-alkyl, welches am Phenylring gegebenenfalls einfach oder mehrfach, gleich oder verschieden substituiert ist durch Halogen, C₁-C₆-Alkyl, C₁-C₆-Haloalkyl, C₁-C₆-Alkoxy, Nitro oder Cyano; Heteroaryl, welches gegebenenfalls einfach oder mehrfach, gleich oder verschieden substituiert ist durch -OH, -SH, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, Mono-C₁-C₆-alkylamino, Di-C₁-C₆-alkylamino, Nitro oder Cyano; Heteroaryl-C₁-C₆-alkyl, welches am Heteraromaten gegebenenfalls einfach oder mehrfach, gleich oder verschieden substituiert ist durch -OH, -SH, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, Mono-C₁-C₆-alkylamino, Di-C₁-C₆-alkylamino, Nitro oder Cyano; Heterocyclyl-C₁-C₆-alkyl steht, welches am Heterocyclus gegebenenfalls einfach oder mehrfach, gleich oder verschieden substituiert ist durch -OH / =O, -SH / =S, - NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, Mono-C₁-C₆-alkylamino, Di-C₁-C₆-alkylamino, Nitro oder Cyano,
R² für einen Rest aus der Reihe Wasserstoff, C₁-C₆-Alkyl, C₂-C₆-Alkenyl, C₂-C₆-Alkinyl, C₁-C₆-Haloalkyl, C₃-C₈-Cycloalkyl, C₃-C₈-Halocycloalkyl, C₃-C₈-Cycloalkenyl, C₁-C₆-Alkoxy-C₁-C₆-alkyl, C₁-C₆-Alkylcarbonyl-C₁-C₆-alkyl, C₁-C₆-Alkoxycarbonyl-C₁-C₆-alkyl, Phenyl-C₁-C₆-alkyl, welches am Phenylring gegebenenfalls einfach oder mehrfach, gleich oder verschieden substituiert ist durch Halogen, C₁-C₆-Alkyl, C₁-C₆-Haloalkyl, C₁-C₆-Alkoxy, Nitro oder Cyano; Heteroaryl, welches gegebenenfalls einfach oder mehrfach, gleich oder verschieden substituiert ist durch -OH, -SH, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, Mono-C₁-C₆-alkylamino, Di-C₁-C₆-alkylamino, Nitro oder Cyano; Heteroaryl-C₁-C₆-alkyl, welches am Heteraromaten gegebenenfalls einfach oder mehrfach, gleich oder verschieden substituiert ist durch -OH, -SH, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, Mono-C₁-C₆-alkylamino, Di-C₁-C₆-alkylamino, Nitro oder Cyano; Heteroeyelyl-C₁-C₆-alkyl steht, welches am Heterocyclus gegebenenfalls einfach oder mehrfach, gleich oder verschieden substituiert ist durch -OH / =O, -SH / =S, - NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, Mono-C₁-C₆-alkylamino, Di-C₁-C₆-alkylamino, Nitro oder Cyano,
R³ einen Rest aus der Reihe Wasserstoff, C₁-C₆-Alkyl, C₂-C₆-Alkenyl, C₂-C₆-Alkinyl, C₁-C₆-Haloalkyl, C₃-C₈-Cycloalkyl, C₃-C₈-Halocycloalkyl, C₃-C₈-Cycloalkenyl, C₁-C₆-Alkoxy-C₁-C₆-alkyl, C₁-C₆-Alkylcarbonyl-C₁-C₆-alkyl, C₁-C₆-Alkoxycarbonyl-C₁-C₆-alkyl, Phenyl-C₁-C₆-alkyl, welches am Phenylring gegebenenfalls einfach oder mehrfach, gleich oder verschieden substituiert ist durch Halogen, C₁-C₆-Alkyl, C₁-C₆-Haloalkyl, C₁-C₆-Alkoxy, Nitro oder Cyano; Heteroaryl, welches gegebenenfalls einfach oder mehrfach, gleich oder verschieden substituiert ist durch - OH, -SH, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, Mono-C₁-C₆-alkylamino, Di-C₁-C₆-alkylamino, Nitro oder Cyano; Heteroaryl-C₁-C₆-alkyl, welches am Heteraromaten gegebenenfalls einfach oder mehrfach, gleich oder verschieden substituiert ist durch -OH, -SH, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, Mono-C₁-C₆-alkylamino, Di-C₁-C₆-alkylamino, Nitro oder Cyano; Heterocyclyl-C₁-C₆-alkyl steht, welches am Heterocyclus gegebenenfalls einfach oder mehrfach, gleich oder verschieden substituiert ist durch -OH / =O, -SH / =S, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, Mono-C₁-C₆-alkylamino, Di-C₁-C₆-alkylamino, Nitro oder Cyano,
R⁴ für 3-Pyridyl oder Pyrazolyl, welche jeweils gegebenenfalls einfach oder mehrfach, gleich oder verschieden substituiert sind durch Reste aus der Reihe Halogen, C₁-C₆-Alkyl, C₁-C₆-Haloalkyl, C₃-C₆-Cycloalkyl, C₃-C₆-Halocycloalkyl, C₃-C₆-Cycloalkyl-C₁-C₆-alkyl, C₁-C₆-Alkoxy, C₁-C₆-Alkoxy-C₁-C₆-alkyl, C₁-C₆-Alkoxy-C₁-C₆-alkoxy, COOH, Nitro, Amino, Mono-C₁-C₆-alkylamino, Di-C₁-C₆-alkylamino, C₁-C₆-Alkylaminocarbonyl und C₃-C₆-Cycloalkylaminocarbonyl,
R⁴ weiterhin für Pyrazolyl steht, welches gegebenenfalls substituiert ist durch Halogen oder C₁-C₆-Alkyl, Phenyl (welches selbst wiederum substituiert sein kann durch Halogen) oder durch Pyrazolylmethyl (welches selbst wieder substituiert sein kann durch C₁-C₆-Halogenalkyl),
R⁵ für Phenyl, 2-Pyridyl oder 3-Pyridyl steht, welche jeweils gegebenenfalls einfach oder mehrfach, gleich oder verschieden substituiert sind durch Reste aus der Reihe Halogen, C₁-C₆-Alkyl, C₁-C₆-Haloalkyl, C₃-C₆-Cycloalkyl, C₃-C₆-Halocycloalkyl, C₁-C₆-Alkoxy, C₁-C₆-Haloalkoxy, C₁-C₆-Alkoxy-C₁-C₆-alkyl, C₁-C₆-Alkoxy-C₁-C₆-alkoxy, Nitro, Amino, Cyano, Mono-C₁-C₆-alkylamino, Di-C₁-C₆-alkylamino, C₁-C₆-Alkylsulfanyl, C₁-C₆-Alkylsulfinyl, C₁-C₆-Alkylsulfonyl, C₁-C₆-Haloalkylsulfanyl, C₁-C₆-Haloalkylsulfinyl, C₁-C₆-Haloalkylsulfonyl, Hetaryloxy-C₁-C₆-alkyl, Hetaryloxy und Hetaryl-C₁-C₆-alkyl wobei der Hetarylrest ausgewählt ist aus der Reihe Pyrrolyl, Imidazolyl, Pyrazolyl, Triazolyl, Pyridyl, Pyrimidinyl und Tetrazolyl und selbst wiederum substituiert sein kann durch Substituenten aus der Reihe Halogen, Cyano, C₁-C₆-Alkyl, C₁-C₆-Haloalkyl, C₃-C₈-Cycloalkyl und C₃-C₈-Halocycloalkyl,
R⁵ auch für Wasserstoff, Halogen, C₁-C₄-Alkyl und C₃-C₆-Cycloalkyl steht,
R⁶ für einen Rest aus der Reihe Wasserstoff, Halogen, C₁-C₆-Alkyl, C₁-C₆-Haloalkyl, C₁-C₆-Alkoxy-C₁-C₆-alkyl, C₁-C₆-Alkylearbonyl, C₁-C₆-Haloalkylcarbonyl, C₃-C₆-Cycloalkyl, C₃-C₆-Cycloalkyl-C₁-C₆-alkyl, C₁-C₆-Alkylsulfanyl, C₂-C₆-Alkenyl, C₂-C₆-Alkinyl, C₁-C₆-Alkoxycarbonyl, C₂-C₆-Alkenyloxycarbonyl, C₂-C₆-Alkinyloxycarbonyl, C₁-C₆-Alkoxycarbonyl-C₁-C₆-alkyl, Phenyl, Phenyl-C₁-C₆-alkyl, welche jeweils am Phenylring gegebenenfalls einfach oder mehrfach, gleich oder verschieden substituiert sind durch Halogen, C₁-C₆-Alkyl, C₁-C₆-Haloalkyl, C₁-C₆-Alkoxy, Nitro oder Cyano; Heterocyclyl-C₁-C₆-alkyl, welches am Heterocyclus gegebenenfalls einfach oder mehrfach, gleich oder verschieden substituiert sein kann durch Halogen, C₁-C₆-Alkyl, C₁-C₆-Haloalkyl, C₁-C₆-Alkoxy, Nitro oder Cyano; und Phenylcarbonyl steht, welches am Phenylring gegebenenfalls einfach oder mehrfach, gleich oder verschieden substituiert ist durch Halogen, C₁-C₆-Alkyl, C₁-C₆-Haloalkyl, C₁-C₆-Alkoxy, Nitro oder Cyano und
X für Sauerstoff oder Schwefel steht.

3. Verbindungen der Formel (I) gemäß Anspruch 1, worin
R¹ für einen Rest aus der Reihe C₁-C₆-Alkyl, C₁-C₆-Haloalkyl, C₂-C₆-Alkenyl, C₂-C₆-Alkinyl, C₃-C₆-Cycloalkyl, C₃-C₆-Halocycloalkyl, C₁-C₆-Alkoxy-C₁-C₆-alkyl, C₁-C₆-Alkylsulfanyl-C₁-C₆-alkyl, C₁-C₆-Alkoxycarbonyl-C₁-C₆-alkyl und C₃-C₆-Cycloalkyl-C₁-C₆-alkyl und halogeniertes C₃-C₆-Cycloalkyl-C₁-C₆-alkyl steht,
R² für Wasserstoff steht,
R³ für Wasserstoff steht,
R⁴ für 3-Pyridyl steht, welches einfach oder mehrfach substituiert ist durch Reste aus der Reihe Halogen, C₁-C₆-Alkyl, C₁-C₆-Haloalkyl, C₃-C₆-Cycloalkyl, C₃-C₆-Halocycloalkyl, C₃-C₆-Cycloalkyl-C₁-C₆-alkyl, C₁-C₆-Alkoxy, C₁-C₆-Alkylaminocarbonyl und C₃-C₆-Cycloalkylaminocarbonyl,
R⁴ weiterhin für Pyrazolyl steht, welches substituiert sein kann durch Halogen (bevorzugt F, Cl, Br) und C₁-C₆-Alkyl (bevorzugt Methyl, Ethyl und Propyl), Phenyl (welches selbst wiederum substituiert sein kann durch Halogen) oder durch Pyrazolylmethyl (welches selbst wieder substituiert sein kann durch C₁-C₆-Halogenalkyl, insbesondere fluoriertes C₁-C₆-Alkyl),
R⁵ für einen Rest aus der Reihe Phenyl, 2-Pyridyl und 3-Pyridyl steht, welcher jeweils einfach oder mehrfach, substituiert ist durch mindestens einen Rest aus der Reihe Halogen, C₁-C₆-Alkyl, C₁-C₆-Haloalkyl, C₁-C₆-Alkoxy, C₁-C₆-Haloalkoxy, Nitro, Cyano, C₁-C₆-Alkylsulfanyl, C₁-C₆-Alkylsulfinyl, C₁-C₆-Alkylsulfonyl, C₁-C₆-Haloalkylsulfanyl, C₁-C₆-Haloalkylsulfinyl, C₁-C₆-Haloalkylsulfonyl, Hetaryloxy-C₁-C₆-alkyl, Hetaryloxy und Hetaryl-C₁-C₆-alkyl, wobei der Hetarylrest ausgewählt ist aus der Reihe Pyrrolyl, Imidazolyl, Pyrazolyl, Triazolyl, Pyridyl, Pyrimidinyl und Tetrazolyl und selbst wiederum substituiert sein kann durch Substituenten aus der Reihe Halogen, Cyano, C₁-C₆-Alkyl, C₁-C₆-Haloalkyl, C₃-C₈-Cycloalkyl und C₃-C₈-Halocycloalkyl,
R⁵ auch für einen Rest aus der Reihe Wasserstoff, Halogen, C₁-C₄-Alkyl und C₃-C₆-Cycloalkyl steht,
R⁶ für einen Rest aus der Reihe Wasserstoff, Halogen, C₁-C₆-Alkyl, C₁-C₆-Haloalkyl, C₃-C₆-Cycloalkyl, C₁-C₆-Alkylsulfanyl und Phenyl steht, welches durch Halogen substituiert sein kann und
X für Sauerstoff steht.

4. Verbindungen der Formel (I) gemäß Anspruch 1, worin
R¹ für einen Rest aus der Reihe CH₃, CH₂CH₃, CH(CH₃)₂, Cyclopropyl, Halocyclopropyl, C(CH₃)₃, CH(CH₃)CH₂SCH₃, CH₂CH₂CH₃, CH(CH₃)CH₂CH₃, CH₂CF₃, CHCH₃CF₃, CH(CH₃)Cyclopropyl steht,
R² für Wasserstoff steht,
R³ für Wasserstoff steht,
R⁴ für 3-Pyridyl steht, welches in der 2-Position substituiert ist durch einen Substituenten aus der Reihe F, Cl, Br, Methyl, Ethyl, CF₃, CH₂CF₃, CHFCH₃, CHFCF₃, Cyclopropyl und Cyclopropylmethyl,
R⁴ weiterhin für Pyrazolyl steht, welches substituiert sein kann durch F, Cl, Methyl, Ethyl oder durch Pyrazolylmethyl, welches selbst wiederum substituiert sein kann durch C₁-C₆-Halogenalkyl,
R⁵ für Phenyl steht, welches einfach oder mehrfach substituiert ist durch einen Rest aus der Reihe CH₃, F, Cl, CF₃, CF(CF₃)₂, OCH₃, OCF₃> NO₂, CN, SCF₃, S(O)CF₃ und S(O)₂CF₃ und CH₂-Q,
Q für einen Rest aus der Reihe 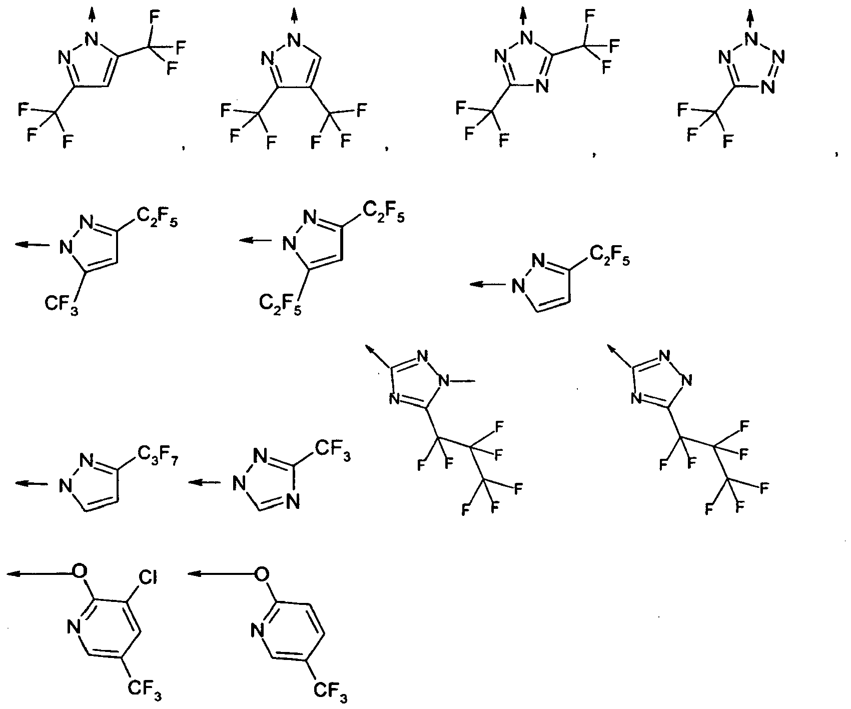 steht, wobei der Pfeil die Stelle der Verknüpfung mit der CH₂-Gruppe angibt,
R⁶ für Wasserstoff steht und.
X für Sauerstoff steht.

5. Mittel, **gekennzeichnet durch** einen Gehalt von mindestens einer Verbindung der Formel (I) gemäß Anspruch 1 und üblichen Streckmitteln und/oder oberflächenaktiven Substanzen.

6. Verfahren zum Bekämpfen von Pflanzen-, Hygiene- und Vorratsschädlingen, **dadurch gekennzeichnet, dass** man eine Verbindung der Formel (I) gemäß Anspruch 1 oder ein Mittel gemäß Anspruch 5 auf die Schädlinge und/oder ihren Lebensraum einwirken lässt.

7. Verwendung von Verbindungen der Formel (I) gemäß Anspruch 1 oder von Mitteln gemäß Anspruch 5 zum Bekämpfen von Pflanzen-, Hygiene- und Vorratsschädlingen.

8. Pharmazeutische Zusammensetzungen, enthaltend wenigstens eine Verbindung gemäß Anspruch 1.

9. Verwendung wenigstens einer Verbindung gemäß Anspruch 1 zur Herstellung pharmazeutischer Zusammensetzungen zur Bekämpfung von Parasiten auf Tieren.

## Claims

1. Compounds of the formula (I) 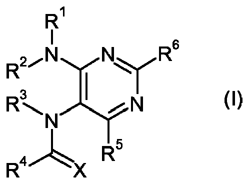 in which
R¹ represents a radical from the group consisting of C₁-C₆-alkyl, C₂-C₆-alkenyl, C₂-C₆-alkynyl, C₁-C₆-haloalkyl, cyano-C₁-C₆-alkyl, C₃-C₈-cycloalkyl, C₃-C₈-halocycloalkyl, C₃-C₈-cycloalkenyl, C₃-C₈-halocycloalkenyl, C₁-C₆-alkoxy-C₁-C₆-alkyl, C₁-C₆-haloalkoxy-C₁-C₆-alkyl, C₁-C₆-alkoxy-C₁-C₆-haloalkyl, C₁-C₆-alkylcarbonyl-C₁-C₆-alkyl, C₁-C₆-alkoxycarbonyl-C₁-C₆-alkyl, C₁-C₆-alkylsulphanyl-C₁-C₆-alkyl, C₁-C₆-alkylsulphinyl-C₁-C₆-alkyl, C₁-C₆-alkylsulphonyl-C₁-C₆-alkyl, C₁-C₆-haloalkylsulphanyl-C₁-C₆-alkyl, C₁-C₆-haloalkylsulphinyl-C₁-C₆-alkyl, C₁-C₆-haloalkylsulphonyl-C₁-C₆-alkyl, C₃-C₈-cycloalkyl-C₁-C₆-alkyl, halogenated C₃-C₈-cycloalkyl-C₁-C₆-alkyl, phenyl-C₁-C₆-alkyl which is optionally mono- or polysubstituted at the phenyl ring by identical or different substituents from the group consisting of halogen, C₁-C₆-alkyl, C₁-C₆-haloalkyl, C₁-C₆-alkoxy, C₁-C₆-haloalkoxy, C₁-C₆-alkylsulphanyl, C₁-C₆-alkylsulphinyl, C₁-C₆-alkylsulphonyl, C₁-C₆-haloalkylsulphanyl, C₁-C₆-haloalkylsulphinyl, C₁-C₆-haloalkylsulphonyl, nitro or cyano; heteroaryl which is optionally mono- or polysubstituted by identical or different substituents from the group consisting of -OH, - SH, -NH₂, halogen, C₁-C₆-alkyl, C₁-C₆-haloalkyl, C₁-C₆-alkoxy, C₁-C₆-haloalkoxy, C₁-C₆-alkylsulphanyl, C₁-C₆-alkylsulphinyl, C₁-C₆-alkylsulphonyl, C₁-C₆-haloalkylsulphanyl, C₁-C₆-haloalkylsulphinyl, C₁-C₆-haloalkylsulphonyl, mono-C₁-C₆-alkylamino, di-C₁-C₆-alkylamino, nitro or cyano; heteroaryl-C₁-C₆-alkyl which is optionally mono- or polysubstituted at the heteroaromatic moiety by identical or different substituents from the group consisting of -OH, -SH, -NH₂, halogen, C₁-C₆-alkyl, C₁-C₆-haloalkyl, C₁-C₆-alkoxy, C₁-C₆-haloalkoxy, C₁-C₆-alkylsulphanyl, C₁-C₆-alkylsulphinyl, C₁-C₆-alkylsulphonyl, C₁-C₆-haloalkylsulphanyl, C₁-C₆-haloalkylsulphinyl, C₁-C₆-haloalkylsulphonyl, mono-C₁-C₆-alkylamino, di-C₁-C₆-alkylamino, nitro or cyano; heterocyclyl which is optionally mono-or polysubstituted by identical or different substituents from the group consisting of -OH / =0, -SH / =S, -NH₂, halogen, C₁-C₆-alkyl, C₁-C₆-haloalkyl, C₁-C₆-alkoxy, C₁-C₆-haloalkoxy, C₁-C₆-alkylsulphanyl, C₁-C₆-alkylsulphinyl, C₁-C₆-alkylsulphonyl, C₁-C₆-haloalkylsulphanyl, C₁-C₆-haloalkylsulphinyl, C₁-C₆-haloalkylsulphonyl, mono-C₁-C₆-alkylamino, di-C₁-C₆-alkylamino, nitro or cyano and heterocyclyl-C₁-C₆-alkyl which is optionally mono- or polysubstituted at the heterocycle by identical or different substituents from the group consisting of -OH / =0, -SH / =S, - NH₂, halogen, C₁-C₆-alkyl, C₁-C₆-haloalkyl, C₁-C₆-alkoxy, C₁-C₆-haloalkoxy, C₁-C₆-alkylsulphanyl, C₁-C₆-alkylsulphinyl, C₁-C₆-alkylsulphonyl, C₁-C₆-haloalkylsulphanyl, C₁-C₆-haloalkylsulphinyl, C₁-C₆-haloalkylsulphonyl, mono-C₁-C₆-alkylamino, di-C₁-C₆-alkylamino, nitro or cyano,
R² represents a radical from the group consisting of hydrogen, C₁-C₆-alkyl, C₂-C₆-alkenyl, C₂-C₆-alkynyl, C₁-C₆-haloalkyl, cyano-C₁-C₆-alkyl, C₃-C₈-cycloalkyl, C₃-C₈-halocycloalkyl, C₃-C₈-cycloalkenyl, C₃-C₈-halocycloalkenyl, C₁-C₆-alkoxy-C₁-C₆-alkyl, C₁-C₆-haloalkoxy-C₁-C₆-alkyl, C₁-C₆-alkylcarbonyl-C₁-C₆-alkyl, C₁-C₆-alkoxycarbonyl-C₁-C₆-alkyl, C₁-C₆-alkylsulphanyl-C₁-C₆-alkyl, C₁-C₆-alkylsulphinyl-C₁-C₆-alkyl, C₁-C₆-alkylsulphonyl-C₁-C₆-alkyl, C₁-C₆-haloalkylsulphanyl-C₁-C₆-alkyl, C₁-C₆-haloalkylsulphinyl-C₁-C₆-alkyl, C₁-C₆-haloalkylsulphonyl-C₁-C₆-alkyl, C₃-C₈-cycloalkyl-C₁-C₆-alkyl, phenyl-C₁-C₆-alkyl which is optionally mono- or polysubstituted at the phenyl ring by identical or different substituents from the group consisting of halogen, C₁-C₆-alkyl, C₁-C₆-haloalkyl, C₁-C₆-alkoxy, C₁-C₆-haloalkoxy, C₁-C₆-alkylsulphanyl, C₁-C₆-alkylsulphinyl, C₁-C₆-alkylsulphonyl, C₁-C₆-haloalkylsulphanyl, C₁-C₆-haloalkylsulphinyl, C₁-C₆-haloalkylsulphonyl, nitro or cyano; heteroaryl which is optionally mono- or polysubstituted by identical or different substituents from the group consisting of -OH,-SH, -NH₂, halogen, C₁-C₆-alkyl, C₁-C₆-haloalkyl, C₁-C₆-alkoxy, C₁-C₆-haloalkoxy, C₁-C₆-alkylsulphanyl, C₁-C₆-alkylsulphinyl, C₁-C₆-alkylsulphonyl, C₁-C₆-haloalkylsulphanyl, C₁-C₆-haloalkylsulphinyl, C₁-C₆-haloalkylsulphonyl, mono-C₁-C₆-alkylamino, di-C₁-C₆-alkylamino, nitro or cyano; heteroaryl-C₁-C₆-alkyl which is optionally mono- or polysubstituted at the heteroaromatic moiety by identical or different substituents from the group consisting of -OH, -SH, -NH₂, halogen, C₁-C₆-alkyl, C₁-C₆-haloalkyl, C₁-C₆-alkoxy, C₁-C₆-haloalkoxy, C₁-C₆-alkylsulphanyl, C₁-C₆-alkylsulphinyl, C₁-C₆-alkylsulphonyl, C₁-C₆-haloalkylsulphanyl, C₁-C₆-haloalkylsulphinyl, C₁-C₆-haloalkylsulphonyl, mono-C₁-C₆-alkylamino, di-C₁-C₆-alkylamino, nitro or cyano; heterocyclyl which is optionally mono- or polysubstituted by identical or different substituents from the group consisting of -OH / =0, -SH / =S, -NH₂, halogen, C₁-C₆-alkyl, C₁-C₆-haloalkyl, C₁-C₆-alkoxy, C₁-C₆-haloalkoxy, C₁-C₆-alkylsulphanyl, C₁-C₆-alkylsulphinyl, C₁-C₆-alkylsulphonyl, C₁-C₆-haloalkylsulphanyl, C₁-C₆-haloalkylsulphinyl, C₁-C₆-haloalkylsulphonyl, mono-C₁-C₆-alkylamino, di-C₁-C₆-alkylamino, nitro or cyano and optionally mono- or polysubstituted at the heterocycle by identical or different substituents from the group consisting of -OH / =0, -SH / =S,-NH₂, halogen, C₁-C₆-alkyl, C₁-C₆-haloalkyl, ₁-C₆-alkoxy, C₁-C₆-haloalkoxy, C₁-C₆-alkylsulphanyl, C₁-C₆-alkylsulphinyl, C₁-C₆-alkylsulphonyl, C₁-C₆-haloalkylsulphanyl, C₁-C₆-haloalkylsulphinyl, C₁-C₆-haloalkylsulphonyl, mono-C₁-C₆-alkylamino, di-C₁-C₆-alkylamino, nitro or cyano,
R³ represents a radical from the group consisting of hydrogen, C₁-C₆-alkyl, C₂-C₆-alkenyl, C₂-C₆-alkynyl, halo-C₁-C₆-alkyl, cyano-C₁-C₆-alkyl, C₃-C₈-cycloalkyl, halo-C₃-C₈-cycloalkyl, C₃-C₈-cycloalkenyl, halo-C₃-C₈-cycloalkenyl, C₁-C₆-alkoxy-C₁-C₆-alkyl, C₁-C₆-alkylcarbonyl-C₁-C₆-alkyl, C₁-C₆-alkoxycarbonyl-C₁-C₆-alkyl, C₁-C₆-alkylsulphanyl-C₁-C₆-alkyl, C₁-C₆-alkylsulphinyl-C₁-C₆-alkyl, C₁-C₆-alkylsulphonyl-C₁-C₆-alkyl, C₃-C₈-cycloalkyl-C₁-C₆-alkyl, phenyl which is optionally mono- or polysubstituted by identical or different substituents from the group consisting of -OH, -SH, -NH₂, halogen, C₁-C₆-alkyl, C₁-C₆-haloalkyl, C₁-C₆-alkoxy, C₁-C₆-alkylsulphanyl, C₁-C₆-alkylsulphinyl, C₁-C₆-alkylsulphonyl, mono-C₁-C₆-alkylamino, di-C₁-C₆-alkylamino, nitro or cyano; phenyl-C₁-C₆-alkyl which is optionally mono- or polysubstituted at the phenyl ring by identical or different substituents from the group consisting of halogen, C₁-C₆-alkyl, C₁-C₆-haloalkyl, C₁-C₆-alkoxy, nitro or cyano; heteroaryl which is optionally mono- or polysubstituted by identical or different substituents from the group consisting of -OH,-SH, -NH₂, halogen, C₁-C₆-alkyl, C₁-C₆-haloalkyl, C₁-C₆-alkoxy, C₁-C₆-alkylsulphanyl, C₁-C₆-alkylsulphinyl, C₁-C₆-alkylsulphonyl, mono-C₁-C₆-alkylamino, di-C₁-C₆-alkylamino, nitro or cyano; heteroaryl-C₁-C₆-alkyl which is optionally mono- or polysubstituted at the heteroaromatic moiety by identical or different substituents from the group consisting of -OH, -SH, -NH₂, halogen, C₁-C₆-alkyl, C₁-C₆-haloalkyl, C₁-C₆-alkoxy, C₁-C₆-alkylsulphanyl, C₁-C₆-alkylsulphinyl, C₁-C₆-alkylsulphonyl, mono-C₁-C₆-alkylamino, di-C₁-C₆-alkylamino, nitro or cyano; heterocyclyl which is optionally mono- or polysubstituted by identical or different substituents from the group consisting of -OH / =0, -SH / =S, -NH₂, halogen, C₁-C₆-alkyl, C₁-C₆-haloalkyl, C₁-C₆-alkoxy, C₁-C₆-alkylsulphanyl, C₁-C₆-alkylsulphinyl, C₁-C₆-alkylsulphonyl, mono-C₁-C₆-alkylamino, di-C₁-C₆-alkylamino, nitro or cyano and heterocyclyl-C₁-C₆-alkyl which is optionally mono- or polysubstituted at the heterocycle by identical or different substituents from the group consisting of -OH / =0, -SH / =S, -NH₂, halogen, C₁-C₆-alkyl, C₁-C₆-haloalkyl, C₁-C₆-alkoxy, C₁-C₆-alkylsulphanyl, C₁-C₆-alkylsulphinyl, C₁-C₆-alkylsulphonyl, mono-C₁-C₆-alkylamino, di-C₁-C₆-alkylamino, nitro or cyano,
R⁴ represents a radical from the group consisting of 2-pyridyl, 3-pyridyl and pyrazolyl which are in each case optionally mono- or polysubstituted by identical or different radicals from the group consisting of halogen, C₁-C₆-alkyl, C₁-C₆-haloalkyl, C₃-C₆-cycloalkyl, C₃-C₆-halocycloalkyl, C₃-C₆-cycloalkyl-C₁-C₆-alkyl, C₁-C₆-alkoxy, C₁-C₆-haloalkoxy, C₁-C₆-alkoxy-C₁-C₆-alkyl, C₁-C₆-alkoxy-C₁-C₆-alkoxy, COOH, nitro, amino, cyano, C₁-C₆-alkylsulphanyl, C₁-C₆-alkylsulphinyl, C₁-C₆-alkylsulphonyl, C₁-C₆-haloalkylsulphanyl, C₁-C₆-haloalkylsulphinyl, C₁-C₆-haloalkylsulphonyl, mono-C₁-C₆-alkylamino, di-C₁-C₆-alkylamino, C₁-C₆-alkylaminocarbonyl, C₃-C₆-cycloalkylaminocarbonyl, phenyl (which for its part may be substituted by halogen), and hetaryl-C₁-C₆-alkyl (in particular pyrazolyl-C₁-C₆-alkyl (which for its part may be substituted by halogen, C₁-C₆-alkyl and C₁-C₆-haloalkyl),
R⁵ represents a radical from the group consisting of hydrogen, halogen, cyano, C₁-C₆-alkyl, C₁-C₆-haloalkyl, C₃-C₈-cycloalkyl, C₃-C₈-halocycloalkyl and also phenyl, 2-pyridyl or 3-pyridyl which are in each case optionally mono- or polysubstituted by identical or different radicals from the group consisting of halogen, amino, cyano, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₂-C₆-alkenyl, C₁-C₆-alkoxy-C₁-C₆-alkyl, C₁-C₆-alkoxy-C₁-C₆-alkoxy, nitro, cyano, C₁-C₆-alkylcarbonyl, C₁-C₆-alkylcarbonylamino, C₃-C₆-cycloalkylcarbonylamino, C₁-C₆-alkylaminocarbonyl, C₃-C₆-cycloalkylaminocarbonyl C₁-C₆-alkoxycarbonyl, aminocarbonyl, C₁-C₆-alkoxyimino-C₁-C₆-alkyl, C₁-C₆-alkylsulphanyl, C₁-C₆-alkylsulphinyl, C₁-C₆-alkylsulphonyl, C₁-C₆-alkylsulphonyloxy, C₁-C₆-alkylsulphonylamino, C₁-C₆-haloalkylsulphanyl, C₁-C₆-haloalkylsulphinyl, C₁-C₆-haloalkylsulphonyl, tri-C₁-C₆-alkylsilyl, S(O)(=N-CN), S(O) (=N) R (in which R represents C₁-C₆-alkyl or C₁-C₆-haloalkyl), phenyl, phenyl-C₁-C₆-alkyl, phenyl-C₁-C₆-alkyloxy, phenyloxy, pyrazolyl (where the phenyl groups or the pyrazolyl ring for their part may be substituted by halogen, C₁-C₆-alkyl, C₃-C₈-cycloalkyl, C₁-C₆-alkoxy, C₁-C₆-haloalkoxy, nitro, cyano, C₁-C₆-alkylsulphanyl, C₁-C₆-alkylsulphinyl, C₁-C₆-alkylsulphonyl, C₁-C₆-alkylsulphonyloxy, C₁-C₆-alkylsulphonylamino, C₁-C₆-haloalkylsulphanyl, C₁-C₆-haloalkylsulphinyl, C₁-C₆-haloalkylsulphonyl, S(O) (=N-CN), S(O) (=N) R (in which R represents C₁-C₆-alkyl or C₁-C₆-haloalkyl)), hetaryloxy-C₁-C₆-alkyl, hetaryloxy and hetaryl-C₁-C₆-alkyl, (where the hetaryl radical is in each case selected from the group consisting of pyrrolyl, imidazolyl, pyrazolyl, triazolyl, pyridyl, pyrimidinyl and tetrazolyl and may for its part be substituted by halogen, cyano, C₁-C₆-alkyl, C₁-C₆-haloalkyl, C₃-C₈-cycloalkyl, C₃-C₈-halocycloalkyl) and where two adjacent substituents together may also represent -OCH₂O-, -OCF₂O- or -OCH₂CH₂O-,
R⁶ represents a radical from the group consisting of hydrogen, halogen, C₁-C₆-alkyl, C₁-C₆-haloalkyl, cyano-C₁-C₆-alkyl, C₁-C₆-alkoxy-C₁-C₆-alkyl, C₁-C₆-alkylcarbonyl, C₁-C₆-haloalkylcarbonyl, C₃-C₈-cycloalkyl, C₃-C₈-halocycloalkyl, C₃-C₈-cycloalkyl-C₁-C₆-alkyl, C₁-C₆-alkylsulphanyl, C₁-C₆-alkylsulphonyl, C₂-C₆-alkenyl, C₂-C₆-alkynyl, C₁-C₆-alkoxycarbonyl, C₂-C₆-alkenyloxycarbonyl, C₂-C₆-alkynyloxycarbonyl, C₁-C₆-alkoxycarbonyl-C₁-C₆-alkyl; phenyl, phenyl-C₁-C₆-alkyl which are in each case optionally mono- or polysubstituted at the phenyl ring by identical or different substituents from the group consisting of halogen, C₁-C₆-alkyl, C₁-C₆-haloalkyl, C₁-C₆-alkoxy, nitro or cyano; heterocyclyl-C₁-C₆-alkyl which is optionally mono- or polysubstituted at the heterocycle by identical or different substituents from the group consisting of halogen, C₁-C₆-alkyl, C₁-C₆-haloalkyl, C₁-C₆-alkoxy, nitro or cyano; heteroaryl-C₁-C₆-alkyl which is optionally mono- or polysubstituted at the heteroaromatic moiety by identical or different substituents from the group consisting of halogen, C₁-C₆-alkyl, C₁-C₆-haloalkyl, C₁-C₆-alkoxy, nitro or cyano; or phenylcarbonyl which is optionally mono- or polysubstituted at the phenyl ring by identical or different substituents from the group consisting of halogen, C₁-C₆-alkyl, C₁-C₆-haloalkyl, C₁-C₆-alkoxy, nitro or cyano and
X represents oxygen or sulphur.

2. Compounds of the formula (I) according to Claim 1 in which
R¹ represents a radical from the group consisting of C₁-C₆-alkyl, C₂-C₆-alkenyl, C₂-C₆-alkynyl, C₁-C₆-haloalkyl, C₃-C₈-cycloalkyl, C₃-C₈-halocycloalkyl, C₃-C₈-cycloalkenyl, C₁-C₆-alkoxy-C₁-C₆-alkyl, C₁-C₆-alkylsulphanyl-C₁-C₆-alkyl, C₁-C₆-alkylcarbonyl-C₁-C₆-alkyl, C₁-C₆-alkoxycarbonyl-Ci-C6-alkyl, C₃-C₆-cycloalkyl-C₁-C₆-alkyl, halogenated C₃-C₆-cycloalkyl-C₁-C₆-alkyl, phenyl-C₁-C₆-alkyl which is optionally mono- or polysubstituted at the phenyl ring by identical or different substituents from the group consisting of halogen, C₁-C₆-alkyl, C₁-C₆-haloalkyl, C₁-C₆-alkoxy, nitro or cyano; heteroaryl which is optionally mono- or polysubstituted by identical or different substituents from the group consisting of -OH,-SH, -NH₂, halogen, C₁-C₆-alkyl, C₁-C₆-haloalkyl, C₁-C₆-alkoxy, C₁-C₆-alkylsulphanyl, C₁-C₆-alkylsulphinyl, C₁-C₆-alkylsulphonyl, mono-C₁-C₆-alkylamino, di-C₁-C₆-alkylamino, nitro or cyano; heteroaryl-C₁-C₆-alkyl which is optionally mono- or polysubstituted at the heteroaromatic moiety by identical or different substituents from the group consisting of -OH, -SH, -NH₂, halogen, C₁-C₆-alkyl, C₁-C₆-haloalkyl, C₁-C₆-alkoxy, C₁-C₆-alkylsulphanyl, C₁-C₆-alkylsulphinyl, C₁-C₆-alkylsulphonyl, mono-C₁-C₆-alkylamino, di-C₁-C₆-alkylamino, nitro or cyano; heterocyclyl-C₁-C₆-alkyl which is optionally mono- or polysubstituted at the heterocycle by identical or different substituents from the group consisting of -OH / =0, -SH / =S,-NH₂, halogen, C₁-C₆-alkyl, C₁-C₆-haloalkyl, C₁-C₆-alkoxy, C₁-C₆-alkylsulphanyl, C₁-C₆-alkylsulphinyl, C₁-C₆-alkylsulphonyl, mono-C₁-C₆-alkylamino, di-C₁-C₆-alkylamino, nitro or cyano,
R² represents a radical from the group consisting of hydrogen, C₁-C₆-alkyl, C₂-C₆-alkenyl, C₂-C₆-alkynyl, C₁-C₆-haloalkyl, C₃-C₈-cycloalkyl, C₃-C₈-halocycloalkyl, C₃-C₈-cycloalkenyl, C₁-C₆-alkoxy-C₁-C₆-alkyl, C₁-C₆-alkylcarbonyl-C₁-C₆-alkyl, C₁-C₆-alkoxycarbonyl-C₁-C₆-alkyl, phenyl-C₁-C₆-alkyl which is optionally mono- or polysubstituted at the phenyl ring by identical or different substituents from the group consisting of halogen, C₁-C₆-alkyl, C₁-C₆-haloalkyl, C₁-C₆-alkoxy, nitro or cyano; heteroaryl which is optionally mono- or polysubstituted by identical or different substituents from the group consisting of -OH,-SH, -NH₂, halogen, C₁-C₆-alkyl, C₁-C₆-haloalkyl, C₁-C₆-alkoxy, C₁-C₆-alkylsulphanyl, C₁-C₆-alkylsulphinyl, C₁-C₆-alkylsulphonyl, mono-C₁-C₆-alkylamino, di-C₁-C₆-alkylamino, nitro or cyano; heteroaryl-C₁-C₆-alkyl which is optionally mono- or polysubstituted at the heteroaromatic moiety by identical or different substituents from the group consisting of -OH, -SH, -NH₂, halogen, C₁-C₆-alkyl, C₁-C₆-haloalkyl, C₁-C₆-alkoxy, C₁-C₆-alkylsulphanyl, C₁-C₆-alkylsulphinyl, C₁-C₆-alkylsulphonyl, mono-C₁-C₆-alkylamino, di-C₁-C₆-alkylamino, nitro or cyano; heterocyclyl-C₁-C₆-alkyl which is optionally mono- or polysubstituted at the heterocycle by identical or different substituents from the group consisting of -OH / =0, -SH / =S,-NH₂, halogen, C₁-C₆-alkyl, C₁-C₆-haloalkyl, C₁-C₆-alkoxy, C₁-C₆-alkylsulphanyl, C₁-C₆-alkylsulphinyl, C₁-C₆-alkylsulphonyl, mono-C₁-C₆-alkylamino, di-C₁-C₆-alkylamino, nitro or cyano,
R³ represents a radical from the group consisting of hydrogen, C₁-C₆-alkyl, C₂-C₆-alkenyl, C₂-C₆-alkynyl, C₁-C₆-haloalkyl, C₃-C₈-cycloalkyl, C₃-C₈-halocycloalkyl, C₃-C₈-cycloalkenyl, C₁-C₆-alkoxy-C₁-C₆-alkyl, C₁-C₆-alkylcarbonyl-C₁-C₆-alkyl, C₁-C₆-alkoxycarbonyl-C₁-C₆-alkyl, phenyl-C₁-C₆-alkyl which is optionally mono- or polysubstituted at the phenyl ring by identical or different substituents from the group consisting of halogen, C₁-C₆-alkyl, C₁-C₆-haloalkyl, C₁-C₆-alkoxy, nitro or cyano; heteroaryl which is optionally mono- or polysubstituted by identical or different substituents from the group consisting of -OH,-SH, -NH₂, halogen, C₁-C₆-alkyl, C₁-C₆-haloalkyl, C₁-C₆-alkoxy, C₁-C₆-alkylsulphanyl, C₁-C₆-alkylsulphinyl, C₁-C₆-alkylsulphonyl, mono-C₁-C₆-alkylamino, di-C₁-C₆-alkylamino, nitro or cyano; heteroaryl-C₁-C₆-alkyl which is optionally mono- or polysubstituted at the heteroaromatic moiety by identical or different substituents from the group consisting of -OH, -SH, -NH₂, halogen, C₁-C₆-alkyl, C₁-C₆-haloalkyl, C₁-C₆-alkoxy, C₁-C₆-alkylsulphanyl, C₁-C₆-alkylsulphinyl, C₁-C₆-alkylsulphonyl, mono-C₁-C₆-alkylamino, di-C₁-C₆-alkylamino, nitro or cyano; heterocyclyl-C₁-C₆-alkyl which is optionally mono- or polysubstituted at the heterocycle by identical or different substituents from the group consisting of -OH / =0, -SH / =S,-NH₂, halogen, C₁-C₆-alkyl, C₁-C₆-haloalkyl, C₁-C₆-alkoxy, C₁-C₆-alkylsulphanyl, C₁-C₆-alkylsulphinyl, C₁-C₆-alkylsulphonyl, mono-C₁-C₆-alkylamino, di-C₁-C₆-alkylamino, nitro or cyano,
R⁴ represents 3-pyridyl or pyrazolyl which are in each case optionally mono- or polysubstituted by identical or different radicals from the group consisting of halogen, C₁-C₆-alkyl, C₁-C₆-haloalkyl, C₃-C₆-cycloalkyl, C₃-C₆-halocycloalkyl, C₃-C₆-cycloalkyl-C₁-C₆-alkyl, C₁-C₆-alkoxy, C₁-C₆-alkoxy-C₁-C₆-alkyl, C₁-C₆-alkoxy-C₁-C₆-alkoxy, COOH, nitro, amino, mono-C₁-C₆-alkylamino, di-C₁-C₆-alkylamino, C₁-C₆-alkylaminocarbonyl and C₃-C₆-cycloalkylaminocarbonyl,
R⁴ furthermore represents pyrazolyl which is optionally substituted by halogen or C₁-C₆-alkyl, phenyl (which for its part may be substituted by halogen) or by pyrazolylmethyl (which for its part may be substituted by C₁-C₆-haloalkyl),
R⁵ represents phenyl, 2-pyridyl or 3-pyridyl which are in each case optionally mono- or polysubstituted by identical or different radicals from the group consisting of halogen, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkoxy-C₁-C₆-alkyl, C₁-C₆-alkoxy-C₁-C₆-alkoxy, nitro, amino, cyano, mono-C₁-C₆-alkylamino, di-C₁-C₆-alkylamino, C₁-C₆-alkylsulphanyl, C₁-C₆-alkylsulphinyl, C₁-C₆-alkylsulphonyl, C₁-C₆-haloalkylsulphanyl, C₁-C₆-haloalkylsulphinyl, C₁-C₆-haloalkylsulphonyl, hetaryloxy-C₁-C₆-alkyl, hetaryloxy and hetaryl-C₁-C₆-alkyl, where the hetaryl radical is selected from the group consisting of pyrrolyl, imidazolyl, pyrazolyl, triazolyl, pyridyl, pyrimidinyl and tetrazolyl and for its part may be substituted by substituents from the group consisting of halogen, cyano, C₁-C₆-alkyl, C₁-C₆-haloalkyl, C₃-C₈-cycloalkyl and C₃-C₈-halocycloalkyl,
R⁵ also represents hydrogen, halogen, C₁-C₄-alkyl and C₃-C₆-cycloalkyl,
R⁶ represents a radical from the group consisting of hydrogen, halogen, C₁-C₆-alkyl, C₁-C₆-haloalkyl, C₁-C₆-alkoxy-C₁-C₆-alkyl, C₁-C₆-alkylcarbonyl, C₁-C₆-haloalkylcarbonyl, C₃-C₆-cycloalkyl, C₃-C₆-cycloalkyl-C₁-C₆-alkyl, C₁-C₆-alkylsulphanyl, C₂-C₆-alkenyl, C₂-C₆-alkynyl, C₁-C₆-alkoxycarbonyl, C₂-C₆-alkenyloxycarbonyl, C₂-C₆-alkynyloxycarbonyl, C₁-C₆-alkoxycarbonyl-C₁-C₆-alkyl, phenyl, phenyl-C₁-C₆-alkyl which are in each case optionally mono- or polysubstituted at the phenyl ring by identical or different substituents from the group consisting of halogen, C₁-C₆-alkyl, C₁-C₆-haloalkyl, C₁-C₆-alkoxy, nitro or cyano; heterocyclyl-C₁-C₆-alkyl which may optionally be mono- or polysubstituted at the heterocycle by identical or different substituents from the group consisting of halogen, C₁-C₆-alkyl, C₁-C₆-haloalkyl, C₁-C₆-alkoxy, nitro or cyano; and phenylcarbonyl which is optionally mono- or polysubstituted at the phenyl ring by identical or different substituents from the group consisting of halogen, C₁-C₆-alkyl, C₁-C₆-haloalkyl, C₁-C₆-alkoxy, nitro or cyano and
X represents oxygen or sulphur.

3. Compounds of the formula (I) according to Claim 1 in which
R¹ represents a radical from the group consisting of C₁-C₆-alkyl, C₁-C₆-haloalkyl, C₂-C₆-alkenyl, C₂-C₆-alkynyl, C₃-C₆-cycloalkyl, C₃-C₆-halocycloalkyl, C₁-C₆-alkoxy-C₁-C₆-alkyl, C₁-C₆-alkylsulphanyl-C₁-C₆-alkyl, C₁-C₆-alkoxycarbonyl-C₁-C₆-alkyl and C₃-C₆-cycloalkyl-C₁-C₆-alkyl and halogenated C₃-C₆-cycloalkyl-C₁-C₆-alkyl,
R² represents hydrogen,
R³ represents hydrogen,
R⁴ represents 3-pyridyl which is mono- or polysubstituted by radicals from the group consisting of halogen, C₁-C₆-alkyl, C₁-C₆-haloalkyl, C₃-C₆-cycloalkyl, C₃-C₆-halocycloalkyl, C₃-C₆-cycloalkyl-C₁-C₆-alkyl, C₁-C₆-alkoxy, C₁-C₆-alkylaminocarbonyl and C₃-C₆-cycloalkylaminocarbonyl,
R⁴ furthermore represents pyrazolyl which may be substituted by halogen (preferably F, Cl, Br) and C₁-C₆-alkyl (preferably methyl, ethyl and propyl), phenyl (which for its part may be substituted by halogen) or by pyrazolylmethyl (which for its part may be substituted by C₁-C₆-haloalkyl, in particular fluorinated C₁-C₆-alkyl),
R⁵ represents a radical from the group consisting of phenyl, 2-pyridyl and 3-pyridyl, each of which is mono- or polysubstituted by at least one radical from the group consisting of halogen, C₁-C₆-alkyl, C₁-C₆-haloalkyl, C₁-C₆-alkoxy, C₁-C₆-haloalkoxy, nitro, cyano, C₁-C₆-alkylsulphanyl, C₁-C₆-alkylsulphinyl, C₁-C₆-alkylsulphonyl, C₁-C₆-haloalkylsulphanyl, C₁-C₆-haloalkylsulphinyl, C₁-C₆-haloalkylsulphonyl, hetaryloxy-C₁-C₆-alkyl, hetaryloxy and hetaryl-C₁-C₆-alkyl, where the hetaryl radical is selected from the group consisting of pyrrolyl, imidazolyl, pyrazolyl, triazolyl, pyridyl, pyrimidinyl and tetrazolyl and may for its turn be substituted by substituents from the group consisting of halogen, cyano, C₁-C₆-alkyl, C₁-C₆-haloalkyl, C₃-C₈-cycloalkyl and C₃-C₈-halocycloalkyl,
R⁵ also represents a radical from the group consisting of hydrogen, halogen, C₁-C₄-alkyl and C₃-C₆-cycloalkyl,
R⁶ represents a radical from the group consisting of hydrogen, halogen, C₁-C₆-alkyl, C₁-C₆-haloalkyl, C₃-C₆-cycloalkyl, C₁-C₆-alkylsulphanyl and phenyl which may be substituted by halogen and
X represents oxygen.

4. Compounds of the formula (I) according to Claim 1 in which
R¹ represents a radical from the group consisting of CH₃, CH₂CH₃, CH(CH₃)₂, cyclopropyl, halocyclopropyl, C(CH₃)₃, CH(CH₃)CH₂SCH₃, CH₂CH₂CH₃, CH (CH₃) CH₂CH₃, CH₂CF₃, CHCH₃CF₃, CH (CH₃) cyclopropyl,
R² represents hydrogen,
R³ represents hydrogen,
R⁴ represents 3-pyridyl which is substituted in the 2-position by a substituent from the group consisting of F, Cl, Br, methyl, ethyl, CF₃, CH₂CF₃, CHFCH₃, CHFCF₃, cyclopropyl and cyclopropylmethyl,
R⁴ furthermore represents pyrazolyl which may be substituted by F, Cl, methyl, ethyl or by pyrazolylmethyl which for its part may be substituted by C₁-C₆-haloalkyl,
R⁵ represents phenyl which is mono- or polysubstituted by a radical from the group consisting of CH₃, F, Cl, CF₃, CF(CF₃)₂, OCH₃, OCF₃, NO₂, CN, SCF₃, S(O)CF₃ and S(O)₂CF₃ and CH₂-Q,
Q represents a radical from the group consisting of 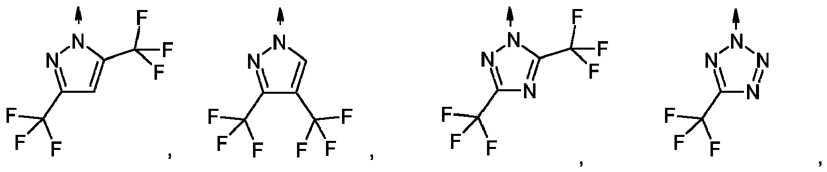 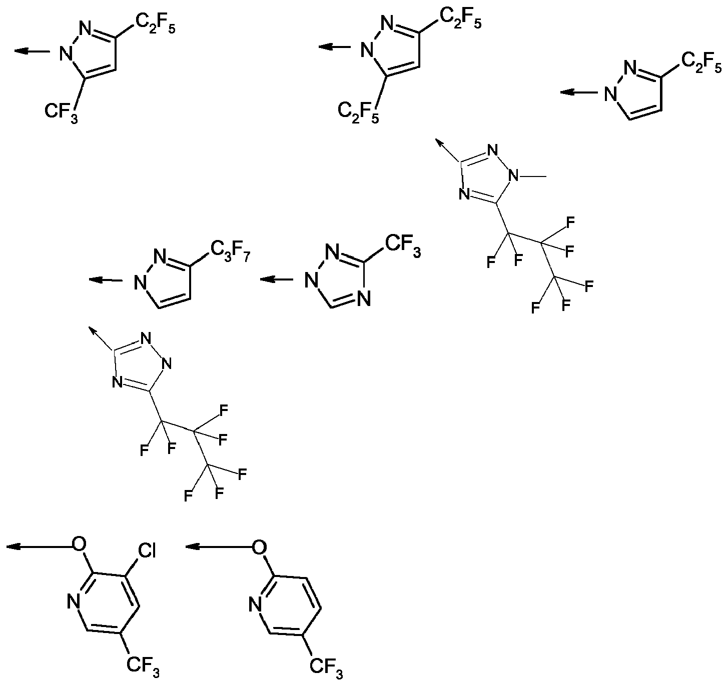 where the arrow indicates the point of attachment to the CH₂ group,
R⁶ represents hydrogen and
X represents oxygen.

5. Composition, **characterized in that** it comprises at least one compound of the formula (I) according to Claim 1 and customary extenders and/or surfactants.

6. Method for controlling plant pests, hygiene pests and pests of stored products, **characterized in that** a compound of the formula (I) according to Claim 1 or a composition according to Claim 5 is allowed to act on the pests and/or their habitat.

7. Use of compounds of the formula (I) according to Claim 1 or of compositions according to Claim 5 for controlling plant pests, hygiene pests and pests of stored products.

8. Pharmaceutical compositions comprising at least one compound according to Claim 1.

9. Use of at least one compound according to Claim 1 for preparing pharmaceutical compositions for controlling parasites on animals.

## Revendications

1. Composés de formule (I) 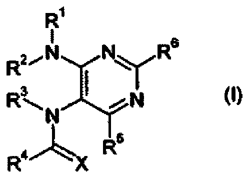 dans lesquels
R¹ représente un radical de la série alkyle en C₁-C₆, alcényle en C₂-C₆, alcynyle en C₂-C₆, haloalkyle en C₁-C₆, cyano-alkyle en C₁-C₆, cycloalkyle en C₃-C₈, halocycloalkyle en C₃-C₈, cycloalcényle en C₃-C₈, halocycloalcényle en C₃-C₈, alcoxy en C₁-C₆-alkyle en C₁-C₆, haloalcoxy en C₁-C₆-alkyle en C₁-C₆, alcoxy en C₁-C₆-haloalkyle en C₁-C₆, alkylcarbonyle en C₁-C₆-alkyle en C₁-C₆, alcoxycarbonyle en C₁-C₆-alkyle en C₁-C₆, alkylsulfanyle en C₁-C₆-alkyle en C₁-C₆, alkylsulfinyle en C₁-C₆-alkyle en C₁-C₆, alkylsulfonyle en C₁-C₆-alkyle en C₁-C₆, haloalkylsulfanyle en C₁-C₆-alkyle en C₁-C₆, haloalkylsulfinyle en C₁-C₆-alkyle en C₁-C₆, haloalkylsulfonyle en C₁-C₆-alkyle en C₁-C₆, cycloalkyle en C₃-C₈-alkyle en C₁-C₆, cycloalkyle en C₃-C₈-alkyle en C₁-C₆ halogéné, phényl-alkyle en C₁-C₆, qui est éventuellement substitué sur le cycle phényle une ou plusieurs fois, de manière identique ou différente, par halogène, alkyle en C₁-C₆, haloalkyle en C₁-C₆, alcoxy en C₁-C₆, haloalcoxy en C₁-C₆, alkylsulfanyle en C₁-C₆, alkylsulfinyle en C₁-C₆, alkylsulfonyle en C₁-C₆, haloalkylsulfanyle en C₁-C₆, haloalkylsulfinyle en C₁-C₆, haloalkylsulfonyle en C₁-C₆, nitro ou cyano ; hétéroaryle, qui est éventuellement substitué une ou plusieurs fois, de manière identique ou différente, par -OH, -SH, -NH₂, halogène, alkyle en C₁-C₆, haloalkyle en C₁-C₆, alcoxy en C₁-C₆, haloalcoxy en C₁-C₆, alkylsulfanyle en C₁-C₆, alkylsulfinyle en C₁-C₆, alkylsulfonyle en C₁-C₆, haloalkylsulfanyle en C₁-C₆, haloalkylsulfinyle en C₁-C₆, haloalkylsulfonyle en C₁-C₆, mono-alkylamino en C₁-C₆, di-alkylamino en C₁-C₆, nitro ou cyano ; hétéroaryl-alkyle en C₁-C₆, qui est éventuellement substitué sur l'hétéroaromatique une ou plusieurs fois, de manière identique ou différente, par -OH, -SH, -NH₂, halogène, alkyle en C₁-C₆, haloalkyle en C₁-C₆, alcoxy en C₁-C₆, haloalcoxy en C₁-C₆, alkylsulfanyle en C₁-C₆, alkylsulfinyle en C₁-C₆, alkylsulfonyle en C₁-C₆, haloalkylsulfanyle en C₁-C₆, haloalkylsulfinyle en C₁-C₆, haloalkylsulfonyle en C₁-C₆, mono-alkylamino en C₁-C₆, di-alkylamino en C₁-C₆, nitro ou cyano ; hétérocyclyle, qui est éventuellement substitué une ou plusieurs fois, de manière identique ou différente, par -OH/=O, -SH/=S, -NH₂, halogène, alkyle en C₁-C₆, haloalkyle en C₁-C₆, alcoxy en C₁-C₆, haloalcoxy en C₁-C₆, alkylsulfanyle en C₁-C₆, alkylsulfinyle en C₁-C₆, alkylsulfonyle en C₁-C₆, haloalkylsulfanyle en C₁-C₆, haloalkylsulfinyle en C₁-C₆, haloalkylsulfonyle en C₁-C₆, mono-alkylamino en C₁-C₆, di-alkylamino en C₁-C₆, nitro ou cyano ; et hétérocyclyle-alkyle en C₁-C₆, qui est éventuellement substitué sur l'hétérocycle une ou plusieurs fois, de manière identique ou différente, par -OH/=O, -SH/=S,-NH₂, halogène, alkyle en C₁-C₆, haloalkyle en C₁-C₆, alcoxy en C₁-C₆, haloalcoxy en C₁-C₆, alkylsulfanyle en C₁-C₆, alkylsulfinyle en C₁-C₆, alkylsulfonyle en C₁-C₆, haloalkylsulfanyle en C₁-C₆, haloalkylsulfinyle en C₁-C₆, haloalkylsulfonyle en C₁-C₆, mono-alkylamino en C₁-C₆, di-alkylamino en C₁-C₆, nitro ou cyano,
R² représente un radical de la série hydrogène, alkyle en C₁-C₆, alcényle en C₂-C₆, alcynyle en C₂-C₆, haloalkyle en C₁-C₆, cyano-alkyle en C₁-C₆, cycloalkyle en C₃-C₈, halocycloalkyle en C₃-C₈, cycloalcényle en C₃-C₈, halocycloalcényle en C₃-C₈, alcoxy en C₁-C₆-alkyle en C₁-C₆, haloalcoxy en C₁-C₆-alkyle en C₁-C₆, alkylcarbonyle en C₁-C₆-alkyle en C₁-C₆, alcoxycarbonyle en C₁-C₆-alkyle en C₁-C₆, alkylsulfanyle en C₁-C₆-alkyle en C₁-C₆, alkylsulfinyle en C₁-C₆-alkyle en C₁-C₆, alkylsulfonyle en C₁-C₆-alkyle en C₁-C₆, haloalkylsulfanyle en C₁-C₆-alkyle en C₁-C₆, haloalkylsulfinyle en C₁-C₆-alkyle en C₁-C₆, haloalkylsulfonyle en C₁-C₆-alkyle en C₁-C₆, cycloalkyle en C₃-C₈-alkyle en C₁-C₆, phényl-alkyle en C₁-C₆, qui est éventuellement substitué sur le cycle phényle une ou plusieurs fois, de manière identique ou différente, par halogène, alkyle en C₁-C₆, haloalkyle en C₁-C₆, alcoxy en C₁-C₆, haloalcoxy en C₁-C₆, alkylsulfanyle en C₁-C₆, alkylsulfinyle en C₁-C₆, alkylsulfonyle en C₁-C₆, haloalkylsulfanyle en C₁-C₆, haloalkylsulfinyle en C₁-C₆, haloalkylsulfonyle en C₁-C₆, nitro ou cyano ; hétéroaryle, qui est éventuellement substitué une ou plusieurs fois, de manière identique ou différente, par -OH, -SH, -NH₂, halogène, alkyle en C₁-C₆, haloalkyle en C₁-C₆, alcoxy en C₁-C₆, haloalcoxy en C₁-C₆, alkylsulfanyle en C₁-C₆, alkylsulfinyle en C₁-C₆, alkylsulfonyle en C₁-C₆, haloalkylsulfanyle en C₁-C₆, haloalkylsulfinyle en C₁-C₆, haloalkylsulfonyle en C₁-C₆, mono-alkylamino en C₁-C₆, di-alkylamino en C₁-C₆, nitro ou cyano ; hétéroaryl-alkyle en C₁-C₆, qui est éventuellement substitué sur l'hétéroaromatique une ou plusieurs fois, de manière identique ou différente, par -OH, -SH, -NH₂, halogène, alkyle en C₁-C₆, haloalkyle en C₁-C₆, alcoxy en C₁-C₆, haloalcoxy en C₁-C₆, alkylsulfanyle en C₁-C₆, alkylsulfinyle en C₁-C₆, alkylsulfonyle en C₁-C₆, haloalkylsulfanyle en C₁-C₆, haloalkylsulfinyle en C₁-C₆, haloalkylsulfonyle en C₁-C₆, mono-alkylamino en C₁-C₆, di-alkylamino en C₁-C₆, nitro ou cyano ; hétérocyclyle, qui est éventuellement substitué une ou plusieurs fois, de manière identique ou différente, par -OH/=O, -SH/=S, -NH₂, halogène, alkyle en C₁-C₆, haloalkyle en C₁-C₆, alcoxy en C₁-C₆, haloalcoxy en C₁-C₆, alkylsulfanyle en C₁-C₆, alkylsulfinyle en C₁-C₆, alkylsulfonyle en C₁-C₆, haloalkylsulfanyle en C₁-C₆, haloalkylsulfinyle en C₁-C₆, haloalkylsulfonyle en C₁-C₆, mono-alkylamino en C₁-C₆, di-alkylamino en C₁-C₆, nitro ou cyano ; et hétérocyclyle-alkyle en C₁-C₆, qui est éventuellement substitué sur l'hétérocycle une ou plusieurs fois, de manière identique ou différente, par -OH/=O, -SH/=S,-NH₂, halogène, alkyle en C₁-C₆, haloalkyle en C₁-C₆, alcoxy en C₁-C₆, haloalcoxy en C₁-C₆, alkylsulfanyle en C₁-C₆, alkylsulfinyle en C₁-C₆, alkylsulfonyle en C₁-C₆, haloalkylsulfanyle en C₁-C₆, haloalkylsulfinyle en C₁-C₆, haloalkylsulfonyle en C₁-C₆, mono-alkylamino en C₁-C₆, di-alkylamino en C₁-C₆, nitro ou cyano,
R³ représente un radical de la série hydrogène, alkyle en C₁-C₆, alcényle en C₂-C₆, alcynyle en C₂-C₆, haloalkyle en C₁-C₆, cyano-alkyle en C₁-C₆, cycloalkyle en C₃-C₈, halocycloalkyle en C₃-C₈, cycloalcényle en C₃-C₈, halocycloalcényle en C₃-C₈, alcoxy en C₁-C₆-alkyle en C₁-C₆, alkylcarbonyle en C₁-C₆-alkyle en C₁-C₆, alcoxycarbonyle en C₁-C₆-alkyle en C₁-C₆, alkylsulfanyle en C₁-C₆-alkyle en C₁-C₆, alkylsulfinyle en C₁-C₆-alkyle en C₁-C₆, alkylsulfonyle en C₁-C₆-alkyle en C₁-C₆, cycloalkyle en C₃-C₈-alkyle en C₁-C₆, phényle, qui est éventuellement substitué une ou plusieurs fois, de manière identique ou différente, par -OH, -SH, -NH₂, halogène, alkyle en C₁-C₆, haloalkyle en C₁-C₆, alcoxy en C₁-C₆, alkylsulfanyle en C₁-C₆, alkylsulfinyle en C₁-C₆, alkylsulfonyle en C₁-C₆, mono-alkylamino en C₁-C₆, di-alkylamino en C₁-C₆, nitro ou cyano ; phényl-alkyle en C₁-C₆, qui est éventuellement substitué sur le cycle phényle une ou plusieurs fois, de manière identique ou différente, par halogène, alkyle en C₁-C₆, haloalkyle en C₁-C₆, alcoxy en C₁-C₆, nitro ou cyano ; hétéroaryle, qui est éventuellement substitué une ou plusieurs fois, de manière identique ou différente, par -OH, -SH, -NH₂, halogène, alkyle en C₁-C₆, haloalkyle en C₁-C₆, alcoxy en C₁-C₆, alkylsulfanyle en C₁-C₆, alkylsulfinyle en C₁-C₆, alkylsulfonyle en C₁-C₆, mono-alkylamino en C₁-C₆, di-alkylamino en C₁-C₆, nitro ou cyano ; hétéroaryl-alkyle en C₁-C₆, qui est éventuellement substitué sur l'hétéroaromatique une ou plusieurs fois, de manière identique ou différente, par -OH, -SH, -NH₂, halogène, alkyle en C₁-C₆, haloalkyle en C₁-C₆, alcoxy en C₁-C₆, alkylsulfanyle en C₁-C₆, alkylsulfinyle en C₁-C₆, alkylsulfonyle en C₁-C₆, mono-alkylamino en C₁-C₆, di-alkylamino en C₁-C₆, nitro ou cyano ; hétérocyclyle, qui est éventuellement substitué une ou plusieurs fois, de manière identique ou différente, par -OH/=O, -SH/=S,-NH₂, halogène, alkyle en C₁-C₆, haloalkyle en C₁-C₆, alcoxy en C₁-C₆, alkylsulfanyle en C₁-C₆, alkylsulfinyle en C₁-C₆, alkylsulfonyle en C₁-C₆, mono-alkylamino en C₁-C₆, di-alkylamino en C₁-C₆, nitro ou cyano ; et hétérocyclyle-alkyle en C₁-C₆, qui est éventuellement substitué sur l'hétérocycle une ou plusieurs fois, de manière identique ou différente, par -OH/=O, -SH/=S,-NH₂, halogène, alkyle en C₁-C₆, haloalkyle en C₁-C₆, alcoxy en C₁-C₆, alkylsulfanyle en C₁-C₆, alkylsulfinyle en C₁-C₆, alkylsulfonyle en C₁-C₆, mono-alkylamino en C₁-C₆, di-alkylamino en C₁-C₆, nitro ou cyano,
R⁴ représente un radical de la série 2-pyridyle, 3-pyridyle et pyrazolyle, qui sont chacun éventuellement substitués une ou plusieurs fois, de manière identique ou différente, par des radicaux de la série halogène, alkyle en C₁-C₆, haloalkyle en C₁-C₆, cycloalkyle en C₃-C₆, halocycloalkyle en C₃-C₆, cycloalkyle en C₃-C₆-alkyle en C₁-C₆, alcoxy en C₁-C₆, haloalcoxy en C₁-C₆, alcoxy en C₁-C₆-alkyle en C₁-C₆, alcoxy en C₁-C₆-alcoxy en C₁-C₆, COOH, nitro, amino, cyano, alkylsulfanyle en C₁-C₆, alkylsulfinyle en C₁-C₆, alkylsulfonyle en C₁-C₆, haloalkylsulfanyle en C₁-C₆, haloalkylsulfinyle en C₁-C₆, haloalkylsulfonyle en C₁-C₆, mono-alkylamino en C₁-C₆, di-alkylamino en C₁-C₆, alkylaminocarbonyle en C₁-C₆, cycloalkylaminocarbonyle en C₃-C₆, phényle (qui peut lui-même être substitué à son tour par halogène) et hétaryl-alkyle en C₁-C₆ (notamment pyrazolyl-alkyle en C₁-C₆ (qui peut lui-même être substitué à son tour par halogène, alkyle en C₁-C₆ et halogénoalkyle en C₁-C₆)), R⁵ représente un radical de la série hydrogène, halogène, cyano, alkyle en C₁-C₆, haloalkyle en C₁-C₆, cycloalkyle en C₃-C₈, halocycloalkyle en C₃-C₈, ainsi que phényle, 2-pyridyle ou 3-pyridyle, qui sont chacun éventuellement substitués une ou plusieurs fois, de manière identique ou différente, par des radicaux de la série halogène, amino, cyano, alkyle en C₁-C₆, haloalkyle en C₁-C₆, cycloalkyle en C₃-C₆, halocycloalkyle en C₃-C₆, alcoxy en C₁-C₆, haloalcoxy en C₁-C₆, alcényle en C₂-C₆, alcoxy en C₁-C₆-alkyle en C₁-C₆, alcoxy en C₁-C₆-alcoxy en C₁-C₆, nitro, cyano, alkylcarbonyle en C₁-C₆, alkylcarbonylamino en C₁-C₆, cycloalkylcarbonylamino en C₃-C₆, alkylaminocarbonyle en C₁-C₆, cycloalkylaminocarbonyle en C₃-C₆, alcoxycarbonyle en C₁-C₆, aminocarbonyle, alcoxyimino en C₁-C₆-alkyle en C₁-C₆, alkylsulfanyle en C₁-C₆, alkylsulfinyle en C₁-C₆, alkylsulfonyle en C₁-C₆, alkylsulfonyloxy en C₁-C₆, alkylsulfonylamino en C₁-C₆, haloalkylsulfanyle en C₁-C₆, haloalkylsulfinyle en C₁-C₆, haloalkylsulfonyle en C₁-C₆, tri-alkylsilyle en C₁-C₆, S(O)(=N-CN), S(O)(=N)R (R représentant alkyle en C₁-C₆ ou haloalkyle en C₁-C₆), phényle, phényl-alkyle en C₁-C₆, phényl-alkyloxy en C₁-C₆, phényloxy, pyrazolyle (les groupes phényle ou le cycle pyrazolyle pouvant à leur tour être substitués par halogène, alkyle en C₁-C₆, cycloalkyle en C₃-C₈, alcoxy en C₁-C₆, haloalcoxy en C₁-C₆, nitro, cyano, alkylsulfanyle en C₁-C₆, alkylsulfinyle en C₁-C₆, alkylsulfonyle en C₁-C₆, alkylsulfonyloxy en C₁-C₆, alkylsulfonylamino en C₁-C₆, haloalkylsulfanyle en C₁-C₆, haloalkylsulfinyle en C₁-C₆, haloalkylsulfonyle en C₁-C₆, S(O)(=N-CN), S(O)(=N)R (R représentant alkyle en C₁-C₆ ou haloalkyle en C₁-C₆)), hétaryloxy-alkyle en C₁-C₆, hétaryloxy et hétaryl-alkyle en C₁-C₆ (le radical hétaryle étant à chaque fois choisi dans la série pyrrolyle, imidazolyle, pyrazolyle, triazolyle, pyridyle, pyrimidinyle et tétrazolyle et pouvant lui-même à son tour être substitué par halogène, cyano, alkyle en C₁-C₆, haloalkyle en C₁-C₆, cycloalkyle en C₃-C₈, halocycloalkyle en C₃-C₈) et deux substituants voisins pouvant également représenter ensemble -OCH₂O-, -OCF₂O- ou -OCH₂CH₂O-,
R⁶ représente un radical de la série hydrogène, halogène, alkyle en C₁-C₆, haloalkyle en C₁-C₆, cyano-alkyle en C₁-C₆, alcoxy en C₁-C₆-alkyle en C₁-C₆, alkylcarbonyle en C₁-C₆, haloalkylcarbonyle en C₁-C₆, cycloalkyle en C₃-C₈, halocycloalkyle en C₃-C₈, cycloalkyle en C₃-C₈-alkyle en C₁-C₆, alkylsulfanyle en C₁-C₆, alkylsulfonyle en C₁-C₆, alcényle en C₂-C₆, alcynyle en C₂-C₆, alcoxycarbonyle en C₁-C₆, alcényloxycarbonyle en C₂-C₆, alcynyloxycarbonyle en C₂-C₆, alcoxycarbonyle en C₁-C₆-alkyle en C₁-C₆ ; phényle, phényl-alkyle en C₁-C₆, qui sont chacun éventuellement substitué sur le cycle phényle une ou plusieurs fois, de manière identique ou différente, par halogène, alkyle en C₁-C₆, haloalkyle en C₁-C₆, alcoxy en C₁-C₆, nitro ou cyano ; hétérocyclyl-alkyle en C₁-C₆, qui est éventuellement substitué sur l'hétérocycle une ou plusieurs fois, de manière identique ou différente, par halogène, alkyle en C₁-C₆, haloalkyle en C₁-C₆, alcoxy en C₁-C₆, nitro ou cyano ; hétéroaryl-alkyle en C₁-C₆, qui est éventuellement substitué sur l'hétéroaromatique une ou plusieurs fois, de manière identique ou différente, par halogène, alkyle en C₁-C₆, haloalkyle en C₁-C₆, alcoxy en C₁-C₆, nitro ou cyano ; ou phénylcarbonyle, qui est éventuellement substitué sur le cycle phényle une ou plusieurs fois, de manière identique ou différente, par halogène, alkyle en C₁-C₆, haloalkyle en C₁-C₆, alcoxy en C₁-C₆, nitro ou cyano, et
X représente oxygène ou soufre.

2. Composés de formule (I) selon la revendication 1, dans lesquels
R¹ représente un radical de la série alkyle en C₁-C₆, alcényle en C₂-C₆, alcynyle en C₂-C₆, haloalkyle en C₁-C₆, cycloalkyle en C₃-C₈, halocycloalkyle en C₃-C₈, cycloalcényle en C₃-C₈, alcoxy en C₁-C₆-alkyle en C₁-C₆, alkylsulfanyle en C₁-C₆-alkyle en C₁-C₆, alkylcarbonyle en C₁-C₆-alkyle en C₁-C₆, alcoxycarbonyle en C₁-C₆-alkyle en C₁-C₆, cycloalkyle en C₃-C₆-alkyle en C₁-C₆, cycloalkyle en C₃-C₆-alkyle en C₁-C₆ halogéné, phényl-alkyle en C₁-C₆, qui est éventuellement substitué sur le cycle phényle une ou plusieurs fois, de manière identique ou différente, par halogène, alkyle en C₁-C₆, haloalkyle en C₁-C₆, alcoxy en C₁-C₆, nitro ou cyano ; hétéroaryle, qui est éventuellement substitué une ou plusieurs fois, de manière identique ou différente, par -OH, -SH, -NH₂, halogène, alkyle en C₁-C₆, haloalkyle en C₁-C₆, alcoxy en C₁-C₆, alkylsulfanyle en C₁-C₆, alkylsulfinyle en C₁-C₆, alkylsulfonyle en C₁-C₆, mono-alkylamino en C₁-C₆, di-alkylamino en C₁-C₆, nitro ou cyano ; hétéroaryl-alkyle en C₁-C₆, qui est éventuellement substitué sur l'hétéroaromatique une ou plusieurs fois, de manière identique ou différente, par -OH, -SH, -NH₂, halogène, alkyle en C₁-C₆, haloalkyle en C₁-C₆, alcoxy en C₁-C₆, alkylsulfanyle en C₁-C₆, alkylsulfinyle en C₁-C₆, alkylsulfonyle en C₁-C₆, mono-alkylamino en C₁-C₆, di-alkylamino en C₁-C₆, nitro ou cyano ; hétérocyclyle-alkyle en C₁-C₆, qui est éventuellement substitué sur l'hétérocycle une ou plusieurs fois, de manière identique ou différente, par -OH/=O, -SH/=S, -NH₂, halogène, alkyle en C₁-C₆, haloalkyle en C₁-C₆, alcoxy en C₁-C₆, alkylsulfanyle en C₁-C₆, alkylsulfinyle en C₁-C₆, alkylsulfonyle en C₁-C₆, mono-alkylamino en C₁-C₆, di-alkylamino en C₁-C₆, nitro ou cyano,
R² représente un radical de la série hydrogène, alkyle en C₁-C₆, alcényle en C₂-C₆, alcynyle en C₂-C₆, haloalkyle en C₁-C₆, cycloalkyle en C₃-C₈, halocycloalkyle en C₃-C₈, cycloalcényle en C₃-C₈, alcoxy en C₁-C₆-alkyle en C₁-C₆, alkylcarbonyle en C₁-C₆-alkyle en C₁-C₆, alcoxycarbonyle en C₁-C₆-alkyle en C₁-C₆, phényl-alkyle en C₁-C₆, qui est éventuellement substitué sur le cycle phényle une ou plusieurs fois, de manière identique ou différente, par halogène, alkyle en C₁-C₆, haloalkyle en C₁-C₆, alcoxy en C₁-C₆, nitro ou cyano ; hétéroaryle, qui est éventuellement substitué une ou plusieurs fois, de manière identique ou différente, par -OH, -SH, -NH₂, halogène, alkyle en C₁-C₆, haloalkyle en C₁-C₆, alcoxy en C₁-C₆, alkylsulfanyle en C₁-C₆, alkylsulfinyle en C₁-C₆, alkylsulfonyle en C₁-C₆, mono-alkylamino en C₁-C₆, di-alkylamino en C₁-C₆, nitro ou cyano ; hétéroaryl-alkyle en C₁-C₆, qui est éventuellement substitué sur l'hétéroaromatique une ou plusieurs fois, de manière identique ou différente, par -OH, -SH, -NH₂, halogène, alkyle en C₁-C₆, haloalkyle en C₁-C₆, alcoxy en C₁-C₆, alkylsulfanyle en C₁-C₆, alkylsulfinyle en C₁-C₆, alkylsulfonyle en C₁-C₆, mono-alkylamino en C₁-C₆, di-alkylamino en C₁-C₆, nitro ou cyano ; hétérocyclyle-alkyle en C₁-C₆, qui est éventuellement substitué sur l'hétérocycle une ou plusieurs fois, de manière identique ou différente, par -OH/=O, -SH/=S, -NH₂, halogène, alkyle en C₁-C₆, haloalkyle en C₁-C₆, alcoxy en C₁-C₆, alkylsulfanyle en C₁-C₆, alkylsulfinyle en C₁-C₆, alkylsulfonyle en C₁-C₆, mono-alkylamino en C₁-C₆, di-alkylamino en C₁-C₆, nitro ou cyano,
R³ représente un radical de la série hydrogène, alkyle en C₁-C₆, alcényle en C₂-C₆, alcynyle en C₂-C₆, haloalkyle en C₁-C₆, cycloalkyle en C₃-C₈, halocycloalkyle en C₃-C₈, cycloalcényle en C₃-C₈, alcoxy en C₁-C₆-alkyle en C₁-C₆, alkylcarbonyle en C₁-C₆-alkyle en C₁-C₆, alcoxycarbonyle en C₁-C₆-alkyle en C₁-C₆ ; phényl-alkyle en C₁-C₆, qui est éventuellement substitué sur le cycle phényle une ou plusieurs fois, de manière identique ou différente, par halogène, alkyle en C₁-C₆, haloalkyle en C₁-C₆, alcoxy en C₁-C₆, nitro ou cyano ; hétéroaryle, qui est éventuellement substitué une ou plusieurs fois, de manière identique ou différente, par -OH, -SH, -NH₂, halogène, alkyle en C₁-C₆, haloalkyle en C₁-C₆, alcoxy en C₁-C₆, alkylsulfanyle en C₁-C₆, alkylsulfinyle en C₁-C₆, alkylsulfonyle en C₁-C₆, mono-alkylamino en C₁-C₆, di-alkylamino en C₁-C₆, nitro ou cyano ; hétéroaryl-alkyle en C₁-C₆, qui est éventuellement substitué sur l'hétéroaromatique une ou plusieurs fois, de manière identique ou différente, par -OH, -SH, -NH₂, halogène, alkyle en C₁-C₆, haloalkyle en C₁-C₆, alcoxy en C₁-C₆, alkylsulfanyle en C₁-C₆, alkylsulfinyle en C₁-C₆, alkylsulfonyle en C₁-C₆, mono-alkylamino en C₁-C₆, di-alkylamino en C₁-C₆, nitro ou cyano ; hétérocyclyle-alkyle en C₁-C₆, qui peut éventuellement être substitué sur l'hétérocycle une ou plusieurs fois, de manière identique ou différente, par -OH/=O, -SH/=S, -NH₂, halogène, alkyle en C₁-C₆, haloalkyle en C₁-C₆, alcoxy en C₁-C₆, alkylsulfanyle en C₁-C₆, alkylsulfinyle en C₁-C₆, alkylsulfonyle en C₁-C₆, mono-alkylamino en C₁-C₆, di-alkylamino en C₁-C₆, nitro ou cyano,
R⁴ représente 3-pyridyle ou pyrazolyle, qui sont chacun éventuellement substitués une ou plusieurs fois, de manière identique ou différente, par des radicaux de la série halogène, alkyle en C₁-C₆, haloalkyle en C₁-C₆, cycloalkyle en C₃-C₆, halocycloalkyle en C₃-C₆, cycloalkyle en C₃-C₆-alkyle en C₁-C₆, alcoxy en C₁-C₆, alcoxy en C₁-C₆-alkyle en C₁-C₆, alcoxy en C₁-C₆-alcoxy en C₁-C₆, COOH, nitro, amino, mono-alkylamino en C₁-C₆, di-alkylamino en C₁-C₆, alkylaminocarbonyle en C₁-C₆ et cycloalkylaminocarbonyle en C₃-C₆,
R⁴ représente également pyrazolyle, qui est éventuellement substitué par halogène ou alkyle en C₁-C₆, phényle (qui peut lui-même à son tour être substitué par halogène) ou par pyrazolylméthyle (qui peut lui-même à son tour être substitué par halogénoalkyle en C₁-C₆),
R⁵ représente phényle, 2-pyridyle ou 3-pyridyle, qui sont chacun éventuellement substitués une ou plusieurs fois, de manière identique ou différente, par des radicaux de la série halogène, alkyle en C₁-C₆, haloalkyle en C₁-C₆, cycloalkyle en C₃-C₆, halocycloalkyle en C₃-C₆, alcoxy en C₁-C₆, haloalcoxy en C₁-C₆, alcoxy en C₁-C₆-alkyle en C₁-C₆, alcoxy en C₁-C₆-alcoxy en C₁-C₆, nitro, amino, cyano, mono-alkylamino en C₁-C₆, di-alkylamino en C₁-C₆, alkylsulfanyle en C₁-C₆, alkylsulfinyle en C₁-C₆, alkylsulfonyle en C₁-C₆, haloalkylsulfanyle en C₁-C₆, haloalkylsulfinyle en C₁-C₆, haloalkylsulfonyle en C₁-C₆, hétaryloxy-alkyle en C₁-C₆, hétaryloxy et hétaryl-alkyle en C₁-C₆, le radical hétaryle étant choisi dans la série pyrrolyle, imidazolyle, pyrazolyle, triazolyle, pyridyle, pyrimidinyle et tétrazolyle et pouvant lui-même à son tour être substitué par des substituants de la série halogène, cyano, alkyle en C₁-C₆, haloalkyle en C₁-C₆, cycloalkyle en C₃-C₈ et halocycloalkyle en C₃-C₈,
R⁵ représente également hydrogène, halogène, alkyle en C₁-C₄ et cycloalkyle en C₃-C₆,
R⁶ représente un radical de la série hydrogène, halogène, alkyle en C₁-C₆, haloalkyle en C₁-C₆, alcoxy en C₁-C₆-alkyle en C₁-C₆, alkylcarbonyle en C₁-C₆, haloalkylcarbonyle en C₁-C₆, cycloalkyle en C₃-C₆, cycloalkyle en C₃-C₆-alkyle en C₁-C₆, alkylsulfanyle en C₁-C₆, alcényle en C₂-C₆, alcynyle en C₂-C₆, alcoxycarbonyle en C₁-C₆, alcényloxycarbonyle en C₂-C₆, alcynyloxycarbonyle en C₂-C₆, alcoxycarbonyle en C₁-C₆-alkyle en C₁-C₆ ; phényle, phényl-alkyle en C₁-C₆, qui sont chacun éventuellement substitués sur le cycle phényle une ou plusieurs fois, de manière identique ou différente, par halogène, alkyle en C₁-C₆, haloalkyle en C₁-C₆, alcoxy en C₁-C₆, nitro ou cyano ; hétérocyclyl-alkyle en C₁-C₆, qui est éventuellement substitué sur l'hétérocycle une ou plusieurs fois, de manière identique ou différente, par halogène, alkyle en C₁-C₆, haloalkyle en C₁-C₆, alcoxy en C₁-C₆, nitro ou cyano ; et phénylcarbonyle, qui est éventuellement substitué sur le cycle phényle une ou plusieurs fois, de manière identique ou différente, par halogène, alkyle en C₁-C₆, haloalkyle en C₁-C₆, alcoxy en C₁-C₆, nitro ou cyano, et
X représente oxygène ou soufre.

3. Composés de formule (I) selon la revendication 1, dans lesquels
R¹ représente un radical de la série alkyle en C₁-C₆, haloalkyle en C₁-C₆, alcényle en C₂-C₆, alcynyle en C₂-C₆, cycloalkyle en C₃-C₆, halocycloalkyle en C₃-C₆, alcoxy en C₁-C₆-alkyle en C₁-C₆, alkylsulfanyle en C₁-C₆-alkyle en C₁-C₆, alcoxycarbonyle en C₁-C₆-alkyle en C₁-C₆ et cycloalkyle en C₃-C₆-alkyle en C₁-C₆ et cycloalkyle en C₃-C₆-alkyle en C₁-C₆ halogéné,
R² représente hydrogène,
R³ représente hydrogène,
R⁴ représente 3-pyridyle, qui est substitué une ou plusieurs fois par des radicaux de la série halogène, alkyle en C₁-C₆, haloalkyle en C₁-C₆, cycloalkyle en C₃-C₆, halocycloalkyle en C₃-C₆, cycloalkyle en C₃-C₆-alkyle en C₁-C₆, alcoxy en C₁-C₆, alkylaminocarbonyle en C₁-C₆ et cycloalkylaminocarbonyle en C₃-C₆,
R⁴ représente également pyrazolyle, qui peut être substitué par halogène (de préférence F, Cl, Br) et alkyle en C₁-C₆ (de préférence méthyle, éthyle et propyle), phényle (qui peut lui-même à son tour être substitué par halogène) ou par pyrazolylméthyle (qui peut lui-même à son tour être substitué par halogénoalkyle en C₁-C₆, notamment alkyle en C₁-C₆ fluoré),
R⁵ représente un radical de la série phényle, 2-pyridyle et 3-pyridyle, qui est à chaque fois substitué une ou plusieurs fois par au moins un radical de la série halogène, alkyle en C₁-C₆, haloalkyle en C₁-C₆, alcoxy en C₁-C₆, haloalcoxy en C₁-C₆, nitro, cyano, alkylsulfanyle en C₁-C₆, alkylsulfinyle en C₁-C₆, alkylsulfonyle en C₁-C₆, haloalkylsulfanyle en C₁-C₆, haloalkylsulfinyle en C₁-C₆, haloalkylsulfonyle en C₁-C₆, hétaryloxy-alkyle en C₁-C₆, hétaryloxy et hétaryl-alkyle en C₁-C₆, le radical hétaryle étant choisi dans la série pyrrolyle, imidazolyle, pyrazolyle, triazolyle, pyridyle, pyrimidinyle et tétrazolyle et pouvant lui-même à son tour être substitué par des substituants de la série halogène, cyano, alkyle en C₁-C₆, haloalkyle en C₁-C₆, cycloalkyle en C₃-C₈ et halocycloalkyle en C₃-C₈, R⁵ représente également un radical de la série hydrogène, halogène, alkyle en C₁-C₄ et cycloalkyle en C₃-C₆,
R⁶ représente un radical de la série hydrogène, halogène, alkyle en C₁-C₆, haloalkyle en C₁-C₆, cycloalkyle en C₃-C₆, alkylsulfanyle en C₁-C₆ et phényle, qui peut être substitué par halogène, et
X représente oxygène.

4. Composés de formule (I) selon la revendication 1, dans lesquels
R¹ représente un radical de la série CH₃, CH₂CH₃, CH(CH₃)₂, cyclopropyle, halocyclopropyle, C(CH₃)₃, CH(CH₃)CH₂SCH₃, CH₂CH₂CH₃, CH(CH₃)CH₂CH₃, CH₂CF₃, CHCH₃CF₃, CH(CH₃)cyclopropyle,
R² représente hydrogène,
R³ représente hydrogène,
R⁴ représente 3-pyridyle, qui est substitué en position 2 par un substituant de la série F, Cl, Br, méthyle, éthyle, CF₃, CH₂CF₃, CHFCH₃, CHFCF₃, cyclopropyle et cyclopropylméthyle,
R⁴ représente également pyrazolyle, qui peut être substitué par F, Cl, méthyle, éthyle ou par pyrazolylméthyle, qui peut lui-même à son tour être substitué par halogénoalkyle en C₁-C₆,
R⁵ représente phényle, qui est substitué une ou plusieurs fois par un radical de la série CH₃, F, Cl, CF₃, CF(CF₃)₂, OCH₃, OCF₃, NO₂, CN, SCF₃, S(O)CF₃ et S(O)₂CF₃ et CH₂-Q,
Q représente un radical de la série 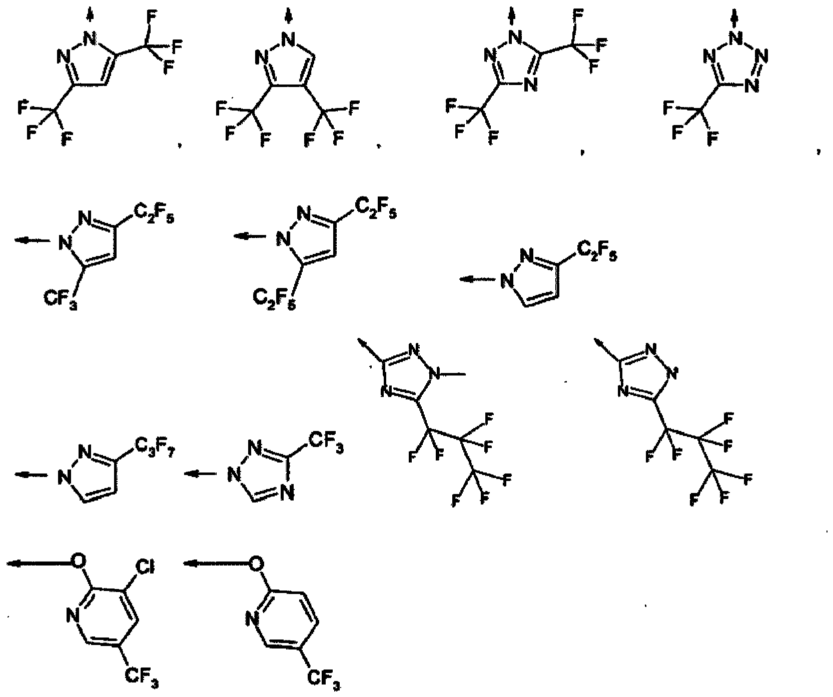 la flèche indiquant l'emplacement de liaison avec le groupe CH₂,
R⁶ représente hydrogène, et
X représente oxygène.

5. Agent, **caractérisé par** une teneur en au moins un composé de formule (I) selon la revendication 1 et en des extendeurs et/ou agents tensioactifs usuels.

6. Procédé de lutte contre les animaux nuisibles pour les plantes, l'hygiène et les stocks, **caractérisé en ce qu'**un composé de formule (I) selon la revendication 1 ou un agent selon la revendication 5 est laissé agir sur les animaux nuisibles et/ou leur habitat.

7. Utilisation de composés de formule (I) selon la revendication 1 ou d'agents selon la revendication 5 pour lutter contre les animaux nuisibles pour les plantes, l'hygiène et les stocks.

8. Compositions pharmaceutiques, contenant au moins un composé selon la revendication 1.

9. Utilisation d'au moins un composé selon la revendication 1 pour la fabrication de compositions pharmaceutiques pour lutter contre les parasites sur les animaux.
